# EUROPEAN PATENT APPLICATION

(11) **EP 4 455 306 A1**
(43) Date of publication of application: **30.10.2024**
(21) Application number: 22909691.2
(22) Date of filing: 30.11.2022
(51) Int. Cl.: C12Q 1/6869, C12Q 1/6886, C40B 50/06

(54) **LABELING AND ANALYSIS METHOD FOR SINGLE-CELL NUCLEIC ACID**

(30) Priority: 24.12.2021 CN 202111600833
(71) Applicant: BGI Shenzhen, Shenzhen, Guangdong 518083 (CN)
(72) Inventor: LIAO, Sha, Shenzhen, Guangdong 518083 (CN); CHEN, Ao, Shenzhen, Guangdong 518083 (CN); ZHANG, Wenwei, Shenzhen, Guangdong 518083 (CN); XU, Xun, Shenzhen, Guangdong 518083 (CN)
(74) Representative: Inspicos P/S
(86) International application number: PCT/CN2022/135478
(87) International publication number: WO 2023/116376

(57) **Abstract**

Provided are a method for positioning and labeling a nucleic acid molecule, and a method for constructing a nucleic acid molecule library for single-cell transcriptome sequencing, which relate to the technical fields of single-cell transcriptome sequencing and biomolecular space information detection. Further provided are a nucleic acid molecule library constructed by using the method, and a kit for implementing the method.

## Description

### Technical Field

The present application relates to the technical fields of single-cell transcriptome sequencing and biomolecule spatial information detection. Specifically, the present application relates to a method for positionally labeling a nucleic acid molecule in a sample of individual cells and a method for constructing a single-cell transcriptome sequencing library. Furthermore, the present application relates to a kit for carrying out the methods.

### Background Art

Single-cell transcriptome sequencing technology is an important tool for identifying cellular heterogeneity. The importance of single-cell transcriptome sequencing technology has promoted the rapid development of this technology in terms of throughput, ease operation, etc. The development of single-cell transcriptome sequencing technology has prompted the international community to spend huge sums of money to launch the Human Cell Atlas Project for creating a 3-dimensional Atlas of human cells. The launch of the Human Cell Atlas Project has put forward higher requirements and challenges for the throughput of single-cell transcriptome sequencing technology. In addition to scientific research needs, single-cell transcriptome sequencing technology is also being used by medical workers to discover a small number of "cancer stem cells" in cancers, so as to find targeted drugs and therapies to overcome malignant tumors. Since malignant tumor cells are relatively rare, a high cell utilization or capture rate is required for single-cell transcriptome sequencing technology to avoid the loss of transcriptome information of malignant tumor cells that are a small number in quantity.

Existing single-cell transcriptome sequencing technologies mainly include two categories: one is low-throughput sequencing technology based on multi-well plates, in which single cells are assigned to individual wells of multi-well plates, such as smart-seq and CEL-seq; the other one is magnetic bead-based sequencing technology, such as 10x chromium, Drop-seq, Seq-well and other technologies, in which a cell and labeled magnetic beads are co-wrapped in microdroplets or microwells through microfluidics. Among the existing single-cell transcriptome sequencing technologies, 10x chromium has the highest throughput, and its throughput for single run is 5,000 to 7,000 cells, and can reach up to 10,000 cells. Moreover, depending on the cell type, its cell capture rate is 30% to 60%.

Taking 10x chromium, which is the most widely used in the market, as an example, its technical feature is the use of a microfluidic system for cell sorting. In short, the gel beads bearing label molecules or barcode molecules (Barcode) are allowed to enter the microfluidic system at a uniform speed; and the cells to be sorted and enzymes are allowed to enter at certain time intervals, bind to the gel beads, and form GEMs (Gel Bead in emulsion) in the oil phase. Ideally, each cell binds to one Gel Bead to form one GEM. Thus, this method can achieve the purpose of single-cell transcriptome sequencing. However, the formation of GEMs follows a Poisson distribution. That is, it is possible that a single GEM contains 0 or more cells. Since the sequencing data generated by this GEM does not correspond to the status of a single cell, it cannot be used later and needs to be filtered by an algorithm. Limited by the number of microdroplets formed in the oil phase, the throughput of this technology is difficult to exceed the level of ten thousands; at the same time, due to the characteristics of the Poisson distribution, the maximum cell capture rate of this technology can theoretically reach up to 60%. Therefore, when single-cell transcriptome sequencing with throughput of 100,000 or even more cells is required or rare cells need to be captured and sequenced, this technology still has major shortcomings and is difficult to meet actual needs. Therefore, there is a need in the field to develop new single-cell transcriptome sequencing methods with higher cell capture rates.

### Contents of the present application

The present application provides a method for positionally labeling nucleic acid molecules in a cell sample, and a method for constructing a single-cell transcriptome sequencing library based on this method. Furthermore, the present application also relates to a kit for carrying out the methods.

### Methods for Generating a Population of Labeled Nucleic Acid Molecules

In one aspect, the present application provides a method for generating a population of labeled nucleic acid molecules, which comprises the following steps:
(1) providing: a sample containing one or more cells, and a nucleic acid array;
   wherein, the sample is a single-cell suspension; the cell comprises (e.g., on its surface) a first binding molecule;
   the nucleic acid array comprises a solid support, the solid support comprises (e.g., on its surface) a first label molecule, and the first binding molecule is capable of forming an interaction pair with the first label molecule;
   moreover, the solid support further comprises a plurality of microdots, the size (e.g., equivalent diameter) of the microdots is less than 5 µm, and the center-to-center distance between adjacent microdots is less than 10 µm; each microdot is coupled with one kind of oligonucleotide probe, each kind of oligonucleotide probe comprises at least one copy; the oligonucleotide probe in the direction from 5' to 3' comprises or consists of: a consensus sequence X1, a tag sequence Y and a consensus sequence X2, wherein,
   oligonucleotide probes coupled to different microdots have different tag sequences Y;
(2) contacting the one or more cells with the solid support of the nucleic acid array, whereby each cell individually occupies at least one microdot in the nucleic acid array (i.e., each cell is individually contacted with at least one microdot in the nucleic acid array), and allowing the first binding molecule of the cell to interact with the first label molecule of the solid support; wherein,
   before or after contacting the one or more cells with the nucleic acid array, performing a pretreatment, including reverse transcription, on an RNA (e.g., an mRNA) of the one or more cells to generate a first nucleic acid molecule population;
   and,
(3) associating the first nucleic acid molecule population derived from each cell obtained in the previous step with an oligonucleotide probe coupled to the microdot occupied by the cell from which the first nucleic acid molecule population is derived, thereby generating a second nucleic acid molecule population labeled with the tag sequence Y.

In some embodiments, the center-to-center distance between adjacent microdots is less than 10 µm, less than 5 µm, less than 1 µm, less than 0.5 µm, less than 0.1 µm, less than 0.05 µm, or less than 0.01 µm; and, the size (e.g., equivalent diameter) of the microdots is less than 5 µm, less than 1 µm, less than 0.3 µm, less than 0.5 µm, less than 0.1 µm, less than 0.05 µm, less than 0.01 µm, or less than 0.001 µm.

In some embodiments, the center-to-center distance between adjacent microdots is 0.5 µm to 1 µm, such as 0.5 µm to 0.9 µm, 0.5 µm to 0.8 µm.

In some embodiments, the size (e.g., equivalent diameter) of the microdots is 0.001 µm to 0.5 µm (e.g., 0.01 µm to 0.1 µm, 0.01 µm to 0.2 µm, 0.2 µm to 0.5 µm, 0.2 µm to 0.4 µm, 0.2 µm to 0.3 µm).

In certain embodiments, the first binding molecule is capable of forming a specific interaction pair or a non-specific interaction pair with the first label molecule.

In certain embodiments, the interaction pair is selected from the group consisting of an interaction pair of positive and negative charges, affinity interaction pair (e.g., biotin/avidin, biotin/streptavidin, antigen/antibody, receptor/ligand, enzyme/cofactor), a pair of molecules capable of undergoing click chemical reaction (e.g., alkynyl-containing compound/azide compound), N-hydroxysulfosuccinate (NHS) ester/amino-containing compound, and any combination thereof.

In certain embodiments, the first label molecule is polylysine, the first binding molecule is a protein capable of binding to polylysine; the first label molecule is an antibody, and the first binding molecule is an antigen capable of binding to the antibody; the first label molecule is an amino-containing compound, and the first binding molecule is a N-hydroxysulfosuccinate (NHS) ester; or, the first label molecule is biotin, and the first binding molecule is streptavidin.

In certain embodiments, the first binding molecule is naturally present in the cell.

In certain embodiments, the first binding molecule is unnaturally present in the cell.

In certain embodiments, the method further comprises a step of binding the first binding molecule to the one or more cells or causing the one or more cells to express the first binding molecule, so as to provide the cell sample of step (1).

In certain embodiments, the method further comprises a step of binding the first label molecule to the solid support, so as to provide the nucleic acid array of step (1).

### Scheme I

In some embodiments, in step (2), the pretreatment comprises:
(i) using a primer I-A to reverse-transcribe the RNA (e.g., mRNA) of the one or more cells to generate an extension product as a first nucleic acid molecule to be labeled, thereby generating a first nucleic acid molecule population; wherein, the primer I-A comprises a consensus sequence A and a capture sequence A, the capture sequence A is capable of annealing to the RNA (e.g., mRNA) to be captured and initiating an extension reaction; the consensus sequence A is located upstream of the capture sequence A (e.g., located at the 5' end of the primer I-A);
   or,
(ii) (a) using a primer I-A to reverse-transcribe the RNA (e.g., mRNA) of the one or more cells to generate a cDNA strand, in which the cDNA strand comprises a cDNA sequence that is formed by reverse transcription primed by the primer I-A and is complementary to the RNA (e.g., mRNA), and a 3'-end overhang; wherein, the primer I-A comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., located at the 5' end of the primer 1-A); and, (b) annealing a primer I-B to the cDNA strand generated in (a) and initiating an extension reaction to generate a first extension product as a first nucleic acid molecule to be labeled, thereby generating a first nucleic acid molecule population; wherein, the primer 1-B comprises a consensus sequence B, a complementary sequence of the 3'-end overhang, and optionally a tag sequence B; the complementary sequence of the 3'-end overhang is located at the 3' end of the primer 1-B; the consensus sequence B is located upstream of the complementary sequence of the 3'-end overhang (e.g., located at the 5' end of the primer 1-B);
   or,
(iii) (a) using a primer I-A' to reverse-transcribe the RNA (e.g., mRNA) of the one or more cells to generate a cDNA strand, in which the cDNA strand comprises a cDNA sequence that is formed by reverse transcription primed by the primer I-A' and is complementary to the RNA (e.g., mRNA), and a 3'-end overhang; wherein, the primer I-A' comprises a capture sequence A, and the capture sequence A is capable of annealing to the RNA (e.g., mRNA) to be captured and initiating an extension reaction; (b) annealing a primer I-B to the cDNA strand generated in (a), and performing an extension reaction to generate a first extension product; wherein, the primer 1-B comprises a consensus sequence B, a complementary sequence of the 3'-end overhang, and optionally a tag sequence B; the complementary sequence of the 3'-end overhang is located at the 3' end of the primer 1-B; the consensus sequence B is located upstream of the complementary sequence of the 3'-end overhang (e.g., located at the 5' end of the primer I-B); and, (c) providing an extension primer, using the first extension product as a template to perform an extension reaction to generate a second extension product as a first nucleic acid molecule to be labeled, thereby generating a first nucleic acid molecule population;
and,
in step (3), generating a second nucleic acid molecule population labeled by the tag sequence Y by associating the first nucleic acid molecule population derived from each cell obtained in the previous step with an oligonucleotide probe coupled to the microdot occupied by the cell from which the first nucleic acid molecule population is derived, which comprises:
under a condition that allows annealing, contacting a bridging oligonucleotide I with the first nucleic acid molecule that is derived from each cell and obtained in step (2) and the oligonucleotide probe coupled to the microdot occupied by the cell, annealing (e.g., in situ annealing) the bridging oligonucleotide I to the first nucleic acid molecule that is derived from each cell and obtained in step (2) and the oligonucleotide probe coupled to the microdot occupied by the cell, and ligating the first nucleic acid molecule and the oligonucleotide probe on the array annealed with the bridging oligonucleotide I to obtain a ligation product as a second nucleic acid molecule with a positioning tag, thereby generating a second nucleic acid molecule population;
wherein, the bridging oligonucleotide I comprises: a first region and a second region, and optionally a third region located between the first region and the second region, the first region is located upstream of the second region (e.g., located 5' of the second region); wherein,
the first region is capable of annealing to the whole or a part of the consensus sequence A of the primer I-A described in step (2)(i) or step (2)(ii) or annealing to the whole or a part of the consensus sequence B of the primer I-B described in step (2)(iii);
the second region is capable of annealing to the whole or a part of the consensus sequence X2.

In certain embodiments, in step (3), the first region and the second region of the bridging oligonucleotide I are directly adjacent, and the ligation of the first nucleic acid molecule to the oligonucleotide probe comprises: using a nucleic acid ligase to ligate the nucleic acid molecule hybridized with the first region and the nucleic acid molecule hybridized with the second region of the same bridging oligonucleotide I, to obtain a ligation product as a second nucleic acid molecule with a positioning tag; or,
the bridging oligonucleotide I comprises a first region, a second region and a third region located between them, and the ligation of the first nucleic acid molecule to the oligonucleotide probe comprises: using a nucleic acid polymerase to perform a polymerization reaction with the third region as a template, and using a nucleic acid ligase to ligate the nucleic acid molecule hybridized with the first region and the nucleic acid molecule hybridized with the third region and the second region of the same bridging oligonucleotide I, to obtain a ligation product as a second nucleic acid molecule with a positioning tag; preferably, the nucleic acid polymerase has no 5' to 3' exonucleolytic activity or strand displacement activity.

In certain embodiments, each kind of oligonucleotide probe comprises one copy.

In certain embodiments, each kind of oligonucleotide probe comprises multiple copies.

It is easy to understand that when each kind of oligonucleotide probe comprises one copy, each microdot is coupled with one oligonucleotide probe, and the oligonucleotide probes of different microdots have different tag sequences Y; when each kind of oligonucleotide probe comprises multiple copies, each microdot is coupled with multiple oligonucleotide probes, and the oligonucleotide probes in the same microdot have the same tag sequence Y, and the oligonucleotide probes of different microdots have different tag sequences Y.

In certain embodiments, the solid support comprises a plurality of microdots, each microdot is coupled with one kind of oligonucleotide probe, and each kind of oligonucleotide probe may comprise one or more copies.

In certain embodiments, the solid support comprises a plurality of (e.g., at least 10, at least 10², at least 10³, at least 10⁴, at least 10⁵, at least 10⁶, at least 10⁷, at least 10⁸, or more) microdots. In certain embodiments, the solid support comprises at least 10⁴ (e.g., at least 10⁴, at least 10⁵, at least 10⁶, at least 10⁷, at least 10⁸, at least 10⁹, at least 10¹⁰, at least 10¹¹, or at least 10¹²) microdots/mm²

### Embodiments comprising step (1), step (2)(i) and step (3)

In certain embodiments, the method comprises step (1), step (2)(i) and step (3); wherein, the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide I, and the sequence of the first nucleic acid molecule to be labeled.

In certain embodiments, in step (2)(i) of the method, the capture sequence A is a random oligonucleotide sequence.

In some embodiments, in step (3), the ligation product derived from each copy of the oligonucleotide probe coupled to the same microdot has a different capture sequence A, and the capture sequence A serves as a unique molecular identifier (UMI) of the second nucleic acid molecule.

In certain embodiments, the extension product (the first nucleic acid molecule to be labeled) in step (2)(i) comprises from 5' to 3': the consensus sequence A, and a cDNA sequence that is formed by reverse transcription primed by the primer I-A and complementary to the RNA.

In certain embodiments, in step (2)(i) of the method, the capture sequence A is a poly(T) sequence or a specific sequence targeting a target nucleic acid.

In certain embodiments, the primer I-A further comprises a tag sequence A, such as a random oligonucleotide sequence.

In certain embodiments, the capture sequence A is located at the 3' end of the primer I-A, and the consensus sequence A is located upstream of the tag sequence A (e.g., located at the 5' end of the primer 1-A).

In certain embodiments, in step (3), the ligation product derived from each copy of the oligonucleotide probe coupled to the same microdot has a different tag sequence A as a UMI.

In some embodiments, the extension product in step (2)(i) comprises from 5' to 3': the consensus sequence A, the tag sequence A, and a cDNA sequence that is formed by reverse transcription primed by the primer I-A and complementary to the RNA.

### Embodiments comprises step (1), step (2)(ii) and step (3)

In some embodiments, the method comprises step (1), step (2)(ii) and step (3); wherein the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide I, and the sequence of the first nucleic acid molecule to be labeled.

In certain embodiments, in step (2)(ii)(a) of the method, the capture sequence A is a random oligonucleotide sequence.

In some embodiments, in step (3), the ligation product derived from each copy of the oligonucleotide probe coupled to the same microdot has a different capture sequence A, and the capture sequence A serves as a unique molecular identifier (UMI) of the second nucleic acid molecule.

In certain embodiments, the first extension product (the first nucleic acid molecule to be labeled) in step (2)(ii) comprises from 5' to 3': the consensus sequence A, a cDNA sequence that is formed by reverse transcription primed by the primer I-A and complementary to the RNA, the 3'-end overhang sequence, optionally a complementary sequence of the tag sequence B, and a complementary sequence of the consensus sequence B.

In certain embodiments, in step (2)(ii)(a), the capture sequence A is a poly(T) sequence or a specific sequence targeting a target nucleic acid.

In certain embodiments, the primer I-A further comprises a tag sequence A, such as a random oligonucleotide sequence.

In certain embodiments, the capture sequence A is located at the 3' end of the primer I-A, and the consensus sequence A is located upstream of the tag sequence A (e.g., located at the 5' end of the primer 1-A).

In certain embodiments, in step (3), the ligation product derived from each copy of the oligonucleotide probe coupled to the same microdot has a different tag sequence A as a UMI.

In certain embodiments, the first extension product (the first nucleic acid molecule to be labeled) in step (2)(ii) comprises from 5' to 3': the consensus sequence A, the tag sequence A, a cDNA sequence that is formed by reverse transcription primed by the primer I-A and complementary to the RNA, the 3'-end overhang sequence, optionally a complementary sequence of the tag sequence B, and a complementary sequence of the consensus sequence B.

In certain embodiments, in the method, the primer I-A comprises a 5' phosphate at the 5'-end.

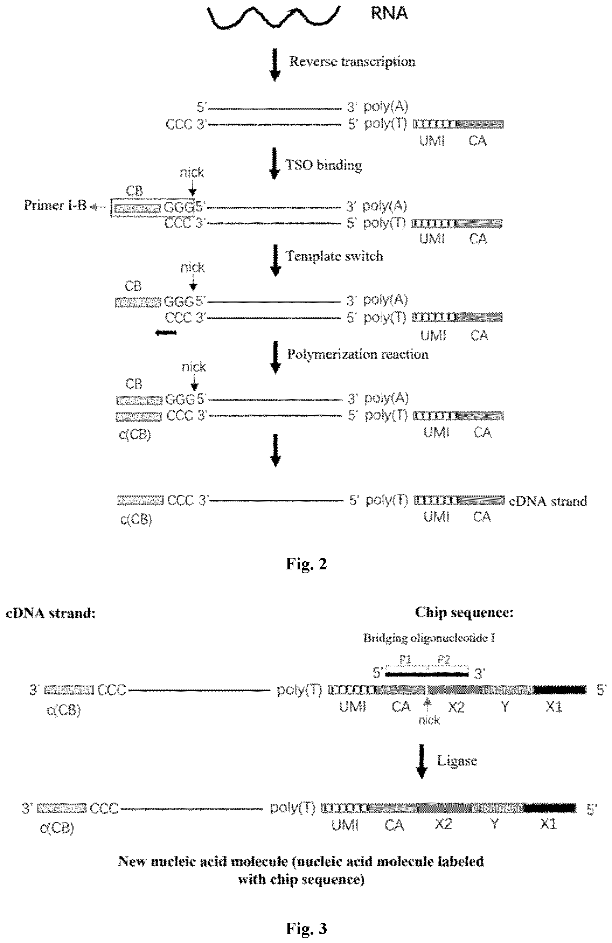

An exemplary embodiment of the present application comprising step (1), step (2)(ii) and step (3) is described in detail as follows:
I. An exemplary embodiment of preparing a cDNA strand using an RNA (e.g., mRNA) in a sample as a template comprises the following steps (as shown in Fig. 2):
   (1) A reverse transcriptase (e.g., a reverse transcriptase with terminal deoxynucleotidyl transferase activity) and a primer I-A are used to reverse-transcribe an RNA molecule (e.g., mRNA molecule) in a permeabilized cell sample to generate a cDNA, and an overhang (e.g., an overhang containing three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.
      The primer I-A comprises a poly(T) sequence and a consensus sequence A (marked as CA in the figure). In certain embodiments (e.g., when the method is used for constructing a 3' transcriptome library), the primer I-A further comprises a unique molecular identifier (UMI) sequence. Normally, the poly(T) sequence is located at the 3' end of the primer I-A to initiate the reverse transcription. In a preferred embodiment, the UMI sequence is located upstream (e.g., 5') of the poly(T) sequence, and the consensus sequence A is located upstream (e.g., 5') of the UMI sequence.
   (2) A primer 1-B, which comprises a consensus sequence B (marked as CB in the figure), is used to anneal to or hybridize with the cDNA strand; subsequently, the nucleic acid fragment hybridized with or annealed to the primer I-B can be extended using the consensus sequence B as a template under the presence of a nucleic acid polymerase to add a complementary sequence of the consensus sequence B to the 3' end of the cDNA strand, thereby generating a nucleic acid molecule that carries the consensus sequence A and the tag sequence A at the 5' end, and a complementary sequence of the consensus sequence B at the 3' end.

The primer I-B may comprise a sequence complementary to the 3'-end overhang of the cDNA strand. For example, when the cDNA strand comprises at the 3' end an overhang of three cytosine nucleotides, the primer I-B may comprise GGG at its 3' end. In addition, the nucleotides of the primer I-B can also be modified (e.g., the primer I-B can be modified to comprise one or more locked nucleic acids) to enhance binding affinity for the complementary pairing between the primer I-B and the 3'-end overhang of the cDNA strand.

Without being limited by any theory, various suitable nucleic acid polymerases (e.g., DNA polymerase or reverse transcriptase) can be used to perform the extension reaction, as long as they can use a partial sequence of the primer I-B as a template to extend the hybridized or annealed nucleic acid fragment (reverse transcription product). In certain exemplary embodiments, the hybridized or annealed nucleic acid fragment (reverse transcription product) can be extended using the same reverse transcriptase used in the aforementioned reverse transcription step.

In certain preferred embodiments, step (2) and step (1) are performed simultaneously.

In certain embodiments, the method optionally further comprises step (3): adding RNase H to digest the RNA strand in the RNA/cDNA hybrid to form a cDNA single strand.

In certain preferred embodiments, the method does not comprise step (3).

An exemplary structure of the cDNA strand prepared by the above exemplary embodiment comprises: the consensus sequence A, the UMI sequence, a sequence complementary to the sequence of RNA (e.g., mRNA), and a complementary sequence of the consensus sequence B.

II. An exemplary embodiment of labeling the 5' end of a cDNA strand with an oligonucleotide probe (also referred as a chip sequence) (that is, ligating the 5' end of the cDNA strand to the 3' end of the chip sequence) to form a new nucleic acid molecule containing the chip sequence information (i.e., a nucleic acid molecule labeled with the chip sequence) comprises the following steps (as shown in Fig. 3):
A bridging oligonucleotide I is provided, which comprises at 5' end a sequence (a first region, P1) that is at least partially complementary to the 5' end of the cDNA sequence (e.g., at least partially complementary to the consensus sequence A (CA)), and which comprises at 3' end a sequence (a second region, P2) that is at least partially complementary to the 3' end of the chip sequence (e.g., at least partially complementary to the consensus sequence X2).

In certain preferred embodiments, the P1 and P2 sequences in the bridging oligonucleotide I are directly adjacent without an intermediate nucleotide between them.

In certain preferred embodiments, the P1 sequence, P2 sequence, consensus sequence A, and consensus sequence X2 each independently have a length of 20 to 100 nt (e.g., 20 to 70 nt). The bridging oligonucleotide I is annealed to or hybridized with the oligonucleotide probe and the cDNA strand, and then the 5' end of the cDNA strand is ligated to the 3' end of the oligonucleotide probe by a DNA ligase and/or a DNA polymerase, thereby forming a new nucleic acid molecule comprising the sequence information of the oligonucleotide probe (i.e., a nucleic acid molecule labeled with the oligonucleotide probe). In certain preferred embodiments, the DNA polymerase has no 5' to 3' exonucleolytic activity or strand displacement activity.

An exemplary structure of the new nucleic acid molecule comprising the chip sequence information formed by the above exemplary embodiment comprises: the consensus sequence X1, the tag sequence Y, the consensus sequence X2, the consensus sequence A, the UMI sequence, and a sequence complementary to the sequence of the RNA (e.g., mRNA) and a complementary sequence of the consensus sequence B.

### Embodiments comprising step (1), step (2)(iii) and step (3)

In some embodiments, the method comprises step (1), step (2)(iii) and step (3); wherein, the ligation product obtained in step (3) is taken as the second nucleic acid molecule with a positioning tag, which comprises from 5' to 3': the consensus sequence X1, the tag sequence Y, the consensus sequence X2, optionally a complementary sequence of the third region of the bridging oligonucleotide I, and the sequence of the first nucleic acid molecule to be labeled.

In certain embodiments, in step (2)(iii)(c) of the method, the extension primer is the primer I-B or a primer B", and the primer B" is capable of annealing to a complementary sequence or partial sequence thereof of the consensus sequence B and initiating the extension reaction.

In certain embodiments, in step (2)(iii)(c), the extension primer is the primer B".

In certain embodiments, in step (2)(iii)(a) of the method, the capture sequence A of the primer I-A' is a random oligonucleotide sequence.

In certain embodiments, in step (2)(iii)(b), the primer 1-B comprises the consensus sequence B, a complementary sequence of the 3'-end overhang, and the tag sequence B.

In certain embodiments, the first extension product comprises from 5' to 3': a cDNA sequence that is formed by reverse transcription primed by the primer I-A' and complementary to the RNA sequence, the 3'-end overhang sequence, a complementary sequence of the tag sequence B, and a complementary sequence of the consensus sequence B; wherein the complementary sequence of the tag sequence B serves as a unique molecular identifier (UMI) of the second nucleic acid molecule.

In certain embodiments, in step (2)(iii)(c), the second extension product (the first nucleic acid molecule to be labeled) comprises from 5' to 3': the consensus sequence B or a partial sequence of its 3' end, the tag sequence B, a complementary sequence of the 3'-end overhang sequence, and a complementary sequence of the cDNA sequence in the first extension product; wherein the tag sequence B serves as a unique molecular identifier (UMI) of the second nucleic acid molecule.

In certain embodiments, in step (2)(iii)(a) of the method, the capture sequence A of the primer I-A' is a poly(T) sequence or a specific sequence targeting a target nucleic acid.

In certain embodiments, the primer I-A' further comprises a tag sequence A, such as a random oligonucleotide sequence, and a consensus sequence A.

In certain embodiments, the capture sequence A is located at the 3' end of the primer I-A'.

In certain embodiments, the consensus sequence A is located upstream of the capture sequence A (e.g., at the 5' end of the primer I-A').

In certain embodiments, the primer 1-B comprises the consensus sequence B, a complementary sequence of the 3'-end overhang, and the tag sequence B.

In certain embodiments, in step (2)(iii)(b), the first extension product comprises from 5' to 3': the consensus sequence A, optionally the tag sequence A, a cDNA sequence that is formed by reverse transcription primed by the primer I-A' and complementary to the RNA, the 3'-end overhang sequence, a complementary sequence of the tag sequence B, and a complementary sequence of the consensus sequence B.

In certain embodiments, in step (2)(iii)(c), the second extension product (the first nucleic acid molecule to be labeled) comprises from 5' to 3': the consensus sequence B or a partial sequence of its 3' end, the tag sequence B, a complementary sequence of the 3'-end overhang sequence, a complementary sequence of the cDNA sequence in the first extension product, and optionally a complementary sequence of the tag sequence A, and a complementary sequence of the consensus sequence A.

In some embodiments, in step (3), the ligation product derived from each copy of the oligonucleotide probe coupled to the same microdot has a different tag sequence B as a UMI.

In certain embodiments, the extension primer comprises a 5' phosphate at the 5'-end.

In certain embodiments, before step (2)(iii)(c), the method further comprises processing (e.g., heat-processing) the product of step (2)(iii)(a) or step (2)(iii)(b) to remove RNA.

In certain embodiments, in step (2)(iii)(b) of the method, the cDNA strand anneals through its 3'-end overhang to the primer 1-B, and, the cDNA strand is extended using the primer I-B as a template to generate the first extension product under the presence of a nucleic acid polymerase (e.g., a DNA polymerase or reverse transcriptase).

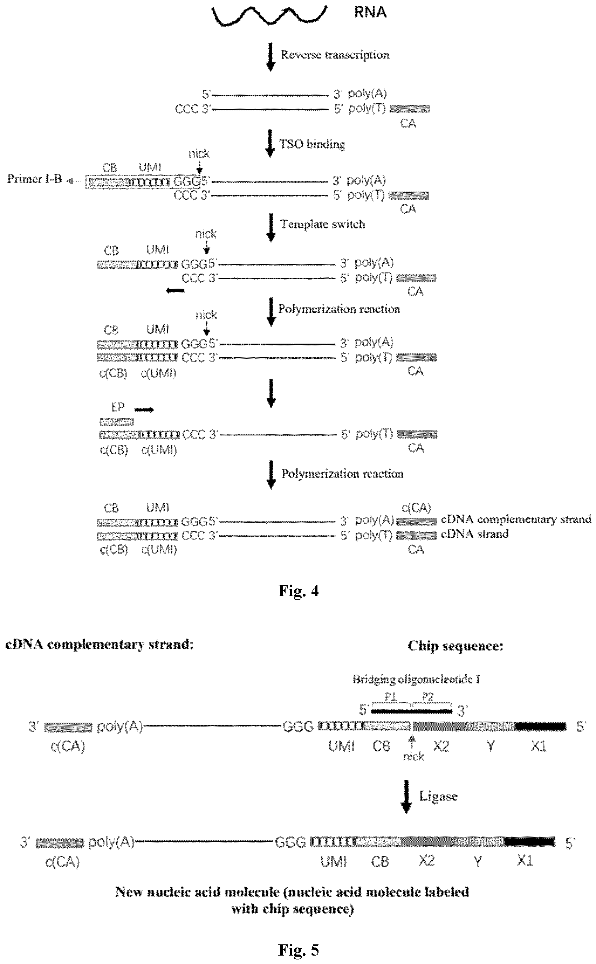

An exemplary embodiment of the present application comprising step (1), step (2)(iii) and step (3) is described in detail as follows:
I. An exemplary embodiment of preparing a complementary strand of a cDNA strand using an RNA (e.g., mRNA) in a sample as a template comprises the following steps (as shown in Fig. 4):
   (1) A reverse transcriptase (e.g., a reverse transcriptase with terminal deoxynucleotidyl transferase activity) and a primer I-A' are used to reverse-transcribe an RNA molecule (e.g., an mRNA molecule) in a permeabilized sample to generate a cDNA, and an overhang (e.g., an overhang comprising three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.
      The reverse transcription primer I-A' comprises a poly(T) sequence and a consensus sequence A (CA). Normally, the poly(T) sequence is located at the 3' end of the primer I-A' to initiate the reverse transcription.
   (2) A primer I-B is used to anneal to or hybridize with the cDNA strand, in which the primer 1-B comprises a consensus sequence B (CB) and a complementary sequence of the 3'-end overhang of the cDNA. In certain embodiments (e.g., when the method is used for constructing a 5' transcriptome library), the primer I-B further comprises a unique molecular identifier (UMI) sequence. Subsequently, the nucleic acid fragment hybridized with or annealed to the primer I-B can be extended under the presence of a nucleic acid polymerase using the consensus sequence B and UMI sequence as a template to add a complementary sequence of the consensus sequence B and a complementary sequence of the UMI sequence to the 3' end of the cDNA strand, thereby generating a nucleic acid molecule that carries the consensus sequence A at the 5' end, and carries a complementary sequence of the consensus sequence B and a complementary sequence of the UMI molecule at the 3' end.

When the cDNA strand comprises at 3' end an overhang of three cytosine nucleotides, the primer I-B can comprise GGG at its 3' end. In addition, the nucleotides of the primer I-B can also be modified (e.g., the primer I-B can be modified to comprise one or more locked nucleic acids) to enhance the binding affinity for the complementary pairing between the primer I-B and the 3'-end overhang of the cDNA strand.

Without being limited by any theory, various suitable nucleic acid polymerases (e.g., DNA polymerase or reverse transcriptase) can be used to perform the extension reaction, as long as they can extend the annealed or hybridized nucleic acid fragment (reverse transcription product) using the sequence of primer I-B or a partial sequence thereof as a template. In certain exemplary embodiments, the annealed or hybridized nucleic acid fragment (reverse transcription product) can be extended using the same reverse transcriptase used in the aforementioned reverse transcription step.

In certain preferred embodiments, step (2) and step (1) are performed simultaneously.

In certain embodiments, the method optionally further comprises step (3): adding RNase H to digest the RNA strand in the RNA/cDNA hybrid to form a cDNA single strand.

In certain preferred embodiments, the method does not comprise step (3).

(4) An extension primer is used to perform an extension reaction using the cDNA single strand obtained in step (3) as a template to obtain an extension product; the extension primer is capable of annealing to a complementary sequence or partial sequence thereof of the consensus sequence B, and initiating the extension reaction.

In certain embodiments, the extension primer is the same as the primer 1-B.

An exemplary structure comprising a complementary strand of a cDNA strand prepared by the above exemplary embodiment comprises: the consensus sequence B, the UMI sequence, a sequence complementary to the 3'-end overhang sequence of the cDNA, a complementary sequence of the cDNA sequence, and a complementary sequence of the consensus sequence A.

II. An exemplary embodiment of using an oligonucleotide probe (also referred as a chip sequence) to label a 5' end of a complementary strand of a cDNA strand (that is, ligating the 5' end of the complementary strand of the cDNA strand to the 3' end of the chip sequence) to generate a new nucleic acid molecule comprising the chip sequence information (i.e., a nucleic acid molecule labeled with the chip sequence) comprises the following steps (as shown in Fig. 5):
A bridging oligonucleotide I is provided, which comprises at 5' end a sequence (a first region, P1) that is at least partially complementary to a consensus sequence B (CB), and comprises at 3' end a sequence (a second region, P2) that is at least partially complementary to a consensus sequence X2.

In certain preferred embodiments, the P1 and P2 sequences in the bridging oligonucleotide I are directly adjacent without an intermediate nucleotide between them.

In certain preferred embodiments, the P1 sequence and P2 sequence each independently have a length of 20 to 100 nt (e.g., 20 to 70 nt).

The bridging oligonucleotide I is annealed to or hybridized with the oligonucleotide probe and a complementary strand of the cDNA strand, and then the 5' end of the complementary strand of the cDNA strand is ligated to the 3' end of the chip sequence by a DNA ligase and/or a DNA polymerase to form a new nucleic acid molecule comprising the sequence information of the oligonucleotide probe (i.e., a nucleic acid molecule labeled with the oligonucleotide probe). In certain preferred embodiments, the DNA polymerase has no 5' to 3' exonucleolytic activity or strand displacement activity.

An exemplary structure of the new nucleic acid molecule comprising the chip sequence information formed by the above exemplary embodiment comprises: the consensus sequence X1, the tag sequence Y, the consensus sequence X2, the consensus sequence B, the UMI sequence, a complementary sequence of the cDNA sequence, and a complementary sequence of the consensus sequence A.

### Scheme II

In some embodiments, in step (2), the pretreatment comprises:
(i) (a) using a primer 11-A to reverse-transcribe the RNA (e.g., mRNA) of the one or more cells to generate a cDNA strand, in which the cDNA strand comprises a cDNA sequence that is formed by reverse transcription primed by the primer 11-A and complementary to the RNA (e.g., mRNA), and a 3'-end overhang; wherein, the primer 11-A comprises a capture sequence A, and the capture sequence A is capable of annealing to the RNA (e.g., mRNA) to be captured and initiating the extension reaction; and, (b) annealing a primer 11-B to the cDNA strand generated in (a) and performing an extension reaction to generate a first extension product as a first nucleic acid molecule to be labeled, thereby generating the first nucleic acid molecule population; wherein, the primer 11-B comprises a consensus sequence B, a complementary sequence of the 3'-end overhang, and optionally a tag sequence B; the complementary sequence of the 3'-end overhang is located at the 3' end of the primer 11-B; the consensus sequence B is located upstream of the complementary sequence of the 3'-end overhang (e.g., located at the 5' end of the primer II-B); or,
(ii) (a) using a primer II-A' to reverse-transcribe the RNA (e.g., mRNA) of the one or more cells to generate a cDNA strand; in which the cDNA strand comprises a cDNA sequence that is formed by reverse transcription primed by the primer II-A' and complementary to the RNA (e.g., mRNA), and a 3'-end overhang; wherein, the primer II-A' comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., located at the 5' end of the primer II-A'); (b) annealing a primer II-B' to the cDNA strand generated in (a), and performing an extension reaction to generate a first extension product; wherein, the primer 11-B' comprises a consensus sequence B and a complementary sequence of the 3'-end overhang, and optionally a tag sequence B; the complementary sequence of the 3'-end overhang is located at the 3' end of the primer II-B'; the consensus sequence B is located upstream of the complementary sequence of the 3'-end overhang (e.g., located at the 5' end of the primer II-B'); and, (c) providing an extension primer, using the first extension product as a template to perform an extension reaction to generate a second extension product as a first nucleic acid molecule to be labeled, thereby generating the first nucleic acid molecule population;
furthermore, in step (3), generating a second nucleic acid molecule population labeled by the tag sequence Y by associating the first nucleic acid molecule population derived from each cell obtained in the previous step with the oligonucleotide probe coupled to the microdot occupied by the cell from which the first nucleic acid molecule population is derived, which comprises:
(i) annealing (e.g., in-situ annealing) the first nucleic acid molecule derived from each cell obtained in step (2) to the oligonucleotide probe coupled to the microdot occupied by the cell, by applying an annealing condition to the product of step (2), and performing an extension reaction to generate an extension product as a second nucleic acid molecule with a positioning tag, thereby generating the second nucleic acid molecule population; wherein, the consensus sequence X2 or partial sequence thereof of the oligonucleotide probe is (a) capable of annealing to a complementary sequence or partial sequence thereof of the consensus sequence B of the first extension product obtained in step (2)(i), or (b) capable of annealing to a complementary sequence or partial sequence thereof of the consensus sequence A of the second extension product obtained in step (2)(ii), or,
(ii) under a condition that allows annealing, contacting a bridging oligonucleotide pair with the first nucleic acid molecule derived from each cell obtained in step (2) and the oligonucleotide probe coupled to the microdot occupied by the cell, and annealing (e.g., in-situ annealing) the bridging oligonucleotide pair to the first nucleic acid molecule derived from each cell obtained in step (2) and the oligonucleotide probe coupled to the microdot occupied by the cell,

wherein, the bridging oligonucleotide pair is composed of a bridging oligonucleotide II-I and a bridging oligonucleotide II-II, the bridging oligonucleotide II-I and the bridging oligonucleotide II-II each independently comprise: a first region, a second region, and optionally a third region located between the first region and the second region, and the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is (a) capable of annealing to a complementary sequence or partial sequence thereof of the consensus sequence B of the first extension product obtained in step (2)(i), or, (b) capable of annealing to a complementary sequence or partial sequence thereof of the consensus sequence A of the second extension product obtained in step (2)(ii);
wherein, among the bridging oligonucleotide pair to be contacted with the first nucleic acid molecule and the oligonucleotide probe, the bridging oligonucleotide II-I and the bridging oligonucleotide II-II of the bridging oligonucleotide pair each exist in a single-strand form, or, the bridging oligonucleotide II-I and the bridging oligonucleotide II-II of the bridging oligonucleotide pair are annealed to each other and exist in a partially double-strand form;
performing a ligation reaction: ligating the nucleic acid molecule hybridized with the first region and the nucleic acid molecule hybridized with the second region of the same bridging oligonucleotide 11-1, and/or, ligating the nucleic acid molecule hybridized with the first region and the nucleic acid molecule hybridized with the second region of the same bridging oligonucleotide II-II; and performing an extension reaction, to obtain a reaction product as a second nucleic acid molecule with a positioning tag, thereby generating a second nucleic acid molecule population; wherein the ligation reaction and the extension reaction are performed in any order.

In certain embodiments, in step (3)(ii):
(1) the first region and the second region of the bridging oligonucleotide II-I are directly adjacent, and the ligation of the nucleic acid molecule hybridized with the first region and the nucleic acid molecule hybridized with the second region of the same bridging oligonucleotide II-I comprises: using a nucleic acid ligase to ligate the nucleic acid molecule hybridized with the first region and the nucleic acid molecule hybridized with the second region of the same bridging oligonucleotide 11-1; or,
   the bridging oligonucleotide 11-1 comprises a first region, a second region and a third region between them, and the ligation of the nucleic acid molecule hybridized with the first region and the nucleic acid molecule hybridized with the second region of the same bridging oligonucleotide 11-1 comprises: using a nucleic acid polymerase (e.g., a nucleic acid polymerase with no 5' to 3' exonucleolytic activity or strand displacement activity) to perform a polymerization reaction with the third region as a template, and using a nucleic acid ligase to ligate the nucleic acid molecule hybridized with the first region and the nucleic acid molecule hybridized with the third region and the second region of the same bridging oligonucleotide 11-1;
   and/or
(2) the first region and the second region of the bridging oligonucleotide II-II are directly adjacent, and the ligation of the nucleic acid molecule hybridized with the first region and the nucleic acid molecule hybridized with the second region of the same bridging oligonucleotide II-II comprises: using a nucleic acid ligase to ligate the nucleic acid molecule hybridized with the first region and the nucleic acid molecule hybridized with the second region of the same bridging oligonucleotide II-II; or,
the bridging oligonucleotide II-II comprises a first region, a second region and a third region between them, and the ligation of the nucleic acid molecule hybridized with the first region and the nucleic acid molecule hybridized with the second region of the same bridging oligonucleotide II-II comprises: using a nucleic acid polymerase (e.g., a nucleic acid polymerase with no 5' to 3' exonucleolytic activity or strand displacement activity) to perform a polymerization reaction with the third region as a template, and using a nucleic acid ligase to ligate the nucleic acid molecule hybridized with the first region and the nucleic acid molecule hybridized with the third region and the second region of the same bridging oligonucleotide II-II.

In certain embodiments, the method comprises step (1), step (2)(i) and step (3); wherein, in step (2)(i)(b), the primer II-B comprises the consensus sequence B, a complementary sequence of the 3'-end overhang, and the tag sequence B.

In some embodiments, the first extension product in step (2)(i)(b) comprises from 5' to 3': a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, in step (3), the second nucleic acid molecule derived from each copy of the oligonucleotide probe coupled to the same microdot has a different tag sequence B as a UMI.

### Embodiments comprising step (1), step (2)(i) and step (3)(i)

In certain embodiments, the method comprises step (1), step (2)(i) and step (3)(i); wherein the consensus sequence X2 or partial sequence thereof is capable of annealing to a complementary sequence or partial sequence thereof of the consensus sequence B; the extension product obtained in step (3)(i) is taken as the labeled nucleic acid molecule, which comprises: a first strand comprising the sequence of the first nucleic acid molecule to be labeled, and/or, a second strand comprising the sequence of the oligonucleotide probe.

It is easy to understand that the "partial sequence of XX (sequence)" or "XX (sequence) partial sequence" refers to a nucleotide sequence of at least one segment of "XX (sequence)".

For example, the consensus sequence X2 is capable of annealing in its entire nucleotide sequence to a complementary sequence or a partial segment thereof of the consensus sequence B, and the consensus sequence X2 is also capable of annealing in its partial segment nucleotide sequence to a complementary sequence or a partial segment thereof of the consensus sequence B.

The "annealing" means that between the two nucleotide sequences that anneal to each other, each base in one nucleotide sequence is capable of being paired with a base in the other nucleotide sequence without mismatching or gap; or, between two nucleotide sequences that anneal to each other, most of the bases in one nucleotide sequence are capable of being paired with the bases in the other nucleotide sequence, which allows for mismatches or gaps (e.g., a mismatch or gap of one or several nucleotides). That is, two nucleotide sequences that are capable of being annealed can be either completely complementary or partially complementary. The description of "annealing" herein applies to the entire text of the present application unless otherwise indicated or otherwise clearly contradicted in the context.

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, a complementary sequence of the tag sequence Y, and a complementary sequence of the consensus sequence X1.

In certain embodiments, the second strand comprises from 5' to 3': the consensus sequence X1, the tag sequence Y, the consensus sequence X2, the tag sequence B, a complementary sequence of the 3'-end overhang sequence, a complementary sequence of the cDNA sequence that is formed by reverse transcription primed by the primer 11-A and complementary to the RNA.

### Embodiments comprising step (1), step (2)(i) and step (3)(i), for generating the first strand

In certain embodiments, the consensus sequence X2 or partial sequence thereof is capable of annealing to a complementary sequence or partial sequence thereof (e.g., a partial sequence at the 3' end thereof) of the consensus sequence B, and the complementary sequence of the consensus sequence B of the first extension product in step (2)(i) has a free 3' end.

In certain embodiments, the extension product obtained in step (3)(i) is the labeled nucleic acid molecule, which comprises the first strand.

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, a complementary sequence of the tag sequence Y, and a complementary sequence of the consensus sequence X1.

In certain embodiments, in step (3)(i), the oligonucleotide probe is incapable of initiating an extension reaction (e.g., the 3' end of the oligonucleotide probe is blocked).

In certain embodiments, in step (2)(i)(a) of the method, the capture sequence A of the primer 11-A is a random oligonucleotide sequence.

In some embodiments, the first extension product in step (2)(i)(b) comprises from 5' to 3': a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, a complementary sequence of the tag sequence Y, and a complementary sequence of the consensus sequence X1.

In certain embodiments, in step (2)(i)(a) of the method, the capture sequence A of the primer 11-A is a poly(T) sequence or a specific sequence targeting a target nucleic acid.

In certain embodiments, the primer II-A further comprises a consensus sequence A, and optionally a tag sequence A, such as a random oligonucleotide sequence.

In certain embodiments, the capture sequence A is located at the 3' end of the primer II-A.

In certain embodiments, the consensus sequence A is located upstream of the capture sequence A (e.g., located at the 5' end of the primer 11-A).

In certain embodiments, the first extension product in step (2)(i)(b) comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the first strand comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, a complementary sequence of the consensus sequence B, a complementary sequence of the tag sequence Y, and a complementary sequence of the consensus sequence X1.

### Embodiments comprising step (1), step (2)(i) and step (3)(i), for generating the second strand

In certain embodiments, the consensus sequence X2 or partial sequence thereof (e.g., a partial sequence at the 3' end thereof) is capable of annealing to a complementary sequence or partial sequence thereof of the consensus sequence B, and the consensus sequence X2 of the oligonucleotide probe has a free 3' end.

In certain embodiments, the extension product obtained in step (3)(i) is the labeled nucleic acid molecule, which comprises the second strand.

In certain embodiments, the second strand comprises from 5' to 3': the consensus sequence X1, the tag sequence Y, the consensus sequence X2, the tag sequence B, a complementary sequence of the 3'-end overhang sequence, and a complementary sequence of the cDNA sequence that is formed by reverse transcription primed by the primer 11-A and complementary to the RNA.

In certain embodiments, the first extension product obtained in step (2)(i) is incapable of initiating the extension reaction (e.g., the 3' end of the first extension product obtained in step (2)(i) is blocked).

In certain embodiments, in step (2)(i)(a) of the method, the capture sequence A of the primer 11-A is a random oligonucleotide sequence.

In some embodiments, the first extension product in step (2)(i)(b) comprises from 5' to 3': a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the second strand comprises from 5' to 3': the consensus sequence X1, the tag sequence Y, the consensus sequence X2, the tag sequence B, a complementary sequence of the 3'-end overhang sequence, and a complementary sequence of the cDNA sequence that is formed by reverse transcription primed by the primer 11-A and complementary to the RNA.

In certain embodiments, in step (2)(i)(a) of the method, the capture sequence A of the primer 11-A is a poly(T) sequence or a specific sequence targeting a target nucleic acid.

In certain embodiments, the primer II-A further comprises a consensus sequence A, and optionally a tag sequence A, such as a random oligonucleotide sequence.

In certain embodiments, the capture sequence A is located at the 3' end of the primer II-A.

In certain embodiments, the consensus sequence A is located upstream of the capture sequence A (e.g., located at the 5' end of the primer 11-A).

In certain embodiments, the first extension product in step (2)(i)(b) comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the second strand comprises from 5' to 3': the consensus sequence X1, the tag sequence Y, the consensus sequence X2, the tag sequence B, a complementary sequence of the 3'-end overhang sequence, a complementary sequence of the cDNA sequence that is formed by reverse transcription primed by the primer 11-A and complementary to the RNA, optionally a complementary sequence of the tag sequence A, and a complementary sequence of the consensus sequence A.

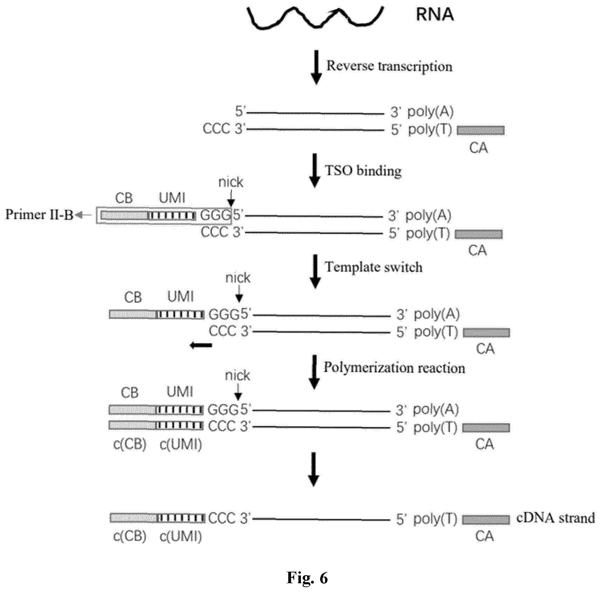

An exemplary embodiment of the present application comprising step (1), step (2)(i) and step (3)(i) is described in detail as follows:
I. An exemplary embodiment of using an RNA (e.g., mRNA) in a sample as a template to prepare a cDNA strand comprising close to its 3' end a complementary sequence of UMI comprises the following steps (as shown in Fig. 6):
   (1) A reverse transcriptase (e.g., a reverse transcriptase with terminal deoxynucleotidyl transferase activity) and a primer 11-A are used to reverse-transcribe an RNA molecule (e.g., mRNA molecule) in a permeabilized sample to generate a cDNA, and an overhang (e.g., an overhang comprising three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.

In certain embodiments, the primer II-A comprises a poly(T) sequence and a consensus sequence A (CA). Normally, the poly(T) sequence is located at the 3' end of the primer II-A to initiate the reverse transcription.

In certain embodiments, the primer II-A comprises a random oligonucleotide sequence that can be used to capture an RNA without a poly(A) tail. Typically, the random oligonucleotide sequence is located at the 3' end of the primer II-A to initiate the reverse transcription.

(2) A primer 11-B is used to anneal to or hybridize with the cDNA strand, and the primer II-B comprises a consensus sequence B (CB), a unique molecular identifier (UMI) sequence, and a complementary sequence of the 3'-end overhang of the cDNA. Subsequently, the nucleic acid fragment hybridized with or annealed to the primer 11-B can be extended under the presence of a nucleic acid polymerase using the UMI sequence and the consensus sequence B as a template, thereby generating a nucleic acid molecule that carries at its 3' end a complementary sequence of the UMI sequence and a complementary sequence of the consensus sequence B.

Typically, the consensus sequence B is located upstream of the UMI sequence (e.g., located 5' of the UMI sequence), and the sequence complementary to the 3'-end overhang of the cDNA strand is located at the 3' end of the primer II-B.

For example, when the cDNA strand comprises an overhang of three cytosine nucleotides at the 3' end, the primer II-B may comprise GGG at its 3' end. In addition, the nucleotides of the primer 11-B can also be modified (e.g., the primer 11-B can be modified to comprise one or more locked nucleic acids) to enhance the binding affinity for the complementary pairing between the primer II-B and the 3'-end overhang of the cDNA strand.

Without being limited by any theory, various suitable nucleic acid polymerases (e.g., DNA polymerase or reverse transcriptase) can be used to perform the extension reaction, as long as they can use the sequence of the primer II-B or partial sequence thereof as a template to extend the annealed or hybridized nucleic acid fragment (reverse transcription product). In certain exemplary embodiments, the annealed or hybridized nucleic acid fragment (reverse transcription product) can be extended using the same reverse transcriptase used in the aforementioned reverse transcription step.

In some embodiments, this step is performed simultaneously with step (1) (e.g., in the same reaction system).

In certain embodiments, the method optionally further comprises step (3): adding RNase H to digest the RNA strand in the RNA/cDNA hybrid to form a cDNA single strand.

In certain embodiments, the method does not comprise the step (3).

An exemplary structure of the cDNA strand prepared by the above exemplary embodiment comprises: the consensus sequence A, the cDNA sequence, the 3'-end overhang sequence, a complementary sequence of the UMI sequence, and a complementary sequence of the consensus sequence B.

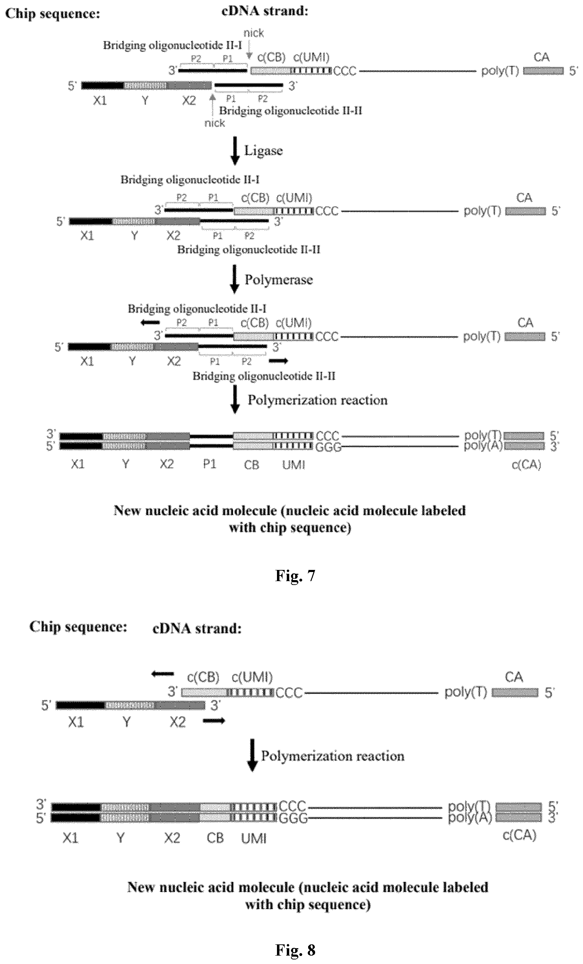

II. An exemplary embodiment of using a complementary sequence of an oligonucleotide probe (also referred as a chip sequence) to label the 3' end of a cDNA strand so as to form a new nucleic acid molecule comprising the chip sequence information (i.e., a nucleic acid molecule labeled by the chip sequence) comprises the following steps (as shown in Fig. 8):
in some embodiments, the consensus sequence X2 or a partial sequence thereof of the chip sequence is capable of annealing to a complementary sequence or partial sequence thereof of the consensus sequence B of the cDNA strand obtained in the above step I. Thereby a new nucleic acid molecule comprising the chip sequence information (i.e., a nucleic acid molecule labeled with the chip sequence) can be obtained by annealing or hybridizing the cDNA strand to the chip sequence, and performing an extension reaction under the presence of a polymerase.

An exemplary structure of the new nucleic acid molecule comprising the chip sequence information formed by the above exemplary embodiment comprises: a nucleic acid strand and/or a complementary nucleic acid strand thereof, wherein the nucleic acid strand comprises from 5' to 3': the consensus sequence A, the cDNA sequence, the 3'-end overhang sequence, a complementary sequence of the UMI sequence, a complementary sequence of the consensus sequence B, a complementary sequence of the tag sequence Y, and a complementary sequence of the consensus sequence X1.

### Embodiments comprising step (1), step (2)(i) and step (3)(ii)

In certain embodiments, the method comprises step (1), step (2)(i) and step (3)(ii); wherein the second region of the bridging oligonucleotide II-II is capable of annealing to a complementary sequence or partial sequence thereof of the consensus sequence B of the first extension product obtained in step (2)(i); and the reaction product obtained in step (3)(ii) is taken as the labeled nucleic acid molecule, which comprises: the first strand comprising the sequence of the first nucleic acid molecule to be labeled, and/or the second strand comprising the sequence of the oligonucleotide probe.

It is easy to understand that the second region of the bridging oligonucleotide II-II is capable of annealing to a complementary sequence or a partial segment thereof of the consensus sequence B of the first extension product obtained in step (2)(i).

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, optionally a complementary sequence of the third region of the bridging oligonucleotide II-II, the sequence of the bridging oligonucleotide 11-1, a complementary sequence of the tag sequence Y, and a complementary sequence of the consensus sequence X1.

In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, the tag sequence B, a complementary sequence of the 3'-end overhang sequence, a complementary sequence of the cDNA sequence that is formed by reverse transcription primed by the primer 11-A and complementary to the RNA.

### Embodiments comprising step (1), step (2)(i) and step (3)(ii), for generating the first strand

In certain embodiments, the second region of the bridging oligonucleotide II-II is capable of annealing to a complementary sequence or partial sequence thereof (e.g., the 3' end partial sequence thereof) of the consensus sequence B of the first extension product obtained in step (2)(i), and the second region of the bridging oligonucleotide II-I has a free 3' end.

In certain embodiments, the reaction product obtained in step (3)(ii) is the labeled nucleic acid molecule, which comprises the first strand.

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, optionally a complementary sequence of the third region of the bridging oligonucleotide II-II, the sequence of the bridging oligonucleotide 11-1, a complementary sequence of the tag sequence Y, and a complementary sequence of the consensus sequence X1.

In certain embodiments, the second region of the bridging oligonucleotide 11-1 is located at the 3' end of the bridging oligonucleotide 11-1.

In certain embodiments, the first region of the bridging oligonucleotide 11-1 is located at the 5' end of the bridging oligonucleotide 11-1.

In certain embodiments, the bridging oligonucleotide II-I does not comprise the third region, and/or the bridging oligonucleotide II-II does not comprise the third region.

In certain embodiments, the bridging oligonucleotide 11-1 comprises a 5' phosphate at the 5' end.

In certain embodiments, the bridging oligonucleotide 11-1 comprises a free -OH at the 3' end.

In certain embodiments, in step (3)(ii), the bridging oligonucleotide II-II is incapable of initiating an extension reaction (e.g., the 3' end of the bridging oligonucleotide II-II is blocked), and/or, the oligonucleotide probe is incapable of initiating an extension reaction (i.e., the 3' end of the oligonucleotide probe is blocked).

In some embodiments, in step (2)(i)(a) of the method, the capture sequence A of the primer 11-A is a random oligonucleotide sequence.

In some embodiments, the first extension product in step (2)(i)(b) of the method comprises from 5' to 3': a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the first strand comprises from 5' to 3': a cDNA sequence that is formed by reverse transcription primed by the primer 11-A and complementary to the RNA, the 3'-end overhang sequence, a complementary sequence of the tag sequence B, a complementary sequence of the consensus sequence B, optionally a complementary sequence of the third region of the bridging oligonucleotide II-II, the sequence of the bridging oligonucleotide 11-1, a complementary sequence of the tag sequence Y, and a complementary sequence of the consensus sequence X1.

In certain embodiments, in step (2)(i)(a), the capture sequence A of the primer 11-A is a poly(T) sequence or a specific sequence targeting a target nucleic acid.

In certain embodiments, the primer II-A further comprises a consensus sequence A, and optionally a tag sequence A, such as a random oligonucleotide sequence.

In certain embodiments, the capture sequence A is located at the 3' end of the primer II-A.

In certain embodiments, the first extension product in step (2)(i)(b) comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the first strand comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, a complementary sequence of the consensus sequence B, optionally a complementary sequence of the third region of the bridging oligonucleotide II-II, the sequence of the bridging oligonucleotide 11-1, a complementary sequence of the tag sequence Y, and a complementary sequence of the consensus sequence X1.

It is easy to understand that in step (3)(ii), after the bridging oligonucleotide II-I and the bridging oligonucleotide II-II are annealed to the oligonucleotide probe and the first nucleic acid molecule to be labeled which is at the corresponding position of the oligonucleotide probe, the ligation reaction for ligating the nucleic acid molecule hybridized with the first region and the nucleic acid molecule hybridized with the second region of the same bridging oligonucleotide II-I, and/or ligating the nucleic acid molecule hybridized with the first region and the nucleic acid molecule hybridized with the second region of the same bridging oligonucleotide II-II, and the extension reaction in step (3)(ii) can be performed in any order, as long as the second nucleic acid molecule with a positioning tag can be obtained.

For example, when the ligation reaction and the extension reaction are carried out in the same system, the first strand can be obtained by ligating the nucleic acid molecule hybridized with the first region and the nucleic acid molecule hybridized with the second region of the same bridging oligonucleotide II-II, and extending the bridging oligonucleotide II-I by an extension reaction. In this case, the polymerase used in the extension reaction preferably does not have strand displacement activity or 5' to 3' exonucleolytic activity.

For example, when the ligation reaction and the extension reaction are performed in different systems, and the ligation reaction is performed followed by the extension reaction, the first strand can be obtained in the following exemplary manner:
(A) ligating the nucleic acid molecule hybridized with the first region and the nucleic acid molecule hybridized with the second region of the same bridging oligonucleotide II-II, and extending the bridging oligonucleotide II-I by an extension reaction to obtain the first strand; wherein, the polymerase used for the extension reaction preferably has or does not have strand displacement activity or 5' to 3' exonucleolytic activity;
   or,
(B) ligating the nucleic acid molecule hybridized with the first region and the nucleic acid molecule hybridized with the second region of the same bridging oligonucleotide 11-1, and extending the first nucleic acid molecule to be labeled by an extension reaction to obtain the first strand; wherein, the polymerase used for the extension reaction preferably has strand displacement activity or 5' to 3' exonucleolytic activity.

For example, when the ligation reaction and the extension reaction are performed in different systems, and the extension reaction is performed followed by the ligation reaction, the first strand can be obtained by extending the bridging oligonucleotide II-I by an extension reaction, and then ligating the nucleic acid molecule hybridized with the first region and the nucleic acid molecule hybridized with the second region of the same bridging oligonucleotide II-II. In this case, the polymerase used in the extension reaction preferably does not have strand displacement activity or 5' to 3' exonucleolytic activity.

### Embodiments comprising step (1), step (2)(i) and step (3)(ii), for generating the second strand

In certain embodiments, the second region of the bridging oligonucleotide II-II is capable of annealing to a complementary sequence or partial sequence thereof of the consensus sequence B of the first extension product obtained in step (2)(i), and the second region of the bridging oligonucleotide II-II has a free 3' end.

In certain embodiments, the reaction product obtained in step (3)(ii) is the labeled nucleic acid molecule, which comprises the second strand.

In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, the tag sequence B, a complementary sequence of the 3'-end overhang sequence, and a complementary sequence of the cDNA sequence that is formed by reverse transcription primed by the primer 11-A and complementary to the RNA.

In certain embodiments, the second region of the bridging oligonucleotide II-II is located at the 3' end of the bridging oligonucleotide II-II.

In certain embodiments, the first region of the bridging oligonucleotide II-II is located at the 5' end of the bridging oligonucleotide II-II.

In certain embodiments, the bridging oligonucleotide II-I does not comprise the third region, and/or the bridging oligonucleotide II-II does not comprise the third region.

In certain embodiments, the bridging oligonucleotide II-II comprises a 5' phosphate at the 5' end.

In certain embodiments, the bridging oligonucleotide II-II comprises a free -OH at the 3' end.

In certain embodiments, in step (3)(ii), the bridging oligonucleotide II-I is incapable of initiating the extension reaction (e.g., the 3' end of the bridging oligonucleotide II-I is blocked), and/or, the first extension product obtained in step (2)(i) is incapable of initiating the extension reaction (e.g., the 3' end of the first extension product obtained in step (2)(i) is blocked).

In certain embodiments, in step (2)(i)(a), the capture sequence A of the primer 11-A is a random oligonucleotide sequence.

In some embodiments, the first extension product in step (2)(i)(b) comprises from 5' to 3': a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, the tag sequence B, a complementary sequence of the 3'-end overhang sequence, a complementary sequence of the cDNA sequence that is formed by reverse transcription primed by the primer 11-A and complementary to the RNA.

In certain embodiments, in step (2)(i)(a), the capture sequence A of the primer 11-A is a poly(T) sequence or a specific sequence targeting a target nucleic acid.

In certain embodiments, the primer II-A further comprises a consensus sequence A, and optionally a tag sequence A, such as a random oligonucleotide sequence.

In certain embodiments, the capture sequence A is located at the 3' end of the primer II-A.

In certain embodiments, the first extension product in step (2)(i)(b) comprises from 5' to 3': the consensus sequence A, optionally the tag sequence A, a cDNA sequence that is formed by reverse transcription primed by the primer 11-A and complementary to the RNA, the 3'-end overhang sequence, a complementary sequence of the tag sequence B, and a complementary sequence of the consensus sequence B.

In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, the tag sequence B, a complementary sequence of the 3'-end overhang sequence, a complementary sequence of the cDNA sequence that is formed by reverse transcription primed by the primer 11-A and complementary to the RNA, optionally a complementary sequence of the tag sequence A, and a complementary sequence of the consensus sequence A.

It is easy to understand that in step (3)(ii), after the bridging oligonucleotide II-I and the bridging oligonucleotide II-II are annealed to the oligonucleotide probe and the first nucleic acid molecule to be labeled which is at the corresponding position of the oligonucleotide probe, the ligation reaction for ligating the nucleic acid molecule hybridized with the first region and the nucleic acid molecule hybridized with the second region of the same bridging oligonucleotide II-I, and/or ligating the nucleic acid molecule hybridized with the first region and the nucleic acid molecule hybridized with the second region of the same bridging oligonucleotide II-II, and the extension reaction in step (3)(ii) can be performed in any order, as long as the second nucleic acid molecule with a positioning tag can be obtained.

For example, when the ligation reaction and the extension reaction are carried out in the same system, the second strand can be obtained by ligating the nucleic acid molecule hybridized with the first region and the nucleic acid molecule hybridized with the second region of the same bridging oligonucleotide 11-1, and extending the bridging oligonucleotide II-II by an extension reaction. In this case, the polymerase used in the extension reaction preferably does not have strand displacement activity or 5' to 3' exonucleolytic activity.

For example, when the ligation reaction and the extension reaction are performed in different systems, and the ligation reaction is performed followed by the extension reaction, the second strand can be obtained in the following exemplary manner:
(A) ligating the nucleic acid molecule hybridized with the first region and the nucleic acid molecule hybridized with the second region of the same bridging oligonucleotide 11-1, and extending the bridging oligonucleotide II-II by an extension reaction to obtain the second strand; wherein, the polymerase used for the extension reaction preferably has or does not have strand displacement activity or 5' to 3' exonucleolytic activity;
   or,
(B) ligating the nucleic acid molecule hybridized with the first region and the nucleic acid molecule hybridized with the second region of the same bridging oligonucleotide II-II, and extending the oligonucleotide probe by an extension reaction to obtain the second strand; wherein, the polymerase used for the extension reaction preferably has strand displacement activity or 5' to 3' exonucleolytic activity.

For example, when the ligation reaction and the extension reaction are performed in different systems, and the extension reaction is performed followed by the ligation reaction, the second strand can be obtained by extending the bridging oligonucleotide II-II by an extension reaction, and then ligating the nucleic acid molecule hybridized with the first region and the nucleic acid molecule hybridized with the second region of the same bridging oligonucleotide 11-1. In this case, the polymerase used in the extension reaction preferably does not have strand displacement activity or 5' to 3' exonucleolytic activity.

An exemplary embodiment of the present application comprising step (1), step (2)(i) and step (3)(ii) is described in detail as follows:
I. An exemplary embodiment of using an RNA (e.g., mRNA) in a sample as a template to prepare a cDNA strand comprises the following steps (as shown in Fig. 6):
   (1) A reverse transcriptase (e.g., a reverse transcriptase with terminal deoxynucleotidyl transferase activity) and a primer 11-A are used to reverse-transcribe an RNA molecule (e.g., mRNA molecule) in a permeabilized sample to generate a cDNA, and an overhang (e.g., an overhang comprising three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.

In certain embodiments, the primer II-A comprises a poly(T) sequence and a consensus sequence A (CA). Normally, the poly(T) sequence is located at the 3' end of the primer II-A to initiate the reverse transcription.

In certain embodiments, the primer II-A comprises a random oligonucleotide sequence that can be used to capture an RNA without a poly(A) tail. Typically, the random oligonucleotide sequence is located at the 3' end of the primer II-A to initiate the reverse transcription.

(2) A primer 11-B is used to anneal to or hybridize with the cDNA strand, and the primer II-B comprises a consensus sequence B (CB), a unique molecular identifier (UMI) sequence, and a complementary sequence of the 3'-end overhang of the cDNA. Subsequently, the nucleic acid fragment hybridized with or annealed to the primer 11-B can be extended under the presence of a nucleic acid polymerase using the UMI sequence and the consensus sequence B as a template, thereby generating a nucleic acid molecule that carries at its 3' end a complementary sequence of the UMI sequence and a complementary sequence of the consensus sequence B.

Typically, the consensus sequence B is located upstream of the UMI sequence (e.g., located 5' of the UMI sequence), and the sequence complementary to the 3'-end overhang of the cDNA strand is located at the 3' end of the primer II-B.

For example, when the cDNA strand comprises an overhang of three cytosine nucleotides at the 3' end, the primer II-B may comprise GGG at its 3' end. In addition, the nucleotides of the primer 11-B can also be modified (e.g., the primer 11-B can be modified to comprise one or more locked nucleic acids) to enhance the binding affinity for the complementary pairing between the primer II-B and the 3'-end overhang of the cDNA strand.

Without being limited by any theory, various suitable nucleic acid polymerases (e.g., DNA polymerase or reverse transcriptase) can be used to perform the extension reaction, as long as they can use the sequence of the primer II-B or partial sequence thereof as a template to extend the annealed or hybridized nucleic acid fragment (reverse transcription product). In certain exemplary embodiments, the annealed or hybridized nucleic acid fragment (reverse transcription product) can be extended using the same reverse transcriptase used in the aforementioned reverse transcription step.

In some embodiments, this step is performed simultaneously with step (1) (e.g., in the same reaction system).

In certain embodiments, the method optionally further comprises step (3): adding RNase H to digest the RNA strand in the RNA/cDNA hybrid to form a cDNA single strand.

In certain embodiments, the method does not comprise the step (3).

An exemplary structure of the cDNA strand prepared by the above exemplary embodiment comprises: the consensus sequence A, the cDNA sequence, the 3'-end overhang sequence, a complementary sequence of the UMI sequence, and a complementary sequence of the consensus sequence B.

II. An exemplary embodiment of using a complementary sequence of an oligonucleotide probe (also referred as a chip sequence) to label the 3' end of a cDNA strand to form a new nucleic acid molecule comprising the chip sequence information (i.e., a nucleic acid molecule labeled with the chip sequence) comprises the following steps (as shown in Fig. 7):
providing a bridging oligonucleotide pair consisting of a bridging oligonucleotide II-I and a bridging oligonucleotide II-II, wherein the bridging oligonucleotide II-I and the bridging oligonucleotide II- II each independently comprise: a first region (P1) and a second region (P2), the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence B in the cDNA strand obtained in the above step I.

In certain embodiments, the bridging oligonucleotide 11-1 comprises an intermediate nucleotide sequence, such as an intermediate nucleotide sequence of 1nt to 5 nt or 5nt to 10nt, between the first region and the second region, that is, the bridging oligonucleotide 11-1 comprises a third region located between the first region and the second region. In certain preferred embodiments, the first region and the second region in the bridging oligonucleotide II-I are directly adjacent without extra nucleotides between them, that is, the bridging oligonucleotide II-I does not comprise a third region located between the first region and the second region.

In certain embodiments, the bridging oligonucleotide II-II comprises an intermediate nucleotide sequence, such as an intermediate nucleotide sequence of 1 nt to 5 nt or 5 nt to 10 nt, between the first region and the second region, that is, the bridging oligonucleotide II-II comprises a third region located between the first region and the second region. In certain preferred embodiments, the first region and the second region in the bridging oligonucleotide II-II are directly adjacent without extra nucleotides between them, that is, the bridging oligonucleotide II-II does not comprise a third region located between the first region and the second region.

A new nucleic acid molecule comprising the chip sequence information (i.e., a nucleic acid molecule labeled by the chip sequence) can be obtained by: annealing or hybridizing the bridging oligonucleotide II-I and the bridging oligonucleotide II-II with the chip sequence and the cDNA strand obtained in the above step I, and ligating the nucleic acid molecule hybridized with the first region and the nucleic acid molecule hybridized with the second region of the same bridging oligonucleotide 11-1, and/or, ligating the nucleic acid molecule hybridized with the first region and the nucleic acid molecule hybridized with the second region of the same bridging oligonucleotide II-II using a DNA ligase, and performing an extension reaction under the presence of a DNA polymerase. The ligation process and the extension reaction can be performed in any order.

An exemplary structure of the new nucleic acid molecule comprising the chip sequence information formed by the above exemplary embodiment comprises: a nucleic acid strand and/or a complementary nucleic acid strand thereof, wherein the nucleic acid strand comprises from 5' to 3': the consensus sequence A, the cDNA sequence, the 3'-end overhang sequence, a complementary sequence of the UMI sequence, a complementary sequence of the consensus sequence B, a sequence of the bridging oligonucleotide 11-1, a complementary sequence of the tag sequence Y, and a complementary sequence of the consensus sequence X1.

In certain embodiments, the method comprises step (1), step (2)(ii), and step (3). In certain embodiments, in step (2)(ii)(b), the first extension product comprises from 5' to 3': the consensus sequence A, a cDNA sequence that is formed by reverse transcription primed by the primer II-A' and complementary to the RNA, the 3'-end overhang sequence, optionally a complementary sequence of the tag sequence B, and a complementary sequence of the consensus sequence B.

In certain embodiments, in step (2)(ii)(c), the extension primer is the primer II-B' as described above or a primer B", wherein the primer B" is capable of annealing to a complementary sequence or partial sequence thereof of the consensus sequence B, and initiating an extension reaction.

In certain embodiments, in step (2)(ii)(c), the second extension product comprises from 5' to 3': a sequence that is formed by an extension reaction primed by the extension primer and complementary to the cDNA sequence, and a complementary sequence of the consensus sequence A.

### Embodiments comprising step (1), step (2)(ii) and step (3)(i)

In certain embodiments, the method comprises step (1), step (2)(ii) and step (3)(i); wherein the consensus sequence X2 or partial sequence thereof is capable of annealing to a complementary sequence or partial sequence thereof of the consensus sequence A; and the extension product obtained in step (3)(i) is the labeled nucleic acid molecule, which comprises: the first strand comprising the sequence of the first nucleic acid molecule to be labeled, and/or, the second strand comprising the sequence of the oligonucleotide probe.

It is easy to understand that the consensus sequence X2 is capable of annealing in its entire nucleotide sequence to a complementary sequence or a partial segment thereof of the consensus sequence A, and that the consensus sequence X2 is also capable of annealing in its partial segment nucleotide sequence to a complementary sequence or a partial segment thereof of the consensus sequence A.

In certain embodiments, the first strand comprises from 5' to 3': the sequence of the first nucleic acid molecule to be labeled, a complementary sequence of the tag sequence Y, and a complementary sequence of the consensus sequence X1.

In certain embodiments, the second strand comprises from 5' to 3': the consensus sequence X1, the tag sequence Y, the consensus sequence X2, and a cDNA sequence complementary to the sequence of the first nucleic acid molecule to be labeled.

### Embodiments comprising step (1), step (2)(ii) and step (3)(i), for generating the first strand

In certain embodiments, the consensus sequence X2 or partial sequence thereof is capable of annealing to a complementary sequence or partial sequence thereof (e.g., a partial sequence at the 3' end thereof) of the consensus sequence A; the extension product obtained in step (3)(i) is the labeled nucleic acid molecule, which comprises the first strand comprising the sequence of the first nucleic acid molecule to be labeled.

In certain embodiments, in step (3)(i), the oligonucleotide probe is incapable of initiating an extension reaction (e.g., the 3' end of the oligonucleotide probe is blocked).

In certain embodiments, in step (2)(ii)(a), the capture sequence A of the primer II-A' is a random oligonucleotide sequence.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, and a complementary sequence of the consensus sequence A. In certain embodiments, the first strand comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the consensus sequence A, a complementary sequence of the tag sequence Y, and a complementary sequence of the consensus sequence X1.

In some embodiments, in step (3), the first strand derived from each copy of the oligonucleotide probe coupled to the same microdot has a different complementary sequence of the capture sequence A as a UMI.

In certain embodiments, in step (2)(ii)(a), the capture sequence A of the primer II-A' is a poly(T) sequence or a specific sequence targeting a target nucleic acid.

In certain embodiments, the primer II-A' further comprises a tag sequence A, such as a random oligonucleotide sequence.

In certain embodiments, the capture sequence A is located at the 3' end of the primer II-A'.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the tag sequence A, and a complementary sequence of the consensus sequence A. In certain embodiments, the first strand comprises from 5' to 3': the consensus sequence B, optionally the tag sequence B, a complementary sequence of the 3'-end overhang sequence, and a complementary sequence of the cDNA sequence that is formed by reverse transcription primed by the primer II-A' and complementary to the RNA, a complementary sequence of the tag sequence A, a complementary sequence of the consensus sequence A, a complementary sequence of the tag sequence Y, and a complementary sequence of the consensus sequence X1.

In some embodiments, in step (3), the first strand derived from each copy of the oligonucleotide probe coupled to the same microdot has a different complementary sequence of the tag sequence A as a UMI.

### Embodiments comprising step (1), step (2)(ii) and step (3)(i), for generating the second strand

In some embodiments, the consensus sequence X2 or partial sequence thereof (e.g., a partial sequence at the 3' end thereof) is capable of annealing to a complementary sequence or partial sequence thereof of the consensus sequence A; and the extension product obtained in step (3)(i) is the labeled nucleic acid molecule, which comprises the second strand comprising the sequence of the oligonucleotide probe.

In certain embodiments, the second extension product obtained in step (2)(ii) is incapable of initiating an extension reaction (e.g., the 3' end of the second extension product obtained in step (2)(ii) is blocked).

In certain embodiments, in step (2)(ii)(a), the capture sequence A of the primer II-A' is a random oligonucleotide sequence.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, and a complementary sequence of the consensus sequence A. In certain embodiments, the second strand comprises from 5' to 3': the consensus sequence X1, the tag sequence Y, the consensus sequence X2, a cDNA sequence complementary to the sequence of the first nucleic acid molecule to be labeled, the 3'-end overhang sequence, optionally a complementary sequence of the tag sequence B, and a complementary sequence of the consensus sequence B.

In certain embodiments, in step (3), the second strand derived from each copy of the oligonucleotide probe coupled to the same microdot has a different capture sequence A as a UMI.

In certain embodiments, in step (2)(ii)(a), the capture sequence A of the primer II-A' is a poly(T) sequence or a specific sequence targeting a target nucleic acid.

In certain embodiments, the primer II-A' further comprises a tag sequence A, such as a random oligonucleotide sequence.

In certain embodiments, the capture sequence A is located at the 3' end of the primer II-A'.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the tag sequence A, and a complementary sequence of the consensus sequence A. In certain embodiments, the second strand comprises from 5' to 3': the consensus sequence X1, the tag sequence Y, the consensus sequence X2, the tag sequence A, a cDNA sequence complementary to the sequence of the first nucleic acid molecule to be labeled, the 3'-end overhang sequence, optionally a complementary sequence of the tag sequence B, and a complementary sequence of the consensus sequence B.

In certain embodiments, in step (3), the second strand derived from each copy of the oligonucleotide probe coupled to the same microdot has a different tag sequence A as a UMI.

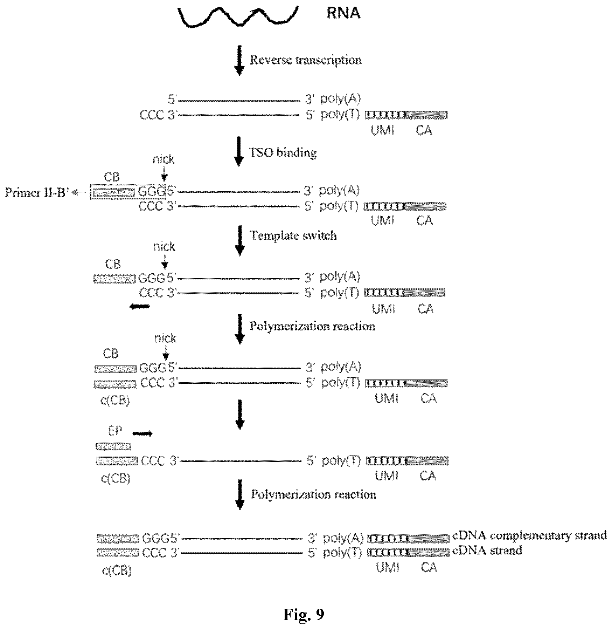

An exemplary embodiment of the present application comprising step (1), step (2)(ii) and step (3)(i) is described in detail as follows:
I. An exemplary embodiment of using an RNA (e.g., mRNA) in a sample as a template to prepare a complementary strand of a cDNA strand comprising close to its 3' end a complementary sequence of UMI comprises the following steps (as shown in Fig. 9):
   (1) A reverse transcriptase (e.g., a reverse transcriptase with terminal deoxynucleotidyl transferase activity) and a primer II-A' are used to reverse-transcribe an RNA molecule (e.g., mRNA molecule) in a permeabilized sample to generate a cDNA, and an overhang (e.g., an overhang comprising three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.

In certain embodiments, the primer II-A' comprises a poly(T) sequence, a UMI sequence, and a consensus sequence A (CA). Typically, the poly(T) sequence is located at the 3' end of the primer II-A' to initiate the reverse transcription, and the consensus sequence A is located upstream of the UMI sequence (e.g., located 5' of the UMI sequence).

In certain embodiments, the primer II-A' comprises a random oligonucleotide sequence and the consensus sequence A, and is capable of being used to capture an RNA without a poly-A tail. Typically, the random oligonucleotide sequence is located at the 3' end of the primer II-A' to initiate the reverse transcription.

(2) A primer 11-B' is used to anneal to or hybridize with the cDNA strand, in which the primer 11-B' comprises a consensus sequence B (CB) and a complementary sequence of the 3'-end overhang of the cDNA. Subsequently, the nucleic acid fragment hybridized with or annealed to the primer 11-B' can be extended under the presence of a nucleic acid polymerase using the consensus sequence B as a template, to add a complementary sequence of the consensus sequence B (c(CB)) to the 3' end of the cDNA strand, thereby generating a nucleic acid molecule that carries at its 3' end a complementary sequence of the consensus sequence B.

Typically, the sequence complementary to the 3'-end overhang of the cDNA strand is located at the 3' end of the primer II-B'.

For example, when the cDNA strand comprises an overhang of three cytosine nucleotides at the 3' end, the primer 11-B' may comprise GGG at its 3' end. In addition, the nucleotides of the primer II-B' can also be modified (e.g., the primer 11-B' can be modified to comprise one or more locked nucleic acids) to enhance the binding affinity for the complementary pairing between the primer II-B' and the 3'-end overhang of the cDNA strand.

Without being limited by any theory, various suitable nucleic acid polymerases (e.g., DNA polymerase or reverse transcriptase) can be used to perform the extension reaction, as long as they can use the sequence of the primer II-B' or partial sequence thereof as a template to extend the annealed or hybridized nucleic acid fragment (reverse transcription product). In certain exemplary embodiments, the annealed or hybridized nucleic acid fragment (reverse transcription product) can be extended using the same reverse transcriptase used in the aforementioned reverse transcription step.

In some embodiments, this step is performed simultaneously with step (1) (e.g., in the same reaction system).

In certain embodiments, the method optionally further comprises step (3): adding RNase H to digest the RNA strand in the RNA/cDNA hybrid to form a cDNA single strand.

In certain embodiments, the method does not comprise the step (3).

(4) An extension primer is used to perform an extension reaction with the cDNA strand obtained in the previous step as a template to obtain an extension product; the extension primer is the primer II-B' as describe above or a primer B", and the primer B" is capable of annealing to the consensus sequence B or partial sequence thereof and initiating the extension reaction.

An exemplary structure of the complementary strand of the cDNA strand prepared by the above exemplary embodiment comprises: the consensus sequence B, a complementary sequence of the 3'-end overhang, a complementary sequence of the cDNA sequence, a complementary sequence of the UMI sequence, and a complementary sequence of the consensus sequence A.

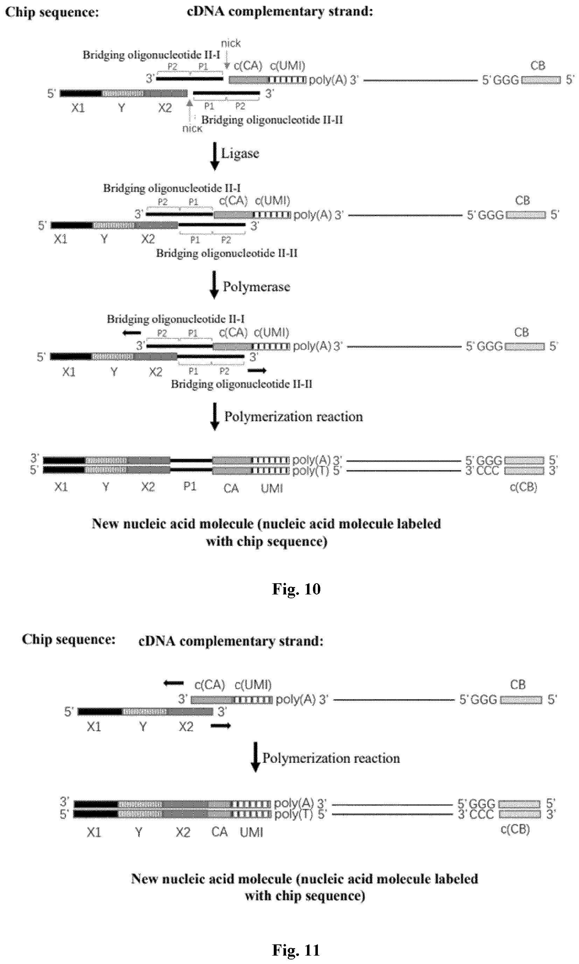

II. An exemplary embodiment of using a complementary sequence of an oligonucleotide probe (also referred as a chip sequence) to label the 3' end of a complementary strand of a cDNA strand to form a new nucleic acid molecule comprising the chip sequence information (i.e., a nucleic acid molecule labeled with the chip sequence) comprises the following steps (as shown in Fig. 11):
in some embodiments, the consensus sequence X2 of the chip sequence or partial sequence thereof is capable of annealing to a complementary sequence or partial sequence thereof of the consensus sequence A of the complementary strand of the cDNA strand obtained in the above step I. Thereby a new nucleic acid molecule comprising the chip sequence information (i.e., a nucleic acid molecule labeled by the chip sequence) can be obtained by annealing or hybridizing the complementary strand of the cDNA strand to the chip sequence, and performing an extension reaction under the presence of a polymerase.

An exemplary structure of the new nucleic acid molecule comprising the chip sequence information formed by the above exemplary embodiment comprises: a nucleic acid strand and/or a complementary nucleic acid strand thereof, wherein the nucleic acid strand comprises from 5' to 3': the consensus sequence B, a complementary sequence of the 3'-end overhang, a complementary sequence of the cDNA sequence, a complementary sequence of the UMI sequence, a complementary sequence of the consensus sequence A, a complementary sequence of the tag sequence Y, and a complementary sequence of the consensus sequence X1.

### Embodiments comprising step (1), step (2)(ii) and step (3)(ii)

In certain embodiments, the method comprises step (1), step (2)(ii) and step (3)(ii); wherein the second region of the bridging oligonucleotide II-II is capable of annealing to a complementary sequence or partial sequence thereof of the consensus sequence A of the second extension product obtained in step (2)(ii); and the reaction product obtained in step (3)(ii) is the labeled nucleic acid molecule, which comprises: the first strand comprising the sequence of the first nucleic acid molecule to be labeled, and/or the second strand comprising the sequence of the oligonucleotide probe.

It is easy to understand that the second region of the bridging oligonucleotide II-II is capable of annealing to a complementary sequence or a partial segment thereof of the consensus sequence A of the second extension product obtained in step (2)(ii).

In certain embodiments, the first strand comprises from 5' to 3': the sequence of the first nucleic acid molecule to be labeled, and optionally a complementary sequence of the third region of the bridging oligonucleotide II-II, the sequence of the bridging oligonucleotide 11-1, a complementary sequence of the tag sequence Y, and a complementary sequence of the consensus sequence X1.

In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, and a cDNA sequence complementary to the sequence of the first nucleic acid molecule to be labeled.

### Embodiments comprising step (1), step (2)(ii) and step (3)(ii), for generating the first strand

In certain embodiments, the second region of the bridging oligonucleotide II-II is capable of annealing to a complementary sequence or a partial sequence at the 3' end thereof of the consensus sequence A of the second extension product obtained in step (2)(ii), and the second region of the bridging oligonucleotide II-I has a free 3' end.

In certain embodiments, the reaction product obtained in step (3)(ii) is the labeled nucleic acid molecule, which comprises the first strand.

In certain embodiments, the second region of the bridging oligonucleotide 11-1 is located at the 3' end of the bridging oligonucleotide 11-1.

In certain embodiments, the first region of the bridging oligonucleotide 11-1 is located at the 5' end of the bridging oligonucleotide 11-1.

In certain embodiments, the bridging oligonucleotide II-I does not comprise the third region, and/or the bridging oligonucleotide II-II does not comprise the third region.

In certain embodiments, the bridging oligonucleotide 11-1 comprises a 5' phosphate at the 5' end.

In certain embodiments, the bridging oligonucleotide 11-1 comprises a free -OH at the 3' end.

In certain embodiments, in step (3)(ii), the bridging oligonucleotide II-II is incapable of initiating an extension reaction (e.g., the 3' end of the bridging oligonucleotide II-II is blocked), and/or, the oligonucleotide probe is incapable of initiating an extension reaction (i.e., the 3' end of the oligonucleotide probe is blocked).

In certain embodiments, in step (2)(ii)(a), the capture sequence A of the primer II-A' is a random oligonucleotide sequence.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, and a complementary sequence of the consensus sequence A. In certain embodiments, the first strand comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the consensus sequence A, optionally a complementary sequence of the third region of the bridging oligonucleotide II-II, the sequence of the bridging oligonucleotide 11-1, a complementary sequence of the tag sequence Y, and a complementary sequence of the consensus sequence X1.

In some embodiments, in step (3), the first strand derived from each copy of the oligonucleotide probe coupled to the same microdot has a different complementary sequence of the capture sequence A as a UMI.

In certain embodiments, in step (2)(ii)(a), the capture sequence A of the primer II-A' is a poly(T) sequence or a specific sequence targeting a target nucleic acid.

In certain embodiments, the primer II-A' further comprises a tag sequence A, such as a random oligonucleotide sequence.

In certain embodiments, the capture sequence A is located at the 3' end of the primer II-A'.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the tag sequence A, and a complementary sequence of the consensus sequence A. In certain embodiments, the first strand comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the tag sequence A, a complementary sequence of the consensus sequence A, optionally a complementary sequence of the third region of the bridging oligonucleotide II-II, the sequence of the bridging oligonucleotide 11-1, a complementary sequence of the tag sequence Y, and a complementary sequence of the consensus sequence X1.

In some embodiments, in step (3), the first strand derived from each copy of the oligonucleotide probe coupled to the same microdot has a different complementary sequence of the tag sequence A as a UMI.

It is easy to understand that in step (3)(ii), after the bridging oligonucleotide II-I and the bridging oligonucleotide II-II are annealed to the oligonucleotide probe and the first nucleic acid molecule to be labeled which is at the corresponding position of the oligonucleotide probe, the ligation reaction for ligating the nucleic acid molecule hybridized with the first region and the nucleic acid molecule hybridized with the second region of the same bridging oligonucleotide II-I, and/or, ligating the nucleic acid molecule hybridized with the first region and the nucleic acid molecule hybridized with the second region of the same bridging oligonucleotide II-II, and the extension reaction in step (3)(ii) can be performed in any order, as long as the second nucleic acid molecule with a positioning tag can be obtained.

For example, when the ligation reaction and the extension reaction are carried out in the same system, the first strand can be obtained by ligating the nucleic acid molecule hybridized with the first region and the nucleic acid molecule hybridized with the second region of the same bridging oligonucleotide II-II, and extending the bridging oligonucleotide II-I by an extension reaction. In this case, the polymerase used in the extension reaction preferably does not have strand displacement activity or 5' to 3' exonucleolytic activity.

For example, when the ligation reaction and the extension reaction are performed in different systems, and the ligation reaction is performed followed by the extension reaction, the first strand can be obtained in the following exemplary manner:
(A) ligating the nucleic acid molecule hybridized with the first region and the nucleic acid molecule hybridized with the second region of the same bridging oligonucleotide II-II, and extending the bridging oligonucleotide II-I by an extension reaction to obtain the first strand; wherein, the polymerase used for the extension reaction preferably has or does not have strand displacement activity or 5' to 3' exonucleolytic activity;
   or,
(B) ligating the nucleic acid molecule hybridized with the first region and the nucleic acid molecule hybridized with the second region of the same bridging oligonucleotide 11-1, and extending the first nucleic acid molecule to be labeled by an extension reaction to obtain the first strand; wherein, the polymerase used for the extension reaction preferably has strand displacement activity or 5' to 3' exonucleolytic activity.

For example, when the ligation reaction and the extension reaction are performed in different systems, and the extension reaction is performed followed by the ligation reaction, the first strand can be obtained by extending the bridging oligonucleotide II-I by an extension reaction, and then ligating the nucleic acid molecule hybridized with the first region and the nucleic acid molecule hybridized with the second region of the same bridging oligonucleotide II-II. In this case, the polymerase used in the extension reaction preferably does not have strand displacement activity or 5' to 3' exonucleolytic activity.

### Embodiments comprising step (1), step (2)(ii) and step (3)(ii), for generating the second strand

In certain embodiments, the second region of the bridging oligonucleotide II-II is capable of annealing to a complementary sequence or partial sequence thereof of the consensus sequence A of the second extension product obtained in step (2)(ii), and the second region of the bridging oligonucleotide II-II has a free 3' end.

In certain embodiments, the reaction product obtained in step (3)(ii) is the labeled nucleic acid molecule, which comprises the second strand.

In certain embodiments, the second region of the bridging oligonucleotide II-II is located at the 3' end of the bridging oligonucleotide II-II.

In certain embodiments, the first region of the bridging oligonucleotide II-II is located at the 5' end of the bridging oligonucleotide II-II.

In certain embodiments, the bridging oligonucleotide II-I does not comprise the third region, and/or the bridging oligonucleotide II-II does not comprise the third region.

In certain embodiments, the bridging oligonucleotide II-II comprises a 5' phosphate at the 5' end.

In certain embodiments, the bridging oligonucleotide II-II comprises a free -OH at the 3' end.

In certain embodiments, in step (3)(ii), the bridging oligonucleotide II-I is incapable of initiating the extension reaction (e.g., the 3' end of the bridging oligonucleotide II-I is blocked), and/or, the second extension product obtained in step (2)(ii) is incapable of initiating the extension reaction (e.g., the 3' end of the second extension product obtained in step (2)(ii) is blocked).

In certain embodiments, in step (2)(ii)(a), the capture sequence A of the primer II-A' is a random oligonucleotide sequence.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, and a complementary sequence of the consensus sequence A. In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, a cDNA sequence complementary to the sequence of the first nucleic acid molecule to be labeled, the 3'-end overhang sequence, optionally a complementary sequence of the tag sequence B, and a complementary sequence of the consensus sequence B.

In certain embodiments, in step (3), the second strand derived from each copy of the oligonucleotide probe coupled to the same microdot has a different capture sequence A as a UMI.

In certain embodiments, in step (2)(ii)(a), the capture sequence A of the primer II-A' is a poly(T) sequence or a specific sequence targeting a target nucleic acid.

In certain embodiments, the primer II-A' further comprises a tag sequence A, such as a random oligonucleotide sequence.

In certain embodiments, the capture sequence A is located at the 3' end of the primer II-A'.

In certain embodiments, in step (2)(ii)(c), the extension primer is the primer II-B'. In certain embodiments, in step (2)(ii)(c), the second extension product comprises from 5' to 3': the consensus sequence B, optionally the tag sequence B, a complementary sequence of the 3'-end overhang sequence, a complementary sequence of the cDNA sequence that is formed by reverse transcription primed by the primer II-A' and complementary to the RNA, a complementary sequence of the tag sequence A, and a complementary sequence of the consensus sequence A. In certain embodiments, the second strand comprises from 5' to 3': the consensus sequence X1, the tag sequence Y, the consensus sequence X2, optionally a complementary sequence of the third region of the bridging oligonucleotide 11-1, the sequence of the bridging oligonucleotide II-II, the tag sequence A, a cDNA sequence complementary to the sequence of the first nucleic acid molecule to be labeled, the 3'-end overhang sequence, optionally a complementary sequence of the tag sequence B, and a complementary sequence of the consensus sequence B.

In certain embodiments, in step (3), the second strand derived from each copy of the oligonucleotide probe coupled to the same microdot has a different tag sequence A as a UMI.

It is easy to understand that in step (3)(ii), after the bridging oligonucleotide II-I and the bridging oligonucleotide II-II are annealed to the oligonucleotide probe and the first nucleic acid molecule to be labeled which is at the corresponding position of the oligonucleotide probe, the ligation reaction for ligating the nucleic acid molecule hybridized with the first region and the nucleic acid molecule hybridized with the second region of the same bridging oligonucleotide II-I, and/or, ligating the nucleic acid molecule hybridized with the first region and the nucleic acid molecule hybridized with the second region of the same bridging oligonucleotide II-II, and the extension reaction in step (3)(ii) can be performed in any order, as long as the second nucleic acid molecule with a positioning tag can be obtained.

For example, when the ligation reaction and the extension reaction are carried out in the same system, the second strand can be obtained by ligating the nucleic acid molecule hybridized with the first region and the nucleic acid molecule hybridized with the second region of the same bridging oligonucleotide 11-1, and extending the bridging oligonucleotide II-II by an extension reaction. In this case, the polymerase used in the extension reaction preferably does not have strand displacement activity or 5' to 3' exonucleolytic activity.

For example, when the ligation reaction and the extension reaction are performed in different systems, and the ligation reaction is performed followed by the extension reaction, the second strand can be obtained in the following exemplary manner:
(A) ligating the nucleic acid molecule hybridized with the first region and the nucleic acid molecule hybridized with the second region of the same bridging oligonucleotide 11-1, and extending the bridging oligonucleotide II-II by an extension reaction to obtain the second strand; wherein, the polymerase used for the extension reaction preferably has or does not have strand displacement activity or 5' to 3' exonucleolytic activity;
   or,
(B) ligating the nucleic acid molecule hybridized with the first region and the nucleic acid molecule hybridized with the second region of the same bridging oligonucleotide II-II, and extending the oligonucleotide probe by an extension reaction to obtain the second strand; wherein, the polymerase used for the extension reaction preferably has strand displacement activity or 5' to 3' exonucleolytic activity.

For example, when the ligation reaction and the extension reaction are performed in different systems, and the extension reaction is performed followed by the ligation reaction, the second strand can be obtained by extending the bridging oligonucleotide II-II by an extension reaction, and then ligating the nucleic acid molecule hybridized with the first region and the nucleic acid molecule hybridized with the second region of the same bridging oligonucleotide 11-1. In this case, the polymerase used in the extension reaction preferably does not have strand displacement activity or 5' to 3' exonucleolytic activity.

An exemplary embodiment of the present application comprising step (1), step (2)(ii) and step (3)(ii) is described in detail as follows:
I. An exemplary embodiment for preparing a complementary strand of a cDNA strand using an RNA (e.g., mRNA) in a sample as a template comprises the following steps (as shown in Fig. 9):
   (1) A reverse transcriptase (e.g., a reverse transcriptase with terminal deoxynucleotidyl transferase activity) and a primer II-A' are used to reverse-transcribe an RNA molecule (e.g., mRNA molecule) in a permeabilized sample to generate a cDNA, and an overhang (e.g., an overhang comprising three cytosine nucleotides) is added to the 3' end of the cDNA. Various reverse transcriptases with terminal deoxynucleotidyl transferase activity can be used to perform the reverse transcription. In certain preferred embodiments, the reverse transcriptase used does not have RNase H activity.

In certain embodiments, the primer II-A' comprises a poly(T) sequence, a UMI sequence, and a consensus sequence A (CA). Typically, the poly(T) sequence is located at the 3' end of the primer II-A' to initiate the reverse transcription, and the consensus sequence A is located upstream of the UMI sequence (e.g., located 5' of the UMI sequence).

In certain embodiments, the primer II-A' comprises a random oligonucleotide sequence and the consensus sequence A, and can be used to capture an RNA without a poly A tail. Typically, the random oligonucleotide sequence is located at the 3' end of the primer II-A' to initiate the reverse transcription.

(2) A primer II-B' is used to anneal to or hybridize with the cDNA strand, in which the primer 11-B' comprises a consensus sequence B (CB), and a complementary sequence of the 3'-end overhang of the cDNA. Subsequently, the nucleic acid fragment hybridized with or annealed to the primer 11-B' can be extended under the presence of a nucleic acid polymerase using the consensus sequence B as a template, to add a complementary sequence of the consensus sequence B (c(CB)) at the 3' end of the cDNA strand, thereby generating a nucleic acid molecule that carries at the 3' end a complementary sequence of the consensus sequence B.

Typically, the sequence complementary to the 3'-end overhang of the cDNA strand is located at the 3' end of the primer II-B'.

For example, when the cDNA strand comprises an overhang of three cytosine nucleotides at the 3' end, the primer 11-B' may comprise GGG at its 3' end. In addition, the nucleotides of the primer II-B' can also be modified (e.g., the primer 11-B' can be modified to comprise one or more locked nucleic acids) to enhance the binding affinity for the complementary pairing between the primer II-B' and the 3'-end overhang of the cDNA strand.

Without being limited by any theory, various suitable nucleic acid polymerases (e.g., DNA polymerase or reverse transcriptase) can be used to perform the extension reaction, as long as they can use the sequence of the primer II-B' or partial sequence thereof as a template to extend the annealed or hybridized nucleic acid fragment (reverse transcription product). In certain exemplary embodiments, the annealed or hybridized nucleic acid fragment (reverse transcription product) can be extended using the same reverse transcriptase used in the aforementioned reverse transcription step.

In some embodiments, this step is performed simultaneously with step (1) (e.g., in the same reaction system).

In certain embodiments, the method optionally further comprises step (3): adding RNase H to digest the RNA strand in the RNA/cDNA hybrid to form a cDNA single strand.

In certain embodiments, the method does not comprise the step (3).

(4) An extension primer is used to perform an extension reaction with the cDNA strand obtained in the previous step as a template to obtain an extension product; in which the extension primer is the primer II-B' as described above or a primer B", and the primer B" is capable of annealing to the consensus sequence B or partial sequence thereof, and initiating the extension reaction.

An exemplary structure of the complementary strand of the cDNA strand prepared by the above exemplary embodiment comprises: the consensus sequence B, a complementary sequence of the 3'-end overhang, a complementary sequence of the cDNA sequence, a complementary sequence of the UMI sequence, and a complementary sequence of the consensus sequence A.

II. An exemplary embodiment of using a complementary sequence of an oligonucleotide probe (also referred as a chip sequence) to label the 3' end of a complementary strand of a cDNA strand so as to form a new nucleic acid molecule comprising the chip sequence information (i.e., a nucleic acid molecule labeled with the chip sequence) comprises the following steps (as shown in Fig. 10):
providing a bridging oligonucleotide pair consisting of a bridging oligonucleotide II-I and a bridging oligonucleotide II-II, wherein the bridging oligonucleotide II-I and the bridging oligonucleotide II-II each independently comprise: a first region (P1) and a second region (P2), and the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence A in the complementary strand of the cDNA strand obtained in the above step I.

In certain embodiments, the bridging oligonucleotide 11-1 comprises an intermediate nucleotide sequence, such as an intermediate nucleotide sequence of 1nt to 5 nt or 5nt to 10 nt, between the first region and the second region, that is, the bridging oligonucleotide 11-1 comprises a third region located between the first region and the second region. In certain preferred embodiments, the first region and the second region in the bridging oligonucleotide II-I are directly adjacent without extra nucleotides between them, that is, the bridging oligonucleotide II-I does not comprise a third region located between the first region and the second region.

In certain embodiments, the bridging oligonucleotide II-II comprises an intermediate nucleotide sequence, such as an intermediate nucleotide sequence of 1nt to 5 nt or 5nt to 10 nt, between the first region and the second region, that is, the bridging oligonucleotide II-II comprises a third region located between the first region and the second region. In certain preferred embodiments, the first region and the second region in the bridging oligonucleotide II-II are directly adjacent without extra nucleotides between them, that is, the bridging oligonucleotide II-II does not comprise a third region located between the first region and the second region.

A new nucleic acid molecule comprising the chip sequence information (i.e., a nucleic acid molecule labeled by the chip sequence) can be obtained by: annealing or hybridizing the bridging oligonucleotide II-I and the bridging oligonucleotide II-II with the chip sequence and the complementary strand of the cDNA strand obtained in above step I, and ligating the nucleic acid molecule hybridized with the first region and the nucleic acid molecule hybridized with the second region of the same bridging oligonucleotide 11-1, and/or ligating the nucleic acid molecule hybridized with the first region and the nucleic acid molecule hybridized with the second region of the same bridging oligonucleotide II-II using a DNA ligase, and performing an extension reaction under the presence of a DNA polymerase. The ligation process and the extension reaction are performed in any order.

An exemplary structure of the new nucleic acid molecule comprising the chip sequence information formed by the above exemplary embodiment comprises: a nucleic acid strand and/or a complementary nucleic acid strand thereof, wherein the nucleic acid strand comprises from 5' to 3': the consensus sequence B, a complementary sequence of the 3'-end overhang, a complementary sequence of the cDNA sequence, a complementary sequence of the UMI sequence, a complementary sequence of the consensus sequence A, the sequence of the bridging oligonucleotide 11-1, a complementary sequence of the tag sequence Y, and a complementary sequence of the consensus sequence X1.

In certain embodiments, in step (2)(i)(b), the cDNA strand is annealed via its 3'-end overhang to the primer II-B, and, under the presence of a nucleic acid polymerase (e.g., a DNA polymerase or reverse transcriptase), the cDNA strand is extended using the primer II-B as a template to generate the first extension product.

In certain embodiments, in step (2)(ii)(b), the cDNA strand is annealed via its 3'-end overhang to the primer II-B', and, under the presence of a nucleic acid polymerase (e.g., a DNA polymerase or reverse transcriptase), the cDNA strand is extended using the primer 11-B' as a template to generate the first extension product.

In certain embodiments, the 3'-end overhang has a length of at least 1, at least 2, at least 3, at least 4, at least 5, at least 6, at least 7, at least 8, at least 9, at least 10 or more nucleotides. In certain embodiments, the 3'-end overhang is a 3'-end overhang of 2-5 cytosine nucleotides (e.g., a CCC overhang).

In certain embodiments of Scheme I or Scheme II, in step (2), the pretreatment is performed intracellularly.

In certain embodiments of Scheme I or Scheme II, before or after contacting the one or more cells with the solid support of the nucleic acid array, the RNA (e.g., mRNA) of the one or more cells is subjected to the pretreatment to generate the first nucleic acid molecule population.

In certain embodiments of Scheme I or Scheme II, before the pretreatment, the cells are permeabilized.

In certain embodiments of Scheme I or Scheme II, in step (2), the pretreatment is performed extracellularly.

In certain embodiments of Scheme I or Scheme II, after contacting the one or more cells with the solid support of the nucleic acid array, the RNA (e.g., mRNA) of the one or more cells is subjected to the pretreatment to generate the first nucleic acid molecule population.

In certain embodiments of Scheme I or Scheme II, before performing the pretreatment, the method further comprises releasing intracellular RNA (e.g., mRNA); preferably, the intracellular RNA (e.g., mRNA) is released by cell permeabilization or cell lysis treatment.

In certain embodiments of Scheme I or Scheme II, the reverse transcription in step (2) is performed by using a reverse transcriptase.

In certain embodiments of Scheme I or Scheme II, the reverse transcriptase has terminal deoxynucleotidyl transferase activity.

In certain embodiments of Scheme I or Scheme II, the reverse transcriptase is capable of synthesizing a cDNA strand using the RNA (e.g., mRNA) as a template, and adding an overhang at the 3' end of the cDNA strand.

In certain embodiments of Scheme I or Scheme II, the reverse transcriptase is capable of adding to the 3' end of the cDNA strand an overhang having a length of at least 1, at least 2, at least 3, at least 4, at least 5, at least 6, at least 7, at least 8, at least 9, at least 10 or more nucleotides.

In certain embodiments of Scheme I or Scheme II, the reverse transcriptase is capable of adding to the 3' end of the cDNA strand an overhang of 2-5 cytosine nucleotides (e.g., CCC overhang).

In certain embodiments of Scheme I or Scheme II, the reverse transcriptase is selected from the group consisting of M-MLV reverse transcriptase, HIV-1 reverse transcriptase, AMV reverse transcriptase, telomerase reverse transcriptase, and variants, modified products and derivatives thereof with reverse transcription activity of the above reverse transcriptases.

In certain embodiments of Scheme I or Scheme II, step (2) and step (3) have one or more characteristics selected from the following:
(1) the primer I-A, primer 11-A, primer I-A', primer II-A', primer I-B, primer 11-B, primer II-B', bridging oligonucleotide I, bridging oligonucleotide 11-1, and bridging oligonucleotide II-II each independently comprise or consist of natural nucleotides (e.g., deoxyribonucleotides or ribonucleotides), modified nucleotides, non-natural nucleotides, or any combination thereof; in certain embodiments, the primer I-A, primer 11-A, primer I-A', and primer II-A' are capable of initiating an extension reaction;
(2) the primer I-B, primer 11-B, and primer 11-B' each independently comprise a modified nucleotide (e.g., a locked nucleic acid); in certain embodiments, the primer I-B, primer 11-B, and primer 11-B' each independently comprise at its 3' end one or more modified nucleotides (e.g., one or more locked nucleic acids);
(3) the tag sequence A, and tag sequence B each independently have a length of 5 to 200 nt (e.g., 5 to 30 nt, 6 to 15 nt);
(4) the consensus sequence A, and consensus sequence B each independently have a length of 10 to 200 nt (e.g., 10 to 100 nt, 20 to 100 nt, 25 to 100 nt, 5 to 10 nt, 10 to 15 nt, 15 to 20 nt, 20 to 50 nt, 20 to 30 nt, 30 to 40 nt, 40 to 50 nt, 50 to 100 nt);
(5) the primer 1-A, primer II-A, primer I-A', primer II-A', primer I-B, primer II-B, and primer 11-B' each independently have a length of 4 to 200 nt (e.g., 5 to 200 nt, 15 to 230 nt, 26 to 115 nt, 10 to 130 nt, 10 to 20 nt, 20 to 50 nt, 20 to 30 nt, 30 to 40 nt, 40 to 50 nt, 50 to 100 nt, 100 to 150 nt, 150 to 200 nt);
(6) the first region and second region of the bridging oligonucleotide I, bridging oligonucleotide 11-1, and bridging oligonucleotide II-II each independently have a length of 3 to 100 nt (e.g., 20 to 100 nt, 3 to 10 nt, 10 to 15 nt, 15 to 20 nt, 20 to 70 nt, 20 to 30 nt, 30 to 40 nt, 40 to 50 nt, 50 to 100 nt);
(7) the third region of the bridging oligonucleotide I, bridging oligonucleotide 11-1, and bridging oligonucleotide II-II each independently have a length of 0 to 50 nt (e.g., 0 nt, 0 to 10 nt, 10 to 15 nt, 15 to 20 nt, 20 to 30 nt, 30 to 40 nt, 40 to 50 nt);
(8) the bridging oligonucleotide I, bridging oligonucleotide 11-1, and bridging oligonucleotide II-II each independently have a length of 6 to 200 nt (e.g., 20 to 100 nt, 20 to 70 nt, 6 to 15 nt, 15 to 20 nt, 20 to 30 nt, 30 to 40 nt, 40 to 50 nt, 50 to 100 nt, 100 to 150 nt, 150 to 200 nt);
(9) the poly(T) sequence comprises at least 5, or at least 20 (e.g., 6 to 100, 10 to 50) deoxythymidine residues;
(10) the random oligonucleotide sequence has a length of 5 to 200 (e.g., 5 nt, 5 to 30 nt, 6 to 15 nt).

In certain embodiments of Scheme I or Scheme II, the method further comprises: (4) recovering and purifying the second nucleic acid molecule population.

In certain embodiments of Scheme I or Scheme II, the obtained second nucleic acid molecule population and/or a complement thereof is used for constructing a transcriptome library or for transcriptome sequencing.

In certain embodiments of Scheme I or Scheme II, the oligonucleotide probe in step (1) has one or more characteristics selected from the following:
(1) the consensus sequence X1, the tag sequence Y, and the consensus sequence X2 each independently comprise or consist of natural nucleotides (e.g., deoxyribonucleotides or ribonucleotides), modified nucleotides, non-natural nucleotides (e.g., peptide nucleic acids (PNA) or locked nucleic acids), or any combination thereof;
(2) the consensus sequence X1, the tag sequence Y, and the consensus sequence X2 each independently have a length of 2 to 200 nt (e.g., 10 to 200 nt, 25 to 100 nt, 10 to 30 nt, 10 to 100 nt, 5 to 10 nt, 10 to 15 nt, 15 to 20 nt, 20 to 30 nt, 30 to 40 nt, 40 to 50 nt, 50 to 100nt).

In certain embodiments of Scheme I or Scheme II, the nucleic acid array of step (1) is provided by the steps comprising:
(1) providing multiple kinds of carrier sequences, in which each kind of carrier sequence comprises at least one copy (e.g., multiple copies) of the carrier sequence, and the carrier sequence comprises in the direction from 5' to 3': a complementary sequence of the consensus sequence X2, a complementary sequence of the tag sequence Y, and an immobilization sequence; wherein, each kind of carrier sequence has a different complementary sequence of the tag sequence Y;
(2) attaching the multiple kinds of carrier sequences to the surface of a solid support (e.g., a chip);
(3) providing an immobilization primer, and using the carrier sequence as a template to perform a primer extension reaction to generate an extension product, so as to obtain the oligonucleotide probe; wherein the immobilization primer comprises the sequence of the consensus sequence X1, and is capable of annealing to the immobilization sequence of the carrier sequence and initiating the extension reaction; in some embodiments, the extension product comprises or consists of in the direction from 5' to 3': the consensus sequence X1, the tag sequence Y and the consensus sequence X2;
(4) linking the immobilization primer to the surface of the solid support; wherein step (3) and step (4) are performed in any order;
(5) optionally, the immobilization sequence of the carrier sequence further comprises a cleavage site, and the cleavage can be selected from nicking enzyme digestion, USER enzyme digestion, light-responsive excision, chemical excision, or CRISPR-mediated excision; performing cleavage at the cleavage site contained in the immobilization sequence of the carrier sequence to digest the carrier sequence, so as to separate the extension product in step (3) from the template (i.e., the carrier sequence) from which the extension product is generated, thereby linking the oligonucleotide probe to the surface of the solid support (e.g., chip). In certain embodiments, the method further comprises separating the extension product in step (3) from the template (i.e., the carrier sequence) from which the extension product is generated through high-temperature denaturation.

In certain embodiments of Scheme I or Scheme II, each kind of carrier sequence is a DNB formed from a concatemer of multiple copies of the carrier sequence.

In certain embodiments of Scheme I or Scheme II, the multiple kinds of carrier sequences are provided in step (1) by the following steps:
(i) providing multiple kinds of carrier-template sequences, each carrier-template sequence comprises a complementary sequence of a carrier sequence;
(ii) using each kind of carrier-template sequence as a template to perform a nucleic acid amplification reaction to obtain an amplification product of each kind of carrier-template sequence, in which the amplification product comprises at least one copy of the carrier sequence; in certain embodiments, rolling circle replication is performed to obtain a DNB formed from a concatemer of the carrier sequence.

### Scheme III

In certain embodiments, in step (1) of the method, the consensus sequence X2 comprises a capture sequence, the capture sequence is capable of hybridizing to the whole or a part of the nucleic acid to be captured, and the capture sequence comprises a poly(T) sequence, or a specific sequence targeting a target nucleic acid, or a random oligonucleotide sequence; and, the capture sequence has a free 3' end so that the consensus sequence X2 can serve as an extension primer.

In such embodiments, the step (2) comprises: contacting the one or more cells with the solid support of the nucleic acid array, whereby each cell individually occupies at least one microdot in the nucleic acid array (i.e., each cell is contacted with at least one microdot in the nucleic acid array), and allowing the first binding molecule of the cell to interact with the first label molecule of the solid support; and annealing a nucleic acid of the one or more cells to the capture sequence, by applying an annealing condition, so that the position of the nucleic acid is mapped to the position of the oligonucleotide probe on the nucleic acid array;
furthermore, the step (3) comprises: using the oligonucleotide probe as a primer and the captured nucleic acid molecule as a template to perform a primer extension reaction under a condition that allows primer extension to produce a labeled nucleic acid molecule (e.g., a nucleic acid molecule labeled by the tag sequence Y); and/or, using the captured nucleic acid molecule as a primer and the oligonucleotide probe as a template to perform a primer extension reaction to generate an extended captured nucleic acid molecule, thereby forming a labeled nucleic acid molecule (e.g., a nucleic acid molecule labeled by the complementary sequence of the tag sequence Y).

In certain embodiments, the oligonucleotide probe in step (1) further comprises a unique molecular identifier (UMI) sequence. Preferably, the UMI sequence is located upstream of the capture sequence. Preferably, the oligonucleotide probes coupled to the same microdot comprise UMI sequences that are different from each other.

In some embodiments, the nucleic acid array of step (1) is provided by the following steps:
(1) providing multiple kinds of carrier sequences, in which each kind of carrier sequence comprises multiple copies of the carrier sequence, and the carrier sequence comprises in the direction from 5' to 3': a positioning sequence and a first immobilization sequence,
   the positioning sequence is a complementary sequence of the tag sequence Y;
   the first immobilization sequence is capable of annealing to its complementary nucleotide sequence and initiating an extension reaction;
(2) attaching the multiple kinds of carrier sequences to the surface of a solid support (e.g., a chip);
(3) providing a first primer, and using the carrier sequence as a template to perform a primer extension reaction to obtain a first nucleic acid molecule hybridized with the first immobilization sequence and the positioning sequence of the carrier sequence, thereby forming a double strand region at the first immobilization sequence and the positioning sequence of the carrier sequence, and the first nucleic acid molecule comprises in the direction from 5' to 3' a complementary sequence of the first immobilization sequence and a complementary sequence of the positioning sequence; wherein the first primer comprises at its 3' end a region complementary to the first immobilization sequence, and the region of the first primer comprises a complementary sequence or fragment thereof of the first immobilization sequence and has a free 3' end;
(4) providing a second nucleic acid molecule, in which the second nucleic acid molecule comprises a consensus sequence X2 (i.e., the capture sequence), which has a free 3' end so that the second nucleic acid molecule can be used as an extension primer,
(5) ligating the second nucleic acid molecule to the first nucleic acid molecule (e.g., using a ligase to ligate the second nucleic acid molecule to the first nucleic acid molecule), and the ligation product is the oligonucleotide probe that comprises in the direction from 5' to 3' the consensus sequence X1, the tag sequence Y, and the consensus sequence X2.

In certain embodiments, the carrier sequence is optionally digested so that the ligation product is separated from the carrier sequence in step (5), thereby linking the oligonucleotide probe to the surface of the solid support.

In certain embodiments, the first nucleic acid molecule or the second nucleic acid molecule further comprises a UMI sequence. In certain embodiments, the second nucleic acid molecule comprises a UMI sequence located 5' of the capture sequence.

In certain embodiments, the multiple kinds of carrier sequences are provided by the following steps:
(i) providing multiple kinds of carrier-template sequences, the carrier-template sequence comprises a complementary sequence of the carrier sequence;
(ii) using each kind of carrier-template sequence as a template to perform a nucleic acid amplification reaction to obtain an amplification product of each kind of carrier-template sequence, the amplification product comprises multiple copies of the carrier sequence;
preferably, the amplification is selected from the group consisting of rolling circle replication (RCA), bridge PCR amplification, multiple displacement amplification (MDA) or emulsion PCR amplification; preferably, rolling circle replication is performed to obtain a DNB formed by a concatemer of the carrier sequence, or bridge PCR amplification, emulsion PCR amplification or multiple displacement amplification is performed to obtain a DNA cluster formed by the clonal populations of the carrier sequence.

In certain embodiments of Scheme I, Scheme II or Scheme III, the oligonucleotide probe is coupled to the solid support via a linker.

In certain embodiments of Scheme I, Scheme II or Scheme III, the linker is a linking group capable of coupling with an activating group, and the surface of the solid support is modified with the activating group.

In certain embodiments of Scheme I, Scheme II or Scheme III, the linker comprises -SH, - DBCO or -NHS.

In certain embodiments of Scheme I, Scheme II or Scheme III, the linker is -DBCO, and the surface of the solid support is modified with 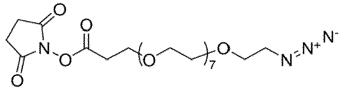 (Azido-dPEG^{®} 8-NHS ester).

In some embodiments of Scheme I, Scheme II or Scheme III, the nucleic acid array of step (1) has one or more characteristics selected from the following:
(1) the oligonucleotide probes coupled to the same solid support have the same consensus sequence X1 and/or the same consensus sequence X2;
(2) the consensus sequence X1 of the oligonucleotide probe comprises a cleavage site; in some embodiments, the cleavage site can be cleaved or broken by nicking enzyme digestion, USER enzyme digestion, light-responsive excision, chemical excision, or CRISPR-mediated excision.

In certain embodiments of Scheme I, Scheme II or Scheme III, the solid support in step (1) has one or more characteristics selected from the following:
(1) the solid support is selected from the group consisting of latex beads, dextran beads, polystyrene surfaces, polypropylene surfaces, polyacrylamide gels, gold surfaces, glass surfaces, chips, sensors, electrodes and silicon wafers; in some embodiments, the solid support is a chip;
(2) the solid support is planar, spherical or porous;
(3) the solid support can be used as a sequencing platform, such as a sequencing chip; in some embodiments, the solid support is a sequencing chip for Illumina, MGI or Thermo Fisher sequencing platform; and
(4) the solid support is capable of releasing the oligonucleotide probe spontaneously or upon exposure to one or more stimuli (e.g., temperature change, pH change, exposure to specific chemicals or phases, exposure to light, exposure to reducing agents, etc.).

### Method for Constructing a Library of Nucleic Acid Molecules

In another aspect, the present application also provides a method for constructing a library of nucleic acid molecules, which comprises,
(a) generating a population of labeled nucleic acid molecules according to the method as described above;
(b) randomly fragmenting the nucleic acid molecules in the population of labeled nucleic acid molecules and linking an adapter thereto; and
(c) optionally, amplifying and/or enriching the product of step (b);
thereby obtaining a library of nucleic acid molecules.

In certain embodiments, the library of nucleic acid molecules comprises nucleic acid molecules from multiple single cells, and the nucleic acid molecules of different single cells have different tag sequences Y.

In certain embodiments, the library of nucleic acid molecules is used for sequencing, such as transcriptome sequencing, such as single-cell transcriptome sequencing (e.g., 5' or 3' transcriptome sequencing).

In certain embodiments, before performing step (b), the method further comprises step (pre-b): amplifying and/or enriching the population of labeled nucleic acid molecules.

In certain embodiments, in step (pre-b), the population of labeled nucleic acid molecules is subjected to a nucleic acid amplification reaction to generate an amplification product.

In certain embodiments, the amplification reaction is performed using at least a primer C and/or a primer D, wherein the primer C is capable of hybridizing with or annealing to a complementary sequence or partial sequence thereof of the consensus sequence X1, and initiating an extension reaction; the primer D is capable of hybridizing with or annealing to the nucleic acid molecule strand comprising the tag sequence Y in the population of labeled nucleic acid molecules, and initiating an extension reaction.

In certain embodiments, the nucleic acid amplification reaction in step (pre-b) is performed by using a nucleic acid polymerase (e.g., a DNA polymerase, for example, a DNA polymerase with strand displacement activity and/or high fidelity).

In certain embodiments, in step (b) of the method, the nucleic acid molecule is randomly fragmented and the resulting fragments are linked with an adapter by using a transposase.

In some embodiments, in step (b) of the method, the nucleic acid molecules obtained in the previous step are randomly fragmented and the resulting fragments are linked with a first adapter and a second adapter at both ends respectively, by using a transposase.

In certain embodiments, the transposase is selected from the group consisting of Tn5 transposase, MuA transposase, Sleeping Beauty transposase, Mariner transposase, Tn7 transposase, Tn10 transposase, Ty1 transposase, Tn552 transposase, as well as variants, modified products and derivatives thereof having the transposase activity of the above-mentioned transposases.

In certain embodiments, the transposase is a Tn5 transposase.

In some embodiments, in step (c), the product of step (b) is amplified using at least a primer C' and/or a primer D', wherein the primer C' is capable of hybridizing with or annealing to the first adapter, and initiating an extension reaction, and the primer D' is capable of hybridizing with or annealing to the second adapter, and initiating an extension reaction.

In some embodiments, in step (c), at least the primer C as described above and/or primer D' is used to amplify the product of step (b); wherein the primer D' is capable of hybridizing with or annealing to the first adapter or the second adapter, and initiating an extension reaction.

### Method for Performing Transcriptome Sequencing

In another aspect, the present application also provides a method for transcriptome sequencing of cells in a sample, which comprises:
(1) constructing a library of nucleic acid molecules according to the method as described above; and
(2) sequencing the library of nucleic acid molecules.

### Method for Performing Single-cell Transcriptome Analysis

In another aspect, the present application also provides a method for performing single-cell transcriptome analysis, which comprises:
(1) performing transcriptome sequencing of single cells in a sample according to the method described above; and
(2) analyzing the sequencing data, which comprises performing match of the sequencing results of the sequencing library with the tag sequence Y of the oligonucleotide probe coupled to each microdot on the nucleic acid array or complementary sequence thereof, wherein the microdot is identified as a positive microdot if the match is successful, and, the sequencing data derived from the positive microdots with regional continuity in the nucleic acid array is identified as the transcription data of the same cell, thereby performing single-cell transcriptome analysis.

### Kit

In another aspect, the application also provides a kit, which comprises:
a nucleic acid array for labeling nucleic acids and optionally a first binding molecule, in which the nucleic acid array comprises a solid support, the solid support comprises (e.g., on its surface) a first label molecule, and the first binding molecule is capable of forming an interaction pair with the first label molecule;
the solid support further comprises a plurality of microdots, the size (e.g., equivalent diameter) of the microdots is less than 5 µm, and the center-to-center distance between adjacent microdots is less than 10 µm; each microdot is coupled with one kind of oligonucleotide probe; each kind of oligonucleotide probe comprises at least one copy; and, the oligonucleotide probe comprises or consists of in the direction from 5' to 3': a consensus sequence X1, a tag sequence Y, and a consensus sequence X2, wherein,
oligonucleotide probes coupled to different microdots have different tag sequences Y.

In certain embodiments, the center-to-center distance between adjacent microdots is less than 10 µm, less than 5 µm, less than 1 µm, less than 0.5 µm, less than 0.1 µm, less than 0.05 µm, or less than 0.01 µm; and, the size (e.g., equivalent diameter) of the microdots is less than 5 µm, less than 1 µm, less than 0.3 µm, less than 0.5 µm, less than 0.1 µm, less than 0.05 µm, less than 0.01 µm, or less than 0.001 µm.

Preferably, the center-to-center distance between adjacent microdots is 0.5 µm to 1 µm, such as 0.5 µm to 0.9 µm, 0.5 µm to 0.8 µm.

Preferably, the size (e.g., equivalent diameter) of the microdots is 0.001 µm to 0.5 µm (e.g., 0.01 µm to 0.1 µm, 0.01 µm to 0.2 µm, 0.2 µm to 0.5 µm, 0.2 µm to 0.4 µm, 0.2 µm to 0.3 µm).

In certain embodiments, the solid support comprises a plurality of (e.g., at least 10, at least 10², at least 10³, at least 10⁴, at least 10⁵, at least 10⁶, at least 10⁷, at least 10⁸, or more) microdots. In certain embodiments, the solid support comprises at least 10⁴ (e.g., at least 10⁴, at least 10⁵, at least 10⁶, at least 10⁷, at least 10⁸, at least 10⁹, at least 10¹⁰, at least 10¹¹, or at least 10¹²) microdots/mm².

In certain embodiments, the first binding molecule is capable of forming a specific interaction pair or a non-specific interaction pair with the first label molecule.

In certain embodiments, the interaction pair is selected from the group consisting of an interaction pair of positive charge and negative charge, affinity interaction pair (e.g., biotin/avidin, biotin/streptavidin, antigen/antibody, receptor/ligand, enzyme/cofactor), a pair of molecules capable of undergoing click chemical reaction (e.g., alkynyl-containing compound/azide compound), N-hydroxysulfosuccinate (NHS) ester/amino-containing compound, and any combination thereof.

For example, the first label molecule is polylysine, and the first binding molecule is a protein capable of binding to polylysine; the first label molecule is an antibody, and the first binding molecule is an antigen capable of binding to the antibody; the first label molecule is an amino-comprising compound, and the first binding molecule is N-hydroxysulfosuccinate (NHS) ester; or the first label molecule is biotin, and the first binding molecule is streptavidin.

In certain embodiments, the kit further comprises:
(i) a primer I-A, or a primer set comprising a primer I-A' and a primer I-B, or a primer set comprising the primer I-A and the primer I-B, wherein:
   the primer I-A comprises a consensus sequence A and a capture sequence A, and the capture sequence A is capable of annealing to an RNA (e.g., mRNA) to be captured and initiating an extension reaction; preferably, the consensus sequence A is located upstream of the capture sequence A (e.g., located at the 5' end of the primer I-A);
   the primer I-A' comprises a capture sequence A, which is capable of annealing to an RNA (e.g., mRNA) to be captured and initiating an extension reaction;
   the primer I-B comprises a consensus sequence B, a complementary sequence of a 3'-end overhang, and optionally a tag sequence B; wherein the complementary sequence of a 3'-end overhang is located at the 3' end of the primer I-B, and the consensus sequence B is located upstream of the complementary sequence of a 3'-end overhang (e.g., located at the 5' end of the primer I-B); wherein the 3'-end overhang refers to one or more non-templated nucleotides contained in the 3' end of the cDNA strand generated by reverse transcription with the RNA captured by the capture sequence A of the primer I-A' as a template;
      and,
(ii) a bridging oligonucleotide I, which comprises: a first region and a second region, and optionally a third region located between the first region and the second region, and the first region is located upstream of the second region (e.g., located 5' of the second region); wherein,
   the first region is capable of (a) annealing to the whole or a part of the consensus sequence A of the primer I-A or (b) annealing to the whole or a part of the consensus sequence B of the primer I-B;
   the second region is capable of annealing to the whole or a part of the consensus sequence X2.

In certain embodiments, the kit comprises: the primer I-A as described in (i), and the bridging oligonucleotide I as described in (ii); wherein, the first region of the bridging oligonucleotide I is capable of annealing to the whole or a part of the consensus sequence A of the primer I-A, and the second region of the bridging oligonucleotide I is capable of annealing to the whole or a part of the consensus sequence X2;
wherein, the capture sequence A of the primer I-A is a random oligonucleotide sequence; or, the capture sequence A of the primer I-A is a poly(T) sequence or a specific sequence targeting a target nucleic acid, and the primer I-A further comprises a tag sequence A, for example, a random oligonucleotide sequence.

In certain embodiments, the primer I-A comprises a 5' phosphate at the 5' end.

In certain embodiments, the kit comprises: the primer set comprising the primer I-A' and the primer I-B as described in (i), and the bridging oligonucleotide I as described in (ii); wherein, the first region of the bridging oligonucleotide I is capable of annealing to the whole or a part of the consensus sequence B of the primer I-B, and the second region of the bridging oligonucleotide I is capable of annealing to the whole or a part of the consensus sequence X2;
wherein, the capture sequence A of the primer I-A' is a random oligonucleotide sequence; or, the capture sequence A of the primer I-A' is a poly(T) sequence or a specific sequence targeting a target nucleic acid, and the primer I-A' further comprises a tag sequence A, and a consensus sequence A;
wherein, the primer I-B comprises the consensus sequence B, a complementary sequence of the 3'-end overhang, and the tag sequence B.

In certain embodiments, the kit further comprises a primer B", and the primer B" is capable of annealing to a complementary sequence or partial sequence thereof of the consensus sequence B and initiating an extension reaction.

In certain embodiments, the primer I-B or primer B" comprises a 5' phosphate at the 5' end.

In certain embodiments, the primer I-B comprises a modified nucleotide (e.g., a locked nucleic acid); preferably, the primer I-B comprises one or more modified nucleotides (e.g., one or more locked nucleic acids) at the 3' end.

In certain embodiments, the kit comprises: the primer set comprising the primer I-A and the primer I-B as described in (i), and the bridging oligonucleotide I as described in (ii); wherein, the first region of the bridging oligonucleotide I is capable of annealing to the whole or a part of the consensus sequence A of the primer I-A, and the second region of the bridging oligonucleotide I is capable of annealing to the whole or a part of the consensus sequence X2;
wherein, the capture sequence A of the primer I-A is a random oligonucleotide sequence; or, the capture sequence A of the primer I-A is a poly(T) sequence or a specific sequence targeting a target nucleic acid, and the primer I-A further comprises a tag sequence A, for example, a random oligonucleotide sequence.

In certain embodiments, the primer I-A comprises a 5' phosphate at the 5' end.

In certain embodiments, the primer I-B comprises a modified nucleotide (e.g., a locked nucleic acid); preferably, the primer I-B comprises one or more modified nucleotides (e.g., one or more locked nucleic acids) at the 3' end.

In certain embodiments, the kit further comprises:
(i) a primer set comprising a primer II-A and a primer II-B, or a primer set comprising a primer II-A' and a primer II-B', wherein:
   the primer II-A comprises a capture sequence A, and the capture sequence A is capable of annealing to an RNA (e.g., mRNA) to be captured and initiating an extension reaction;
   the primer II-B comprises a consensus sequence B, a complementary sequence of a 3'-end overhang, and optionally a tag sequence B; wherein the complementary sequence of a 3'-end overhang is located at the 3' end of the primer II-B, the consensus sequence B is located upstream of the complementary sequence of a 3'-end overhang (e.g., located at the 5' end of the primer II-B); wherein the 3'-end overhang refers to one or more non-templated nucleotides contained in the 3' end of a cDNA strand generated by reverse transcription with the RNA captured by the capture sequence A of the primer II-A as a template;
   the primer II-A' comprises a consensus sequence A and a capture sequence A; wherein the capture sequence A is located at the 3' end of the primer II-A', and the consensus sequence A is located upstream of the capture sequence A (e.g., located at the 5' end of the primer II-A');
   the primer II-B' comprises a consensus sequence B, a complementary sequence of a 3'-end overhang, and optionally a tag sequence B; wherein the complementary sequence of a 3'-end overhang is located at the 3' end of the primer II-B', the consensus sequence B is located upstream of the complementary sequence of a 3'-end overhang (e.g., located at the 5' end of the primer II-B'); wherein the 3'-end overhang refers to one or more non-templated nucleotides contained in the 3' end of a cDNA strand generated by reverse transcription with the RNA captured by the capture sequence A of the primer II-A' as a template.

In certain embodiments, the kit comprises: the primer set comprising the primer II-A and the primer II-B as described in (i), and, (ii) a bridging oligonucleotide II-I and a bridging oligonucleotide II-II; wherein, the bridging oligonucleotide II-I and the bridging oligonucleotide II-II each independently comprise: a first region and a second region, and optionally a third region located between the first region area and the second region, the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence B of the primer II-B;
wherein, the capture sequence A of the primer II-A is a random oligonucleotide sequence; or, the capture sequence A of the primer II-A is a poly(T) sequence or a specific sequence targeting a target nucleic acid, and the primer II-A preferably further comprises a consensus sequence A and optionally a tag sequence A, such as a random oligonucleotide sequence;
wherein, the primer II-B comprises the consensus sequence B, a complementary sequence of the 3'-end overhang, and the tag sequence B.

In certain embodiments, the primer II-B comprises a modified nucleotide (e.g., a locked nucleic acid); preferably, the primer II-B comprises one or more modified nucleotides (e.g., one or more locked nucleic acids) at the 3' end.

In certain embodiments, the kit comprises: the primer set comprising the primer II-A and primer II-B as described in (i);
wherein, the capture sequence A of the primer II-A is a random oligonucleotide sequence; or, the capture sequence A of the primer II-A is a poly(T) sequence or a specific sequence targeting a target nucleic acid, and the primer II-A preferably further comprises a consensus sequence A and optionally a tag sequence A, such as a random oligonucleotide sequence;
wherein, the primer II-B comprises the consensus sequence B, a complementary sequence of the 3'-end overhang, and the tag sequence B.

In certain embodiments, the primer II-B comprises a modified nucleotide (e.g., a locked nucleic acid); preferably, the primer II-B comprises one or more modified nucleotides (e.g., one or more locked nucleic acids) at the 3' end.

In certain embodiments, the kit comprises: the primer set comprising the primer II-A' and primer II-B' as described in (i), and, (ii) a bridging oligonucleotide II-I and a bridging oligonucleotide II-II; wherein, the bridging oligonucleotide II-I and the bridging oligonucleotide II-II each independently comprise: a first region and a second region, and optionally a third region located between the first region and the second region, the first region is located upstream of the second region (e.g., located 5' of the second region); wherein,
the first region of the bridging oligonucleotide II-I is capable of annealing to the first region of the bridging oligonucleotide II-II; the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence A of the primer II-A';
wherein, the capture sequence A of the primer II-A' is a random oligonucleotide sequence; or, the capture sequence A of the primer II-A' is a poly(T) sequence or a specific sequence targeting a target nucleic acid, and the primer II-A' further comprises a tag sequence A, such as a random oligonucleotide sequence.

In certain embodiments, the primer II-B' comprises a modified nucleotide (e.g., a locked nucleic acid); preferably, the primer II-B' comprises one or more modified nucleotides (e.g., one or more locked nucleic acids) at the 3' end.

In certain embodiments, the kit further comprises a primer B", and the primer B" is capable of annealing to a complementary sequence or partial sequence thereof of the consensus sequence B, and initiating an extension reaction.

In certain embodiments, the kit comprises the primer set comprising the primer II-A' and primer II-B' as described in (i);
wherein, the capture sequence A of the primer II-A' is a random oligonucleotide sequence; or, the capture sequence A of the primer II-A' is a poly(T) sequence or a specific sequence targeting a target nucleic acid, and the primer II-A' further comprises a tag sequence A, such as a random oligonucleotide sequence;
wherein, the primer II-B' comprises the consensus sequence B, a complementary sequence of the 3'-end overhang, and the tag sequence B.

In certain embodiments, the primer II-B' comprises a modified nucleotide (e.g., a locked nucleic acid); preferably, the primer II-B' comprises one or more modified nucleotides (e.g., one or more locked nucleic acids) at the 3' end.

In certain embodiments, the kit further comprises a primer B", and the primer B" is capable of annealing to a complementary sequence or partial sequence thereof of the consensus sequence B, and initiating an extension reaction.

In certain embodiments, the kit has one or more characteristics selected from the following:
(1) the oligonucleotide probe, primer I-A, primer II-A, primer I-A', primer II-A', primer I-B, primer II-B, primer II-B', primer B", bridging oligonucleotide I, bridging oligonucleotide II-I, and bridging oligonucleotide II-II each independently comprise or consist of natural nucleotides (e.g., deoxyribonucleotides or ribonucleotides), modified nucleotides, non-natural nucleotides, or any combination thereof;
(2) the oligonucleotide probes each independently have a length of 15 to 300 nt (e.g., 15 to 200 nt, 15 to 20 nt, 20 to 30 nt, 30 to 40 nt, 40 to 50 nt, 50 to 100 nt, 100 to 150 nt, 150 to 200 nt);
(3) the primer I-A, primer II-A, primer I-A', primer II-A', primer I-B, primer II-B, primer II-B', and primer B" each independently have a length of 4 to 200 nt (e.g., 5 to 200 nt, 15 to 230 nt, 26 to 115 nt, 10 to 130 nt, 10 to 20 nt, 20 to 50 nt, 20 to 30 nt, 30 to 40 nt, 40 to 50 nt, 50 to 100 nt, 100 to 150 nt, 150 to 200 nt);
(4) the bridging oligonucleotide I, bridging oligonucleotide II-I, and bridging oligonucleotide II-II each independently have a length of 6 to 200 nt (e.g., 20 to 100 nt, 20 to 70 nt, 6 to 15 nt, 15 to 20 nt, 20 to 30 nt, 30 to 40 nt, 40 to 50 nt, 50 to 100 nt, 100 to 150 nt, 150 to 200 nt);
(5) the oligonucleotide probes coupled to the same solid support have the same consensus sequence X1 and/or the same consensus sequence X2;
(6) the consensus sequence X1 of the oligonucleotide probe comprises a cleavage site; in some embodiment, the cleavage site can be cleaved or broken by nicking enzyme digestion, USER enzyme digestion, light-responsive excision, chemical excision or CRISPR-mediated excision.

In certain embodiments, the kit further comprises a reverse transcriptase, a nucleic acid ligase, a nucleic acid polymerase and/or a transposase.

In certain embodiments, the reverse transcriptase has terminal deoxynucleotidyl transferase activity. In certain embodiments, the reverse transcriptase is capable of synthesizing a cDNA strand using an RNA (e.g., mRNA) as a template, and adding a 3'-end overhang to the 3' end of the cDNA strand. In certain embodiments, the reverse transcriptase is capable of adding to the 3' end of the cDNA strand an overhang having a length of at least 1, at least 2, at least 3, at least 4, at least 5, at least 6, at least 7, at least 8, at least 9, at least 10 or more nucleotides. In certain embodiments, the reverse transcriptase is capable of adding an overhang of 2-5 cytosine nucleotides (e.g., CCC overhang) to the 3' end of the cDNA strand. In certain embodiments, the reverse transcriptase is selected from the group consisting of M-MLV reverse transcriptase, HIV-1 reverse transcriptase, AMV reverse transcriptase, telomerase reverse transcriptase, and variants, modified products and derivatives thereof having the reverse transcription activity of the above-mentioned reverse transcriptases.

In certain embodiments, the nucleic acid polymerase has no 5' to 3' exonucleolytic activity or strand displacement activity.

In certain embodiments, the nucleic acid polymerase has 5' to 3' exonucleolytic activity or strand displacement activity.

In certain embodiments, the transposase is selected from the group consisting of Tn5 transposase, MuA transposase, Sleeping Beauty transposase, Mariner transposase, Tn7 transposase, Tn10 transposase, Ty1 transposase, Tn552 transposase, as well as variants, modified products and derivatives thereof having the transposase activity of the above-mentioned transposases.

In certain embodiments, the kit further comprises: the primer C, the primer D, the primer C' and/or the primer D'. For example, the kit further comprises the primer C, the primer D and the primer D'. For example, the kit further comprises the primer C, the primer D, the primer C' and the primer D'.

In certain embodiments, the kit further comprises: a reagent for nucleic acid hybridization, a reagent for nucleic acid extension, a reagent for nucleic acid amplification, a reagent for recovering or purifying nucleic acid, a reagent for constructing transcriptome sequencing library, a reagent for sequencing (e.g., second- or third-generation sequencing), or any combination thereof.

### Definition of Terms

In the present application, unless otherwise stated, scientific and technical terms used herein have the meanings commonly understood by those skilled in the art. Moreover, the operating steps of molecular biology, biochemistry, nucleic acid chemistry, cell culture, etc., as used herein are all routine steps widely used in the corresponding fields. Meanwhile, in order to better understand the present application, definitions and explanations of relevant terms are provided below.

When the terms "e.g.", "for example", "such as", "comprise", "include", or variants thereof are used herein, these terms will not be considered as limiting terms and will instead be interpreted to mean "but not limited" or "without limitation."

Unless otherwise indicated herein or clearly contradicted by context, the terms "a" and "an" as well as "the" and similar referents in the context of describing the present application (especially in the context of the following claims) are to be construed to cover singular and plural.

As used herein, a cell that can be applied to the method of the present application (e.g., a cell that can be treated using the method of the present application to generate a population of labeled nucleic acid molecules) may be any cell of interest, such as a cancer cell, stem cell, neural cell, fetal cell, and immune cell involved in the immune response. The cell may comprise one cell or multiple cells. The cell may be a mixture of cells of the same type or a completely heterogeneous mixture of cells of different types. Different cell types may comprise cells from different tissues of an individual or cells from the same tissue from different individuals or cells derived from microorganisms of different genera, species, strains, variants, or any combination of any or all of the foregoing. For example, different cell types may comprise normal cells and cancer cells from an individual, various cell types obtained from human subjects, such as various immune cells, various different bacterial species, strains and/or variants from environmental, forensic, microbiome, or other samples; or any other various mixtures of cell types.

As used herein, the term "UMI" refers to a "Unique Molecular Identifier," which can be used to characterize and/or quantify a nucleic acid molecule. Unless otherwise indicated herein or clearly contradicted by the context, the present application does not limit the position and quantity of the UMI or complementary sequence thereof in the nucleic acid molecule. For example, when a cDNA strand comprises the UMI or complementary sequence thereof, the UMI or complementary sequence thereof can be located 3' of the cDNA sequence in the cDNA strand, or can be located 5' of the cDNA sequence, or the UMI or complementary sequence thereof can be contained both 3' and 5' of the cDNA sequence. When a complementary strand of cDNA strand comprises the UMI or complementary sequence thereof, the UMI or complementary sequence thereof can be located 3' of the complementary sequence of the cDNA sequence in the complementary strand of cDNA strand, or can also be located 5' of the complementary sequence of the cDNA sequence, or the UMI or complementary sequence thereof can be contained both 3' and 5' of the complementary sequence of the cDNA sequence.

As used in this application, "DNB" (DNA nanoball) is a typical RCA (rolling circle amplification) product, which has the characteristics of RCA products. Wherein, the RCA product is a single-stranded DNA with multiple copies of a specific sequence, which can form a similar "spherical" structure due to the interaction between the bases comprised in the DNA. Typically, a library molecule is circularized to form a single-stranded circular DNA, and subsequently the single-stranded circular DNA can be amplified by multiple orders of magnitude using the rolling circle amplification technology, thereby generating an amplification product called DNB.

As used herein, a "nucleic acid molecule population" refers to a population or collection of nucleic acid molecules, for example, nucleic acid molecules derived directly or indirectly from a target nucleic acid molecule (e.g., a double-stranded DNA, an RNA/cDNA hybrid, a single-stranded DNA, or a single-stranded RNA). In some embodiments, a nucleic acid molecule population comprises a library of nucleic acid molecules, and the library of nucleic acid molecules comprises sequences that are qualitatively and/or quantitatively representative of a target nucleic acid molecule sequence. In other embodiments, the population of nucleic acid molecules comprises a subset of the library of nucleic acid molecules.

As used herein, a "library of nucleic acid molecules" refers to a collection or population of labeled nucleic acid molecules (e.g., labeled double-stranded DNA, labeled RNA/cDNA hybrid, labeled single-stranded DNA, or labeled single-stranded RNA) or fragments thereof that are generated directly or indirectly from a target nucleic acid molecule, wherein the combination of labeled nucleic acid molecules or fragments thereof in the collection or population is shown to be qualitatively and/or quantitatively representative of the sequence of a target nucleic acid molecule sequence from which the labeled nucleic acid molecules are generated. In certain embodiments, the library of nucleic acid molecules is a sequencing library. In certain embodiments, the library of nucleic acid molecules can be used to construct a sequencing library.

As used herein, a "cDNA" or "cDNA strand" refers to a "complementary DNA" synthesized by extension using at least a portion of an RNA molecule of interest as a template via a primer that anneals to the RNA molecule of interest under catalysis of an RNA-dependent DNA polymerase or reverse transcriptase (this process is also called "reverse transcription"). The synthesized cDNA molecule is "homologous" to or "complementary" to or "to base pair" with or "to form a complex" with at least a portion of the template.

As used herein, the term "upstream" is used to describe the relative positional relationship of two nucleic acid sequences (or two nucleic acid molecules) and has a meaning commonly understood by those skilled in the art. For example, the expression "a nucleic acid sequence is located upstream of another nucleic acid sequence" means that, when aligned in the 5' to 3' direction, the former is located at a more forward position (i.e., a position closer to the 5'-end) than the latter. As used herein, the term "downstream" has the opposite meaning to "upstream."

As used herein, a "tag sequence Y", "tag sequence A", "tag sequence B", "consensus sequence X1", "consensus sequence X2", "consensus sequence A", "consensus sequence B", etc., refer to an oligonucleotide having non-target nucleic acid components that provide a means of identification, recognition, and/or molecular manipulation or biochemical manipulation (e.g., by providing a site for annealing an oligonucleotide, the oligonucleotide being, for example, a primer for DNA polymerase extension or an oligonucleotide for capture reaction or ligation reaction) for a nucleic acid molecule ligated thereto or a derivative of the nucleic acid molecule ligated thereto (e.g., a complementary fragment of the nucleic acid molecule, a short fragment of the nucleic acid molecule, etc.). The oligonucleotide may consist of at least two (preferably about 6 to 100, but there is no definite limit to the length of the oligonucleotide, and the exact size depends on many factors, while these factors in turn depend on the final function or use of the oligonucleotide) nucleotides, and can also be composed of multiple oligonucleotide fragments in a continuous or non-continuous arrangement. The oligonucleotide sequence may be unique to each nucleic acid molecule to which it is ligated, or may be unique to a certain type of nucleic acid molecule to which it is ligated. The oligonucleotide sequence may be reversibly or irreversibly ligated to a polynucleotide sequence to be "labeled" by any method including ligation, hybridization or other methods. The process of ligating the oligonucleotide sequence to a nucleic acid molecule is sometimes referred to herein as "labeling", and a nucleic acid molecule that undergoes the addition of a label or comprises a label sequence is called a "labeled nucleic acid molecule" or "tagged nucleic acid molecule."

For various reasons, the nucleic acid or polynucleotide of the present application (e.g., "tag sequence Y", "tag sequence A", "tag sequence B", "consensus sequence X1", "consensus sequence X2", "consensus sequence A", "consensus sequence B", "primer I-A", "primer I-A'", "primer I-B", "primer II-A", "primer II-A'", "primer II-B", "primer II-B'" "primer B"", "primer C", "primer D", "primer C'", "primer D'", "random primer", "bridging oligonucleotide I", "bridging oligonucleotide sequence II-I", "bridging oligonucleotide sequence II-II", etc.) may comprise one or more modified nucleic acid bases, sugar moieties, or internucleoside linkages. For example, some reasons for using nucleic acids or polynucleotides comprising modified bases, sugar moieties, or internucleoside linkages include, but are not limited to: (1) changes in Tm; (2) changes in the susceptibility of a polynucleotide to one or more nucleases; (3) providing a moiety for linking a label; (4) providing a label or label quencher; or (5) providing a moiety such as biotin for attaching another molecule in solution or bound to a surface. For example, in some embodiments, oligonucleotides such as primers can be synthesized such that the random portions comprise one or more nucleic acid analogs with constrained conformation, including, but not limited to, one or more ribonucleic acid analogs in which ribose ring is "locked" by the methylene bridge that links the 2'-O atom to the 4'-C atom; these modified nucleotides result in an increase in the Tm, or melting temperature, of each molecule by about 2 degrees Celsius to about 8 degrees Celsius. For example, in some embodiments in which an oligonucleotide primer comprising ribonucleotides is used, one indicator of using a modified nucleotide in the method may be that the oligonucleotide comprising the modified nucleotide may be digested by a single-strand specific RNase.

As used herein, the "first binding molecule" is capable of interacting specifically or non-specifically with the "first label molecule". In certain embodiments, the first binding molecule interacts with the first label molecule in a manner selected from the group consisting of interaction between positive charge and negative charge, affinity interaction (e.g., interaction between biotin and avidin, biotin and streptavidin, antigen and antibody, receptor and ligand, enzyme and cofactor), click chemistry reaction (e.g., click chemistry reaction between alkynyl-containing compound and azido compound), and any combination thereof.

For example, the first label molecule is polylysine, and the first binding molecule is a protein capable of binding to polylysine; the first label molecule is an antibody, and the first binding molecule is an antigen capable of binding to the antibody; the first label molecule is biotin, and the first binding molecule is streptavidin; the first binding molecule is a compound comprising an alkynyl group, and the label molecule is an azide compound; or, the first binding molecule is N-hydroxysulfosuccinate (NHS) ester, and the first label molecule is an amino-containing compound.

For example, the first label molecule is an antigen, and the first binding molecule is an antibody capable of binding to the antigen; the first label molecule is streptavidin, and the first binding molecule is biotin; the first binding molecule is an azide compound, and the first label molecule is an alkynyl-containing compound; or, the first binding molecule is an amino-comprising compound, and the first label molecule is N-hydroxysulfosuccinate (NHS) ester.

In the methods of the present application, for example, the nucleic acid bases in single nucleotides at one or more positions in a polynucleotide or oligonucleotide may comprise guanine, adenine, uracil, thymine or cytosine; or optionally, one or more of the nucleic acid bases may comprise modified bases such as, but not limited to, xanthine, allylamino-uracil, allylamino-thymine nucleoside, hypoxanthine, 2-aminoadenine, 5-propynyluracil, 5-propynylcytosine, 4-thiouracil, 6-thioguanine, azauracil, deazauracil, thymine nucleoside, cytosine, adenine or guanine. Furthermore, they may comprise nucleic acid bases derivatized with the following moieties: biotin moiety, digoxigenin moiety, fluorescent or chemiluminescent moiety, quenching moiety or some other moieties. The present application is not limited to the listed nucleic acid bases; the list given illustrates examples of a wide range of bases that may be used in the methods of the present application.

With respect to the nucleic acids or polynucleotides of the present application, one or more of the sugar moieties may comprise 2'-deoxyribose, or optionally, one or more of the sugar moieties may comprise some other sugar moieties, such as, but not limited to: ribose or 2'-fluoro-2'-deoxyribose or 2'-O-methyl-ribose that possesses resistance to some nucleases, or 2'-amino-2'-deoxyribose or 2'-azido-2'-deoxyribose that is labeled by reaction with a visible, fluorescent, infrared fluorescent or other detectable dye or a chemical substance with an electrophilic, photoreactive, alkynyl or other reactive chemical moiety.

The internucleoside linkages of the nucleic acids or polynucleotides of the present application may be phosphodiester linkages, or optionally, one or more of the internucleoside linkages may comprise modified linkages such as, but not limited to: phosphorothioate, phosphorodithioate, phosphoroselenate, or phosphorodiselenate linkages, which are resistant to some nucleases.

As used herein, the term "terminal deoxynucleotidyl transferase activity" refers to an ability to catalyze the template-independent addition (or "tailing") of one or more deoxyribonucleoside triphosphates (dNTPs) or single dideoxyribonucleoside triphosphates to the 3'-end of cDNA. Examples of reverse transcriptases with terminal deoxynucleotidyl transferase activity comprise, but are not limited to, M-MLV reverse transcriptase, HIV-1 reverse transcriptase, AMV reverse transcriptase, telomerase reverse transcriptase, and variants, modified products and derivatives thereof with the reverse transcription activity and terminal deoxynucleotidyl transferase activity of the reverse transcriptases. The reverse transcriptases have or do not have RNase activity (especially RNase H activity). In preferred embodiments, the reverse transcriptases used for the reverse transcription of RNA to generate cDNA do not have RNase activity. Therefore, in a preferred embodiment, the reverse transcriptase used for the reverse transcription of RNA to generate cDNA has terminal deoxynucleotidyl transferase activity, and does not have RNase activity.

As used herein, a nucleic acid polymerase with "strand displacement activity" refers to a nucleic acid polymerase that, during the process of extending a new nucleic acid strand, if it encounters a downstream nucleic acid strand complementary to the template strand, can continue the extension reaction and replace (rather than degrade) the nucleic acid strand that is complementary to the template strand.

As used herein, a nucleic acid polymerase having "5' to 3' exonucleolytic activity" refers to a nucleic acid polymerase that can catalyze the hydrolysis of 3,5-phosphodiester bonds in the order of 5' to 3' of a polynucleotide, thereby degrading nucleotides.

As used herein, a nucleic acid polymerase (or DNA polymerase) with "high fidelity" refers to a nucleic acid polymerase (or DNA polymerase) that has a lower probability of introducing erroneous nucleotides (i.e., an error rate) during the amplification of nucleic acids than the wild-type Taq enzyme (e.g., the Taq enzyme whose sequence is shown in UniProt Accession: P19821.1).

As used herein, the terms "annealed," "annealing," "anneal," "hybridized," or "hybridizing" and the like refer to the formation of complex between nucleotide sequences having sufficient complementarity to form complex via Watson-Crick base pairing. For the purposes of the present application, nucleic acid sequences that "are complementary" or "hybridize" or "anneal" to each other should be capable of forming a sufficiently stable "hybrid" or "complex" for the intended purpose. It is not required that every nucleic acid base within the sequence displayed by a nucleic acid molecule is capable of base pairing or pairing or complexing with every nucleic acid base within the sequence displayed by another nucleic acid molecule such that both nucleic acid molecules or corresponding sequences displayed therein "are complementary" or "anneal" or "hybridize" to each other. As used herein, the term "complementary" or "complementarity" is used when referring to sequences of nucleotides that are related by the rules of base pairing. For example, the sequence 5'-A-G-T-3' is complementary to the sequence 3'-T-C-A-5'. Complementarity can be "partial", in which only some of the nucleic acid bases match according to the rules of base pairing. Optionally, there may be "complete" or "total" complementarity between nucleic acids. The degree of complementarity between nucleic acid strands has a significant impact on the efficiency and strength of hybridization between the nucleic acid strands. The degree of complementarity is particularly important in amplification reactions and detection methods that rely on hybridization of nucleic acids. The term "homology" refers to the degree of complementarity of one nucleic acid sequence to another nucleic acid sequence. There may be partial homology (i.e., complementarity) or complete homology (i.e., complementarity). A partially complementary sequence is a sequence that at least partially inhibits hybridization of a fully complementary sequence to a target nucleic acid and is referred to using a functional term "substantially homologous". Inhibition of hybridization of a fully complementary sequence to a target sequence can be tested under low stringency conditions using hybridization assays (e.g., Southern blotting or Northern blotting, hybridization in solution, etc.). Substantially homologous sequences or probes will compete in or inhibit binding (i.e., hybridization) of fully homologous sequences to the target under conditions of low stringency. This is not to say that low stringency conditions are conditions that allow for nonspecific binding; low stringency conditions require that the two sequences bind to each other via a specific (i.e., selective) interaction. The absence of non-specific binding can be tested by using a second target that lacks complementarity or has only a low degree of complementarity (e.g., less than about 30% complementarity). In cases where specific binding is low or absent, the probe will not hybridize to the nucleic acid target. When used in reference to a double-stranded nucleic acid sequence such as a cDNA or genomic clone, the term "substantially homologous" means it is any oligonucleotide or probe that can hybridize to one or both strands of the double-stranded nucleic acid sequence under the low stringency conditions described herein. As used herein, the terms "annealing" or "hybridization" are used when referring to the pairing of complementary nucleic acid strands. Hybridization and hybridization strength (i.e., strength of association between nucleic acid strands) are affected by many factors known in the art, including the degree of complementarity between nucleic acids, including the stringency of conditions affected by factors such as salt concentration, the Tm (melting temperature) to form a hybrid, the presence of other components (e.g., the presence or absence of polyethylene glycol or betaine), the molar concentration of hybridized strands, and the G:C content of nucleic acid strands.

As described herein, the solid support is capable of releasing the oligonucleotide probe spontaneously or upon exposure to one or more stimuli (e.g., temperature changes, pH changes, exposure to specific chemicals or phases, exposure to light, exposure to reducing agents, etc.). It will be appreciated that the oligonucleotide probe can be released by cleavage of the bond between the oligonucleotide probe and the solid support, or degradation of the solid support itself, or both, and the oligonucleotide probe allows or is capable of being approached by other reagents.

Adding multiple types of labile bonds to the solid support enables the capability of the solid support to respond to different stimuli. Each type of labile bond can be sensitive to an associated stimulus (e.g., chemical stimulus, light, temperature, etc.) such that the release of a substance attached to the solid support through each labile bond can be controlled by applying an appropriate stimulus. In addition to thermally cleavable bonds, disulfide bonds, and UV-sensitive bonds, other non-limiting examples of labile bonds that can be coupled to the solid support comprise ester bonds (e.g., ester bonds that can be cleaved with acids, bases, or hydroxylamine), ortho diol bonds (e.g., ortho diol bonds that can be cleaved by sodium periodate), Diels-Alder bonds (e.g., Diels-Alder bonds that can be cleaved thermally), sulfone bonds (e.g., sulfone bonds that can be cleaved by alkali), silicyl ether bonds (e.g., silicyl ether bonds that can be cleaved by acids), glycosidic bonds (e.g., glycosidic bonds that can be cleaved by amylase), peptide bonds (e.g., peptide bonds that can be cleaved by proteases), or phosphodiester bonds (e.g., phosphodiester bonds that can be cleaved by nucleases (e.g., DNA enzyme)).

In addition to or as an alternative to the cleavable bonds between the solid support and the oligonucleotide described above, the solid support can be degradable, destructible or soluble spontaneously or upon exposure to one or more stimuli (e.g., temperature changes, pH changes, exposure to specific chemical substances or phases, exposure to light, exposure to reducing agents, etc.). In some cases, the solid support may be soluble such that the material components of the solid support dissolve upon exposure to specific chemicals or environmental changes (e.g., changes in temperature or changes in pH). In some cases, the solid support may degrade or dissolve under elevated temperatures and/or alkaline conditions. In some cases, the solid support may be thermally degradable such that the solid support degrades when exposed to appropriate temperature changes (e.g., heating). Degradation or dissolution of the solid support bound with a substance (e.g., an oligonucleotide probe) can result in the release of the substance from the solid support.

As used herein, the terms "transposase" and "reverse transcriptase" and "nucleic acid polymerase" refer to a protein molecule or an aggregate of protein molecules responsible for catalyzing specific chemical and biological reactions. In general, the methods, compositions or kits of the present application are not limited to the use of a specific transposase, reverse transcriptase or nucleic acid polymerase from a specific source. Rather, the methods, compositions, or kits of the present application may comprise any transposases, reverse transcriptases, or nucleic acid polymerases from any sources that have equivalent enzymatic activity to the specific enzymes of the specific methods, compositions, or kits disclosed herein. Furthermore, the methods of the present application also comprise the following embodiments: wherein any one specific enzyme provided and used in the steps of the methods is replaced by a combination of two or more enzymes, when the two or more enzymes are used in combination, whether used separately in a stepwise manner or together simultaneously, the reaction mixtures produce the same results as would be obtained using that specific enzyme. The methods, buffers, and reaction conditions provided herein, including those in the Examples, are currently preferred for embodiments of the methods, compositions, and kits of the present application. However, other enzyme storage buffers, reaction buffers, and reaction conditions can be used for some of the enzymes of the present application are known in the art and may also be suitable for use in the present application and are comprised herein.

### Beneficial effects of the present application

The present application provides a high-resolution nucleic acid array (e.g., chip) and a method capable of positionally labeling nucleic acid molecules, and a method of using the nucleic acid array or the method for high-throughput sequencing (especially, high-throughput single-cell transcriptome sequencing). The method of the present application has one or more beneficial technical effects selected from the following:
(1) The nucleic acid array (e.g., chip) has high resolution and can comprise at least 50 (e.g., at least 50, at least 100, at least 200, at least 300, at least 400, or at least 500) microdots in a single-cell area (e.g., 80 to 100 µm²), each microdot is coupled with one kind of oligonucleotide probe for labeling that comprises position information (e.g., an oligonucleotide probe comprising the tag sequence Y), each kind of oligonucleotide probe comprises at least one copy. Therefore, the nucleic acid array can be used to realize that different cells in a sample (e.g., a cell suspension) are labeled with specific positioning sequences (e.g., tag sequences Y), and thus, by detecting the specific positioning sequence (e.g., tag sequence Y) in the labeled nucleic acid molecule, the spatial position information of the nucleic acid molecule on the nucleic acid array can be determined, and then the nucleic acid molecules from the same single cell can be identified, thereby realizing the analysis of a single-cell sample.
(2) When the nucleic acid array (e.g., chip) is used to construct a high-throughput sequencing library, there is no need to perform single-cell sorting of cells in the sample (e.g., cell suspension), multiple cells can be directly adsorbed on the nucleic acid array (e.g., chip). Due to the high resolution of the nucleic acid array (e.g., chip), the size and spacing of the microdots are much smaller than the size of a single cell. Therefore, each cell (or, nucleic acid molecule from the cell) is captured and labeled with one or more oligonucleotide probes (e.g., oligonucleotide probes comprising the tag sequence Y) for labeling that carry position information and are located on the nucleic acid array (e.g., chip). In other words, the nucleic acid array (e.g., chip) can theoretically capture and label every cell in the sample, which effectively avoids the loss of rare cell information.
(3) Based on the unique design of the nucleic acid array (e.g., chip), the method of the present application can capture millions of cells on a single chip for single-cell sequencing, and the cell capture efficiency can theoretically reach 100%. That is, the cell capture throughput of the method of the present application can reach the scale of millions, and the cell capture efficiency can reach nearly 100%, far exceeding the existing technology (the existing technology, such as the 10x chromium cell sorting platform, is limited by the number of microdroplets formed in the oil phase, so that its throughput is difficult to exceed the scale of ten thousands, and due to the characteristics of Poisson distribution, its cell capture rate is theoretically 60% at most).

The preferred embodiments of the present application will be described in detail below with reference to the accompanying drawings and examples, but those skilled in the art will understand that the following drawings and examples are only used to illustrate the present application and do not limit the scope of the present application. The various objects and advantageous aspects of the present application will become apparent to those skilled in the art from the following detailed description of the accompanying drawings and preferred embodiments.

### Brief Description of the Drawings

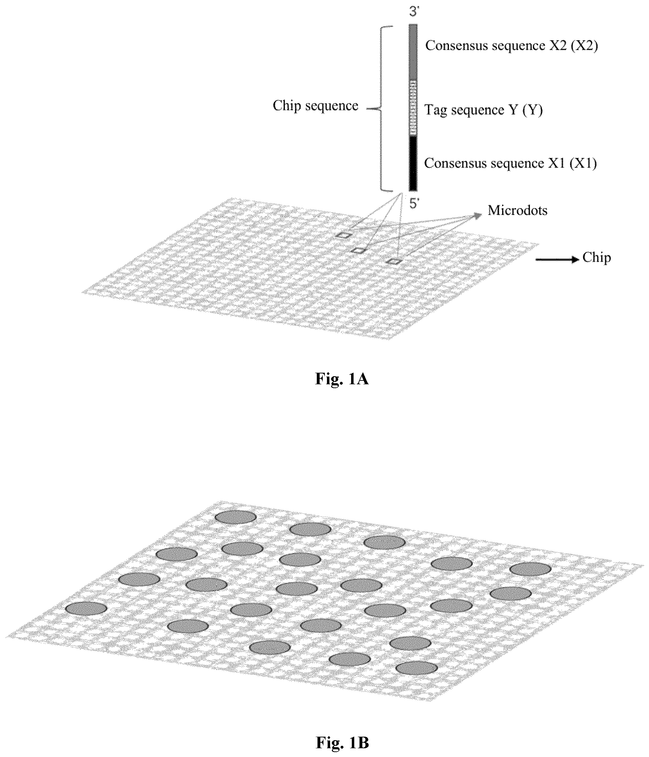

Fig. 1A shows an exemplary structure of a chip used for capturing and labeling nucleic acid molecules in the present application, which comprises: a chip and an oligonucleotide probe coupled to the chip (also referred as a chip sequence). Each oligonucleotide probe comprises a tag sequence Y corresponding to its position on the chip, and a region on the chip to which one kind of oligonucleotide probe is coupled can be called a microdot. Each oligonucleotide probe may comprise a single copy or multiple copies.
Fig. 1B shows that after the cells in a sample is contacted with the chip, they are labeled by one or more microdots on the chip.
Fig. 2 shows an exemplary scheme 1 for preparing a cDNA strand using an RNA (e.g., mRNA) in a sample as a template, and an exemplary structure of the cDNA strand. CA: consensus sequence A; CB: consensus sequence B.
Fig. 3 shows an exemplary scheme of using a chip sequence to label the 5' end of a cDNA strand (i.e., ligating the 5' end of the cDNA strand to the 3' end of the chip sequence) to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1, Y: tag sequence Y; X2: consensus sequence X2.
Fig. 4 shows an exemplary scheme 1 for preparing a complementary strand of a cDNA strand using an RNA (e.g., mRNA) in a sample as a template, and an exemplary structure of the complementary strand of the cDNA strand. CA: consensus sequence A; CB: consensus sequence B; EP: extension primer.
Fig. 5 shows an exemplary scheme of using a chip sequence to label the 5' end of a complementary strand of a cDNA strand (i.e., ligating the 5' end of the complementary strand of the cDNA strand to the 3' end of the chip sequence) to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1, Y: tag sequence Y; X2: consensus sequence X2.
Fig. 6 shows an exemplary scheme 2 for preparing a cDNA strand using an RNA (e.g., mRNA) in a sample as a template, and an exemplary structure of the cDNA strand. CA: consensus sequence A; CB: consensus sequence B.
Fig. 7 shows an exemplary scheme 1 of using a complementary sequence of a chip sequence to label the 3' end of a cDNA strand to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1; Y: tag sequence Y; X2: consensus sequence X2; P1: first region; P2: second region.
Fig. 8 shows an exemplary scheme 2 of using a complementary sequence of a chip sequence to label the 3' end of a cDNA strand to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1; Y: tag sequence Y; X2: consensus sequence X2.
Fig. 9 shows an exemplary scheme 2 for preparing a complementary strand of a cDNA strand using an RNA (e.g., mRNA) in a sample as a template, and an exemplary structure of the complementary strand of the cDNA strand. CA: consensus sequence A; CB: consensus sequence B; EP: extension primer.
Fig. 10 shows an exemplary scheme 1 of using a complementary sequence of a chip sequence to label the 3' end of a complementary strand of a cDNA strand to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1; Y: tag sequence Y; X2: consensus sequence X2; P1: first region; P2: second region.
Fig. 11 shows an exemplary scheme 2 of using a complementary sequence of a chip sequence to label the 3' end of a complementary strand of a cDNA strand to form a new nucleic acid molecule comprising the chip sequence information (i.e., a nucleic acid molecule labeled with the chip sequence), and an exemplary structure of the new nucleic acid molecule comprising the chip sequence information. CA: consensus sequence A; CB: consensus sequence B; X1: consensus sequence X1; Y: tag sequence Y; X2: consensus sequence X2.
Fig. 12 shows the gene expression profile of a subset of Hek293 cells obtained by the method of Example 1.
Fig. 13 shows a partially zoomed view of the gene expression profile of a subset of Hek293 cells obtained by the method of Example 1.
Fig. 14 shows the length distribution of the cDNA amplification product in Example 2.
Fig. 15 shows the gene expression profile of Hek293 cells obtained by the method of Example 2.
Fig. 16 shows the average numbers of genes and UMIs as captured from single cells by the method of Example 2.

### Specific Models for Carrying Out the present application

The present application will now be described with reference to the following examples which are intended to illustrate, but not to limit, the present application. Unless otherwise indicated, the experiments and methods described in the examples were performed essentially according to conventional methods well known in the art and described in various references. In addition, if the specific conditions are not specified in the examples, the conventional conditions or the conditions recommended by the manufacturer should be followed. If the manufacturer of the reagents or instruments used was not indicated, they were all conventional products that could be purchased commercially. Those skilled in the art will appreciate that the examples describe the present application by way of example and are not intended to limit the scope sought to be protected by the present application. All publications and other references mentioned herein are incorporated by reference in their entirety.

### Example 1

The sequence information involved in this example was shown in Table 1-1:

**Table 1-1: Sequence information**

| SEQ ID NO | Description | Sequence information |
|---|---|---|
| 1 | Nucleotide sequence of DNA library molecule | p- GAACGACATGGCTACGATCCGACTTNNNNNNN NNNNNNNNNNAAGTCGGAGGCCAAGCGGTCTT AGGAAGACAANNNNNNNNNNNNNNNNNNNNN NNNNCTGATAAGGTCGCCATGCCTCTCAGTACG TCAGCAGTTNNNNNNNNNNNNNNNNNCAACTC CTTGGCTCACA |
| 2 | Nucleotide sequence of DNB primer | GCCATGTCGTTCGGAACCGAGTGT |
| 3 | Nucleotide sequence of primer for synthesizing chip sequence | CTGCTGACGTACTGAGAGGCATGGCGACCTTAT CAG |
| 4 | Consensus sequence X1 | CTGCTGACGTACTGAGAGGCATGGCGACCTTAT CAG |
| 5 | Consensus sequence X2 | TTGTCTTCCTAAG |
| 6 | Nucleotide sequence of polyT primer | p- ACCGCTTGGCCTCCGNNNNNNNNNNTTTTTTTTT TTTTTTTTTTTTTTTTTV |
| 7 | TSO sequence | AAGCAGTGGTATCAACGCAGAGTACATrGrG+G |
| 8 | Chip sequence | CTGCTGACGTACTGAGAGGCATGGCGACCTTAT CAGNNNNNNNNNNNNNNNNNNNNNNNNNTTGT CTTCCTAAGAC |
| 9 | Sequence of bridging oligonucleotide | AGGCCAAGCGGTCTTAGGAAGACA |
| 10 | Sequence of cDNA amplification primer 1 | CTGCTGACGTACTGAGAGGCATG |
| 11 | Sequence of cDNA amplification primer 2 | AAGCAGTGGTATCAACGCAGAGTAC |
| 12 | Library construction primer 1 | p-CTGCTGACGTACTGAGAGGC*A*T |
| 13 | Library construction primer 2 | GAGACGTTCTCGACTCAGAAGATG |
| 14 | Sequence of primer for preparing DNB for sequencing | GGCCTCCGACTTGAGACGTTCTCG |

| | | |
|---|---|---|
| Note: "r" indicates that the nucleotide at the 3' adjacent position is a ribonucleotide; "+" indicates that the nucleotide at the 3' adjacent position comprises LNA (locked nucleotide); "*" indicates phosphorothioate modification; "p" indicates phosphorylation modification; N=A, T, C or G; V=A, C or G. | | |

I. Preparation of capture chip
1. A sequence of a DNA library molecule comprising the position information of a chip is designed, which comprised from 5' to 3': a coding sequence of a consensus sequence X1 (X1), a coding sequence of a tag sequence (Y) and a coding sequence of a consensus sequence X2 (X2). An exemplary nucleotide sequence of the DNA library molecule was shown in SEQ ID NO: 1. Beijing Liuhe BGI Co., Ltd. was entrusted to synthesize the DNA library molecules.
2. Amplification and loading of library molecules
(1) A DNBSEQ sequencing kit (purchased from MGI, Cat. No. 1000019840) was used to prepare a DNA nanoballs (DNB). The specific embodiment was briefly described below.
Briefly, 40 µL of the reaction system shown in Table 1-2 was prepared. The reaction system was placed in a PCR machine and underwent the reaction according to the following reaction conditions: 95°C for 3 minutes, 40°C for 3 minutes. After the reaction was completed, the reaction product was placed on ice, added with 40 µL of mixed enzyme I, 2 µL of mixed enzyme II (from the DNBSEQ sequencing kit), 1 µL of ATP (100 mM stock solution, obtained from Thermo Fisher), and 0.1 µL of T4 ligase (obtained from NEB, Cat. No.: M0202S). After mixing well, the above reaction system was placed in the PCR machine and reacted at 30°C for 20 minutes to generate DNB.

**Table 1-2: Reaction system for preparing DNB**

| Ingredient | Volume (µL) | Final concentration |
|---|---|---|
| DNA library molecule | X (80 fmol) | - |
| 10X phi29 buffer | 4 | 1X |
| DNB primer, 10 µM (SEQ ID NO:2; synthesized by Liuhe BGI) | 4 | 1µM |
| H₂O | 32-x | - |

(2) Subsequently, the DNB was loaded onto a BGISEQ 500 sequencing chip according to the method described in the BGISEQ 500 high-throughput sequencing reagent set (SE50) (purchased from MGI, Cat. No.: 1000012551).
In the sequencing chip, the MDA reagent in the BGISEQ500 PE50 sequencing kit (purchased from MGI, Cat. No.: 1000012554) was added, and incubated at 37°C for 30 minutes, and then the chip was washed with 5X SSC.
(3) After the chip surface was incubated with N3-PEG3500-NHS (a modification regent purchased from Sigma, Cat. No.: JKA5086) for 30 minutes to be modified with N3-PEG3500-NHS, the DBCO-modified primer for chip sequence synthesis (the sequence was shown in SEQ ID NO: 3) was pumped therein, and incubated overnight at room temperature.
3. The sequencing and decoding of position sequence information were performed. The DNB was sequenced according to the instructions of the BGISEQ-500 high-throughput sequencing reagent set, and the length of SE read was set as 25 bp. The above-mentioned DBCO-modified primer was extended to obtain a strand comprising the position sequence information during the sequencing process, and the strand was decoded to obtain the position sequence information corresponding to the DNB.
4. Continuously extension of the strand obtained in step 3 during the sequencing process: based on the above step 3, a cPAS reaction of 15 bases was continued to obtain the chip sequence (SEQ ID NO: 8, which comprised a consensus sequence X1 (SEQ ID NO: 4), a tag sequence Y, and a consensus sequence X2 (SEQ ID NO: 5)).
5. Restriction endonuclease HaeIII was used to excise the DNB, and the residual fragments of the DNB were removed by denaturation at high temperature so that only the chip sequence in step 4 remained on the chip.
6. Chip cutting: The prepared chip was cut into several small pieces. The size of the pieces was adjusted according to the experimental needs. The chip was immersed in 50mM tris buffer of pH8.0 at 4°C for later use.

### II. Single-cell fixation

1. Approximately 3000 Hek293 cells were taken and prepared into a cell suspension (PBS solution) according to conventional methods.
2. The prepared chip was taken, treated with polylysine for 30 minutes, and then dried for half an hour.
3. The cell suspension was dropped onto the treated chip, and incubated at room temperature for 30 minutes, then the chip was fixed in -20°C frozen methanol for 30 minutes.
4. The cells were permeabilized by treatment using 0.1% triton-100 for 15 minutes.

### III. In-situ synthesis of cDNA

### 1. Synthesis of cDNA

200 µL of the reverse transcriptase reaction system shown in Table 1-3 was prepared, the reaction solution was added to the chip to fully cover it, and reacted at 42°C for 90min to 180min. The synthesis of cDNA was performed with a reverse transcriptase using mRNA as a template, and using a primer comprising polyT (the sequence of the primer was shown in SEQ ID NO: 6, which comprised a consensus sequence A (CA), a UMI sequence (NNNNNNNNNN) and a polyT sequence), and a CCC overhang was added to the 3' end of the cDNA strand. After hybridization and annealing of the TSO sequence (SEQ ID NO: 7, which comprised a consensus sequence B (CB) and a GGG overhang) to the cDNA strand (through the complementary pairing between the GGG at the end of the TSO sequence and the CCC overhang of the cDNA strand), the cDNA strand was continuously extended with the reverse transcriptase using the consensus sequence B as a template, so that the 3' end of the cDNA was labeled with a c(CB) tag (a complementary sequence of the consensus sequence B).

**Table 1-3: cDNA synthesis system**

| Ingredient | Volume (µL) | Final concentration |
|---|---|---|
| Superscript II First strand buffer (5X) | 40 | 1X |
| (purchased from Thermo Fisher, Cat. No.: 18064022) | | |
| Betaine (5M) | 40 | 1M |
| (purchased from Aladdin, Cat. No.: B105554) | | |
| dNTP (10mM) | 20 | 1mM |
| MgCl₂ (100mM) | 15 | 7.5mM |
| TSO sequence (SEQ ID NO:7) | 10 | 1µM |
| (synthesized by Liuhe BGI) | | |
| polyT primer (SEQ ID NO:6) | 10 | 1µM |
| (containing phosphorylation modification at 5' end) | | |
| (synthesized by Liuhe BGI) | | |
| Superscript II RT (200U/µL) | 10 | 10U/µL |
| (purchased from Thermo Fisher, Cat. No.: 18064022) | | |
| DTT (100mM) | 10 | 5mM |
| RNase inhibitor (40U/µL) | 5 | 1U/µL |
| (purchased from Thermo Fisher, Cat. No.:N8080119) | | |
| NF H₂O | 40 | - |

The synthesized cDNA strand comprised the following sequence structure: the sequence of the reverse transcription primer (SEQ ID NO: 6) - the cDNA sequence - the sequence of c(TSO) (a complementary sequence of SEQ ID NO: 7).

### 2. Ligation of chip sequence on sequencing chip to cDNA

After the cDNA was synthesized, the chip was washed twice with 5X SSC, 1 ml of the reaction system as shown in Table 1-4 was prepared, an appropriate volume thereof was pumped into the chip to ensure that the chip was filled with the following ligation reaction solution, and the reaction was performed at room temperature for 30 minutes.

The above reaction could ligate the 5' end of the cDNA sequence to the 3' end of the chip sequence on the single-cell sequencing chip (i.e., the 5' end of the cDNA sequence was labeled with the chip sequence) to obtain a new nucleic acid molecule comprising position information (i.e., the tag sequence Y), which comprised the following sequence structure: the chip sequence (SEQ ID NO: 8) - the sequence of the reverse transcription primer (SEQ ID NO: 6) - the cDNA sequence - the sequence of c(TSO) (a complementary sequence of SEQ ID NO: 7).

After the reaction was completed, the chip was washed with 5X SSC. 200 µL of Bst polymerization reaction solution (NEB, M0275S) was prepared according to the instructions, pumped into the chip, and reacted at 65°C for 60 minutes to obtain a single-stranded nucleic acid molecule comprising position information.

**Table 1-4: Ligation system**

| Ingredient | Volume (µL) | Final concentration |
|---|---|---|
| 10X T4 ligase buffer | 100 | 1X |
| (purchased from NEB, Cat. No.: M0202S) | | |
| T4 ligase | 100 | 60U/µL |
| (purchased from NEB, Cat. No.: M0202S) | | |
| Bridging oligonucleotide (100µM, containing phosphorylation modification at 5' end) (SEQ ID NO:9) | 10 | 1µM |
| (synthesized by Liuhe BGI) | | |
| Glycerin | 10 | 10% |
| H₂O | 780 | - |

### 3. Release of cDNA

75 µL of 80 mM KOH was used to incubate the chip at room temperature for 5 minutes, the resulting liquid was collected, and then added with 10 µL of 1M, pH8.0 Tris-HCl to neutralize the cDNA recovery solution.

### 4. Amplification of cDNA

200 µL of the reaction system as shown in Table 1-5 was prepared, used for 3'-end transcriptome sequencing and library construction, and divided into 2 tubes for PCR, respectively:

**Table 1-5: System for amplification of cDNA**

| Ingredient | Volume (µL) | Final concentration |
|---|---|---|
| cDNA recovery product | 84 | - |
| Primer 1 (SEQ ID NO:10) | 8 | 0.8 µM |
| (synthesized by Liuhe BGI) | | |
| Primer 2 (SEQ ID NO:11) | 8 | 0.8 Mm |
| (synthesized by Liuhe BGI) | | |
| 2x HiFi (NEB, E2621S) | 100 | 1X |

The above reaction system was placed into a PCR machine and the reaction program was set as follows: 95°C for 3min, 11 cycles (98°C for 20s, 58°C for 20s, 72°C for 3min), 72°C for 5min, 4°C ∞ hold. After the reaction was completed, XP beads (purchased from AMPure) were used for magnetic bead-based purification and recovery. The dsDNA concentration was determined using a Qubit instrument, and the length distribution of the cDNA amplification product was detected using a 2100 bioanalyzer (purchased from Agilent).

### IV. Construction and sequencing of cDNA library

### 1. Tn5 tagmentation

According to the cDNA concentration, 20ng of cDNA (obtained in step III) was taken, added with 0.5 µM Tn5 transposase and corresponding buffer (purchased from BGI, Cat. No.: 10000028493, the method for coating Tn5 transposase was in accordance to the operation of Stereomics library preparation kit-S1), and mixed well to prepare 20 µL of the reaction system, the reaction was performed at 55°C for 10 minutes, then 5 µL of 0.1% SDS was added and mixed well at room temperature for 5 minutes to terminate the Tn5 tagmentation.

### 2. PCR amplification

100µL of the following reaction system was prepared:

**Table 1-6: Reaction system for library construction and amplification**

| Ingredient | Volume (µL) | Final concentration |
|---|---|---|
| Product after Tn5 tagmentation | 25 | - |
| 2X Hifi ready mix | 50 | 0.8µM |
| Library construction primer 1 (10µM, comprising phosphorylation modification at the 5' end and phosphorothioate modification of the last two nucleotides at the 3' end) (SEQ ID NO: 12) | 4 | 0.4µM |
| (synthesized by Liuhe BGI) | | |
| Library construction primer 2 (10µM) (SEQ ID NO: 13) | 4 | 0.4µM |
| (synthesized by Liuhe BGI) | | |
| NF H₂O | 17 | - |

After mixing, it was placed in a PCR machine and the program was set as follows: 95°C for 3min, 11 cycles (98°C for 20s, 58°C for 20s, 72°C for 3min), 72°C for 5min, 4°C ∞ hold. After the reaction was completed, XP beads were used for magnetic bead-based purification and recovery. The dsDNA concentration was determined using a Qubit instrument.

### 3. Sequencing

80 fmol of the above amplification product was taken and used to prepare DNB. 40µL of the following reaction system was prepared:

**Table 1-7: Preparation system of DNB for sequencing**

| Ingredient | Volume (µL) | Final concentration |
|---|---|---|
| Amplification product of the aforementioned step 2 | X (80fmol) | - |
| 10X phi29 buffer | 4 | 1X |
| (obtained from Thermo Fisher, Cat. No.: B62) | | |
| Primer for preparing DNB for sequencing, 10µM (SEQ ID NO: 14) | 4 | 1 µM |
| H₂O | 32-x | - |

The above reaction volume was placed in a PCR machine for reaction, and the reaction conditions were as follows: 95°C for 3 minutes, 40°C for 3 minutes. After the reaction was completed, the resulting reaction solution was placed on ice, added with 40 µL of the mixed enzyme I, 2 µL of mixed enzyme II, 1 µL of ATP, and 0.1 µL of T4 ligase required for DNB preparation in the DNBSEQ sequencing kit, and mixed well, and the above reaction system was placed in a PCR machine at 30°C and reacted for 20 minutes to form DNB.

According to the method described in the PE50 kit supporting MGISEQ 2000, the DNB was loaded onto the sequencing chip of MGISEQ 2000, to perform sequencing according to the relevant instructions. The PE50 sequencing model was selected, in which the sequencing of the first strand was divided into two sections of sequencing, that was, the sequencing of 25bp was performed first, followed by 15 cycles of dark reaction, and then the sequencing of 10bp UMI sequence was performed, and the sequencing of the second strand was performed by setting 50bp for sequencing.

### V. Data analysis:

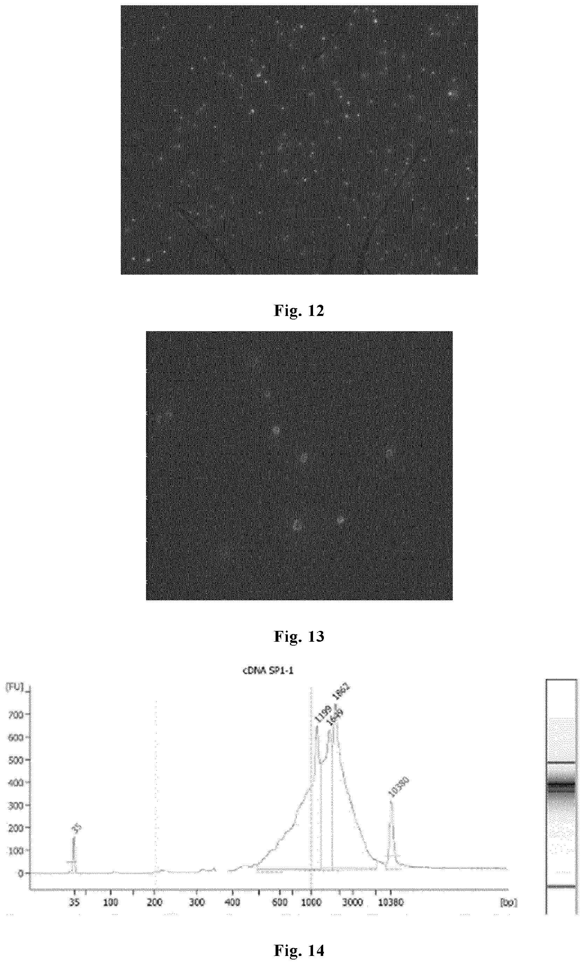

1. Data analysis was carried out by logging into the website http://stereomap.cngb.org/Stereo-Draftsman/report/index and following the website operation guide. For the read1 sequence obtained in PE50 sequencing (from the first strand sequencing), the first 25 bp sequence was aligned with the 25 bp position information during the chip preparation process, and the reads aligned well with the position information on the chip were retained, and mapped to the corresponding chip positions. The read2 (from the second strand sequencing) corresponding to the reads mapped to the chip positions was found out, the read2 was aligned with the human genome, the duplicate reads were removed based on the UMI information, and thus the genes captured from each cell and the number of reads per gene were obtained.
2. A part of the chip was taken, and its analysis results were shown in Fig. 12 and Fig. 13, in which Fig. 13 shows a part of a zoomed view of cells in Fig. 12. From the figure, it could be seen that single cells were scattered on the chip. When one of the cells was circled, the average number of captured genes and UMI numbers of the cell could be obtained.

### Example 2

The sequence information involved in this example was shown in Table 2-1 and Table 1-1:

**Table 2-1: Sequence information**

| SEQ ID NO | Description | Sequence information |
|---|---|---|
| 15 | Probe capture sequence | p- TTGTCTTCCTAAGACNNNNNNNNNNTTTTTT TTTTTTTTTTTTTTTTTTV |
| 16 | Probe sequence | CTGCTGACGTACTGAGAGGCATGGCGACCTT ATCAGNNNNNNNNNNNNNNNNNNNNNNNN NTTGTCTTCCTAAGACNNNNNNNNNNTTTTT TTTTTTTTTTTTTTTTTV |
| 17 | TSO sequence | CTGCTGACGTACTGAGAGGCrGrG+G |
| 18 | cDNA amplification primer | CTGCTGACGTACTGAGAGGCATG |
| 19 | sequence of first strand for Tn5 coating | GAGACGTTCTCGACTCAGCAGAAGATGTGT ATAAGAGACAG |
| 20 | sequence of second strand for Tn5 coating | p-CTGTCTCTTATACACATCT |
| 21 | Library construction primer 2 | GAGACGTTCTCGACTCAGCAGA |
| 22 | Sequence of primer for preparing DNB for sequencing | GTACGTCAGCAGGAGACGTTCTCG |

| | | |
|---|---|---|
| Note: "r" indicates that the nucleotide at the 3' adjacent position is ribonucleotide; "+" indicates that the nucleotide at the 3' adjacent position comprises LNA (locked nucleotide); "*" indicates phosphorothioate modification; "p" indicates phosphorylation modification; N=A, T, C or G; V=A, C or G. | | |

### I. Preparation of capture chip

1. A DNA library sequence comprising position information was designed and synthesized, and its nucleotide sequence was shown in SEQ ID NO: 1. Beijing Liuhe BGI Co., Ltd. was entrusted to perform the sequence synthesis.
2. In-situ amplification of library
(1) Preparation of DNA nanoball (DNB): 40 µL of the reaction system as shown in Table 2-2 was prepared, and added with 80 fmol of the DNA library sequence comprising position sequence information.

**Table 2-2: Reaction system for preparing DNB**

| Ingredient | Volume (µL) | Final concentration |
|---|---|---|
| DNA library sequence comprising position sequence information | 80fmol (X) | - |
| 10X phi29 buffer | 4 | 1X |
| Synthetic DNB primer sequence, 10µM (SEQ ID NO: 2, synthesized by Liuhe BGI) | 4 | 1 µM |
| H₂O | 32-x | - |

The above reaction volume was placed in a PCR machine for reaction, and the reaction conditions were as follows: 95°C for 3 minutes, 40°C for 3 minutes. After the reaction was completed, the resulting reaction solution was placed on ice, added with 40 µL of mixed enzyme I, 2 µL of mixed enzyme II, 1 µL of ATP (100mM stock solution, Thermo Fisher), and 0.1 µL of T4 ligase (purchased from NEB, Cat. No.: M0202S) required for DNB preparation in the DNB SEQ sequencing kit, and mixed well, and the above reaction system was placed in a PCR machine at 30°C, and reacted for 20 minutes to form DNB. The DNB was loaded onto an SEQ 500 sequencing chip according to the method described in the BGISEQ-500 high-throughput sequencing reagent set (SE50).
(2) The MDA reagent in the PE50 sequencing kit (purchased from MGI, Cat. No.: 1000012554) was added into the sequencing chip, and incubated at 37°C for 30 minutes, and then the chip was washed with 5X SSC.
(3) The chip surface was incubated with N3-PEG3500-NHS (purchased from Sigma, Cat. No.: JKA5086) to be modified with N3-PEG3500-NHS, and after the incubation for 30 minutes, the DBCO-modified primer (SEQ ID NO: 3) was pumped therein and incubated at room temperature overnight.
3. The sequencing and decoding of position sequence information were performed. The DNB was sequenced according to the instructions of the BGISEQ-500 high-throughput sequencing reagent set, and the length of SE read was set as 25 bp. The above-mentioned DBCO-modified primer was extended to obtain a strand comprising a position sequence information during the sequencing process, and the strand was decoded to obtain the position sequence information corresponding to the DNB.
4. Liuhe BGI was entrusted to synthesize the probe capture sequence with a UMI (SEQ ID NO: 15, with phosphorylate modification at its 5' end), and T4 ligase was used to ligate the capture sequence to the strand obtained during the sequencing process according to the following reaction system. The ligation reaction system was shown in Table 2-3.

**Table 2-3: Ligation reaction system**

| Ingredient | Volume (µL) | Final concentration |
|---|---|---|
| Probe capture sequence with UMI (SEQ ID NO: 15, 10µM) | 10 | 1µM |
| 10X T4 ligase buffer (purchased from NEB, Cat. No.: M0202S) | 10 | 1X |
| T4 ligase (purchased from NEB, Cat. No.: M0202S), 100 U/µL | 1 | 1U/µL |
| H₂O | 79 | - |

5. Restriction endonucleases HaeIII and MboI were used to excise DNB, and the residual fragments of the DNB were removed by denaturation at high temperature, so that only the probe comprising position information and capture sequence remained on the chip (its sequence was shown in SEQ ID NO: 16):
CTGCTGACGTACTGAGAGGCATGGCGACCTTATCAGNNNNNNNNNNNNNNNNN NNNNTTGTCTTCCTAAGACNNNNNNNNNNTTTTTTTTTTTTTTTTTTTTV, wherein NNNNNNNNNNNNNNNNNNNNNNN represented position information.
6. Chip cutting: The prepared chip was cut into several small pieces. The size of the pieces was adjusted according to the experimental needs. The chip was immersed in 50mM tris buffer of pH8.0 at 4°C for later use.

### II. Single-cell fixation

1. Approximately 3000 Hek293 cells were taken and prepared into a cell suspension (PBS solution) according to conventional methods.
2. The prepared chip was taken, treated with polylysine for 30 minutes, and then dried for half an hour.
3. The cell suspension was dropped onto the treated chip, and incubated at room temperature for 30 minutes, then the chip was fixed in -20°C frozen methanol for 30 minutes.
4. The cells were permeabilized by treatment using 0.1% triton-100 for 15 minutes.

### III. In-situ synthesis of cDNA

1. Synthesis of cDNA. The chip was washed twice with 5X SSC at room temperature. 200 µL of the reverse transcriptase reaction system as shown in Table 2-4 was prepared, the reaction solution was added to the chip containing cells to fully cover them, the reaction was carried out at 42°C for 90min to 180min, and the polyT of the probe on the chip was used as a primer to synthesize cDNA, Wherein the 3' end of the cDNA was labeled with a TSO tag for the synthesis of a complementary strand of the cDNA. The cDNA strand was as follows:
CTGCTGACGTACTGAGAGGCATGGCGACCTTATCAGNNNNNNNNNNNNNNNNN NNNNTTGTCTTCcTAAGACNNNNNNNNTTTTTTTTTTTTTTTTTTTTV(cDNA)CCCGCC TCTCAGTACGTCAGCAG, the RNase H treatment was performed for 30 minutes to digest RNA.

**Table 2-4: Reverse transcriptase reaction system**

| Ingredient | Volume (µL) | Final concentration |
|---|---|---|
| Superscript II First strand buffer (5X) (purchase from Thermo Fisher, Cat. No.: 18064022) | 40 | 1X |
| Betaine (5M) (purchased from Aladdin, Cat. No.: B105554) | 40 | 1M |
| dNTP (10mM) | 20 | 1mM |
| MgCl₂ (100mM) | 15 | 7.5mM |
| TSO sequence (50µM) (SEQ ID NO: 17, synthesized by Liuhe BGI) | 10 | 1µM |
| Superscript II RT (200U/µL) (purchased from Thermo Fisher, Cat. No.: 18064022) | 10 | 10U/µL |
| DTT (100mM) | 10 | 5mM |
| RNase inhibitor (40U/µL) (purchased from Thermo Fisher, Cat. No.: N8080119) | 5 | 1U/µL |
| NF H₂O | 50 | - |

3. cDNA release. 75 µL of 200 mM KOH was used to incubate the chip at room temperature for 5 minutes, the resulting liquid was collected and then added with 15 µL of 1M pH8.0 Tris-HCl to neutralize the cDNA recovery solution.

4. cDNA amplification. 200 µL of the reaction system as shown in Table 2-5 was prepared for construction of the 3' and 5' transcriptome libraries, respectively, which were each divided into 2 tubes for PCR:

**Table 2-5: System for cDNA amplification**

| Ingredient | Volume (µL) | Final concentration |
|---|---|---|
| cDNA recovery product | 90 | - |
| cDNA amplification primer (SEQ ID NO: 18, synthesized by Liuhe BGI) | 10 | 1µM |
| 2x HiFi ready mix | 100 | 1X |

The above reaction system was placed in a PCR machine and the reaction program was set as follows: 95°C for 3 minutes, 11 cycles (98°C for 20 seconds, 58°C for 20 seconds, 72°C for 3 minutes), 72°C for 5 minutes, and 4°C ∞ hold. After the reaction was completed, XP beads were used for magnetic bead-based purification and recovery. The dsDNA concentration was determined using the Qubit kit, and the cDNA fragment distribution was detected using a 2100 bioanalyzer (purchased from Agilent). The detection results are shown in Fig. 14, and the cDNA length was expected.

### IV. Library construction and sequencing of cDNA library

1. Cleavage and tagmentation with Tn5. According to the cDNA concentration, 20ng of the cDNA was taken, added with 0.5 µM Tn5 transposase (which was coated with the first strand as shown in SEQ ID NO: 19 and the second strand as shown in SEQ ID NO: 20) and corresponding buffer (purchased from BGI, Cat. No.: 10000028493, the method for coating Tn5 transposase was in accordance to the operation of Stereomics library preparation kit), and mixed well to prepare 20 µL of the reaction system, the reaction was performed at 55°C for 10 minutes, then 5 µL of 0.1% SDS was added and mixed well at room temperature for 5 minutes to terminate the cleavage and tagmentation.
2. PCR amplification. 100 µL of the reaction system as shown in Table 2-6 was prepared:

**Table 2-6: Reaction system for PCR amplification**

| Ingredient | Volume (µL) | Final concentration |
|---|---|---|
| Product after cleavage and tagmentation with Tn5 | 25 | |
| 2X Hifi ready mix | 50 | 0.8µM |
| Library construction primer 1 (with phosphorylate modification at the 5' end, phosphorothioate modification of two nucleotides at the 3' end; SEQ ID NO: 12, 10 µM) (synthesized by Liuhe BGI) | 4 | 0.4µM |
| Library construction primer 2 (SEQ ID NO: 21, synthesized by Liuhe BGI) | 4 | 0.4µM |
| NF H₂O | 17 | - |

After mixing, it was loaded in a PCR machine and the following program was set: 95°C for 3 minutes, 11 cycles (98°C for 20 seconds, 58°C for 20 seconds, 72°C for 3 minutes), 72°C for 5 minutes, and 4°C ∞ hold. After the reaction was completed, XP beads were used for magnetic bead-based purification and recovery. The dsDNA concentration was determined using the Qubit kit.
3. Sequencing. 80 fmol of the above amplification product was taken to prepare DNB. 40µL of the reaction system as shown in Table 2-7 was prepared:

**Table 2-7: Preparation system of DNB for sequencing**

| Ingredient | Volume (µL) | Final concentration |
|---|---|---|
| Amplification product of step 2 above | 80fmol (X) | - |
| 10X phi29 buffer (purchased from Thermo Fisher, Cat. NO.: B62) | 4 | 1X |
| DNB primer sequence, 10µM (SEQ ID NO: 22, synthesized by Liuhe BGI) | 4 | 1µM |
| H₂O | 32-x | - |

The above reaction volume was placed in a PCR machine for reaction, and the reaction conditions were as follows: 95°C for 3 minutes, 40°C for 3 minutes. After the reaction was completed, the resulting reaction solution was placed on ice, added 40 µL of mixed enzyme I, 2 µL of mixed enzyme II, 1µL of ATP (100mM stock solution, Thermo Fisher), and 0.1µL of T4 ligase required for DNB preparation in the DNBSEQ sequencing kit, and mixed well, then the above reaction system was placed in a PCR machine at 30°C and reacted for 20 minutes to form

### DNB.

According to the method described in the PE50 kit supporting MGISEQ 2000, the DNB was loaded onto the sequencing chip of MGISEQ 2000, to perform sequencing according to the relevant instructions. The PE50 sequencing model was selected, in which the sequencing of the first strand was divided into two sections of sequencing, that was, the sequencing of 25bp was performed first, followed by 15 cycles of dark reaction, and then the sequencing of 10bp UMI sequence was performed, and the sequencing of the second strand was performed by setting 50bp for sequencing.

### V. Data analysis:

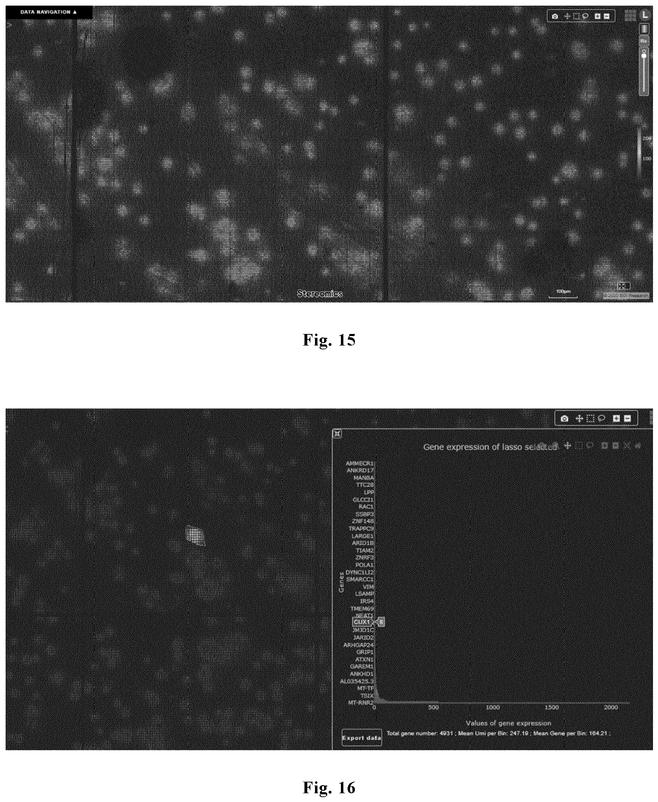

1. Data analysis was carried out by logging into the website http://stereomap.cngb.org/Stereo-Draftsman/report/index and following the website operation guide. For the read1 sequence in PE50, the first 25 bp was aligned with the 25 bp position information during the chip preparation process, and the reads aligned well with the position information on the chip were retained, and mapped to the corresponding chip positions. The read2 corresponding to the reads mapped to the chip positions was aligned with the human genome, the duplicate reads were removed based on the UMI information, and thus the genes captured from each cell and the number of reads per gene were obtained.
2. The analysis results were shown in Fig. 15 and Fig. 16: a part of the chip was taken, in which it could be seen that a large number of single cells were scattered on the chip. When one of the cells was circled, the average number of captured genes and UMI numbers of the cell could be obtained.

Although the specific embodiments of the present application have been described in detail, those skilled in the art will understand that various modifications and changes can be made to the details based on all teachings that have been disclosed, and these changes are all within the protection scope of the present application. The full scope of the present application is given by the appended claims and any equivalents thereof.

## Claims

1. A method for generating a population of labeled nucleic acid molecules, which comprises the following steps:
(1) providing: a sample containing one or more cells, and a nucleic acid array;
wherein, the sample is a single-cell suspension; the cell comprises (e.g., on its surface) a first binding molecule;
the nucleic acid array comprises a solid support, the solid support comprises (e.g., on its surface) a first label molecule, and the first binding molecule is capable of forming an interaction pair with the first label molecule;
and, the solid support further comprises a plurality of microdots, the size (e.g., equivalent diameter) of the microdots is less than 5 µm, and the center-to-center distance between adjacent microdots is less than 10 µm; each microdot is coupled with one kind of oligonucleotide probe, each kind of oligonucleotide probe comprises at least one copy; the oligonucleotide probe in the direction from 5' to 3' comprises or consists of: a consensus sequence X1, a tag sequence Y and a consensus sequence X2, wherein,
oligonucleotide probes coupled to different microdots have different tag sequences Y;
(2) contacting the one or more cells with the solid support of the nucleic acid array, whereby each cell individually occupies at least one microdot in the nucleic acid array (i.e., each cell is individually contacted with at least one microdot in the nucleic acid array), and allowing the first binding molecule of the cell to interact with the first label molecule of the solid support; wherein,
before or after contacting the one or more cells with the nucleic acid array, performing a pretreatment, including reverse transcription, on an RNA (e.g., an mRNA) of the one or more cells to generate a first nucleic acid molecule population;
and,
(3) associating the first nucleic acid molecule population derived from each cell obtained in the previous step with an oligonucleotide probe coupled to the microdot occupied by the cell from which the first nucleic acid molecule population is derived, thereby generating a second nucleic acid molecule population labeled with the tag sequence Y.

2. The method according to claim 1, wherein the center-to-center distance between adjacent microdots is less than 10 µm, less than 5 µm, less than 1 µm, less than 0.5 µm, less than 0.1 µm, less than 0.05 µm, or less than 0.01 µm; and, the size (e.g., equivalent diameter) of the microdots is less than 5 µm, less than 1 µm, less than 0.3 µm, less than 0.5 µm, less than 0.1 µm, less than 0.05 µm, less than 0.01 µm, or less than 0.001 µm;
preferably, the center-to-center distance between adjacent microdots is 0.5 µm to 1 µm, such as 0.5 µm to 0.9 µm, 0.5 µm to 0.8 µm;
preferably, the size (e.g., equivalent diameter) of the microdots is 0.001 µm to 0.5 µm (e.g., 0.01 µm to 0.1 µm, 0.01 µm to 0.2 µm, 0.2 µm to 0.5 µm, 0.2 µm to 0.4 µm, 0.2 µm to 0.3 µm).

3. The method according to claim 1 or 2, wherein the first binding molecule is capable of forming a specific interaction pair or a non-specific interaction pair with the first label molecule;
preferably, the interaction pair is selected from the group consisting of an interaction pair of positive and negative charges, affinity interaction pair (e.g., biotin/avidin, biotin/streptavidin, antigen/antibody, receptor/ligand, enzyme/cofactor), a pair of molecules capable of undergoing click chemical reaction (e.g., alkynyl-containing compound/azide compound), N-hydroxysulfosuccinate (NHS) ester/amino-containing compound, and any combination thereof;
for example, the first label molecule is polylysine, the first binding molecule is a protein capable of binding to polylysine; the first label molecule is an antibody, and the first binding molecule is an antigen capable of binding to the antibody; the first label molecule is an amino-containing compound, and the first binding molecule is a N-hydroxysulfosuccinate (NHS) ester; or, the first label molecule is biotin, and the first binding molecule is streptavidin.

4. The method according to any one of claims 1 to 3, wherein the first binding molecule is naturally present in the cell.

5. The method according to any one of claims 1 to 3, wherein the first binding molecule is unnaturally present in the cell;
preferably, the method further comprises a step of binding the first binding molecule to the one or more cells or causing the one or more cells to express the first binding molecule, so as to provide the cell sample of step (1).

6. The method according to any one of claims 1 to 5, wherein the method further comprises a step of binding the first label molecule to the solid support, so as to provide the nucleic acid array of step (1).

7. The method according to any one of claims 1 to 6, wherein in step (2), the pretreatment comprises:
(i) using a primer I-A to reverse-transcribe the RNA (e.g., mRNA) of the one or more cells to generate an extension product as a first nucleic acid molecule to be labeled, thereby generating the first nucleic acid molecule population; wherein, the primer I-A comprises a consensus sequence A and a capture sequence A, the capture sequence A is capable of annealing to the RNA (e.g., mRNA) to be captured and initiating an extension reaction; the consensus sequence A is located upstream of the capture sequence A (e.g., located at the 5' end of the primer I-A);
or,
(ii) (a) using a primer I-A to reverse-transcribe the RNA (e.g., mRNA) of the one or more cells to generate a cDNA strand, in which the cDNA strand comprises a cDNA sequence that is formed by reverse transcription primed by the primer I-A and complementary to the RNA (e.g., mRNA), and a 3'-end overhang; wherein, the primer I-A comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., located at the 5' end of the primer I-A); and, (b) annealing a primer I-B to the cDNA strand generated in (a) and initiating an extension reaction to generate a first extension product as a first nucleic acid molecule to be labeled, thereby generating the first nucleic acid molecule population; wherein, the primer I-B comprises a consensus sequence B, a complementary sequence of the 3'-end overhang, and optionally a tag sequence B; the complementary sequence of the 3'-end overhang is located at the 3' end of the primer I-B; the consensus sequence B is located upstream of the complementary sequence of the 3'-end overhang (e.g., located at the 5' end of the primer I-B);
or,
(iii) (a) using a primer I-A' to reverse-transcribe the RNA (e.g., mRNA) of the one or more cells to generate a cDNA strand, in which the cDNA strand comprises a cDNA sequence that is formed by reverse transcription primed by the primer I-A' and complementary to the RNA (e.g., mRNA), and a 3'-end overhang; wherein, the primer I-A' comprises a capture sequence A, and the capture sequence A is capable of annealing to the RNA (e.g., mRNA) to be captured and initiating an extension reaction; (b) annealing a primer I-B to the cDNA strand generated in (a), and performing an extension reaction to generate a first extension product; wherein, the primer I-B comprises a consensus sequence B, a complementary sequence of the 3'-end overhang, and optionally a tag sequence B; the complementary sequence of the 3'-end overhang is located at the 3' end of the primer I-B; the consensus sequence B is located upstream of the complementary sequence of the 3'-end overhang (e.g., located at the 5' end of the primer I-B); and, (c) providing an extension primer, using the first extension product as a template to perform an extension reaction to generate a second extension product as a first nucleic acid molecule to be labeled, thereby generating the first nucleic acid molecule population;
and,
in step (3), generating a second nucleic acid molecule population labeled by the tag sequence Y by associating the first nucleic acid molecule population derived from each cell obtained in the previous step with an oligonucleotide probe coupled to the microdot occupied by the cell from which the first nucleic acid molecule population is derived, which comprises:
under a condition that allows annealing, contacting a bridging oligonucleotide I with the first nucleic acid molecule that is derived from each cell and obtained in step (2) and the oligonucleotide probe coupled to the microdot occupied by the cell, annealing (e.g., in situ annealing) the bridging oligonucleotide I to the first nucleic acid molecule that is derived from each cell and obtained in step (2) and the oligonucleotide probe coupled to the microdot occupied by the cell, and ligating the first nucleic acid molecule and the oligonucleotide probe on the array annealed with the bridging oligonucleotide I to obtain a ligation product as a second nucleic acid molecule with a positioning tag, thereby generating the second nucleic acid molecule population;
wherein, the bridging oligonucleotide I comprises: a first region and a second region, and optionally a third region located between the first region and the second region, the first region is located upstream of the second region (e.g., located 5' of the second region); wherein,
the first region is capable of annealing to the whole or a part of the consensus sequence A of the primer I-A in step (2)(i) or step (2)(ii) or annealing to the whole or a part of the consensus sequence B of the primer I-B in step (2)(iii);
the second region is capable of annealing to the whole or a part of the consensus sequence X2.

8. The method according to claim 7, wherein in step (3), the first region and the second region of the bridging oligonucleotide I are directly adjacent, and the ligation of the first nucleic acid molecule to the oligonucleotide probe comprises: using a nucleic acid ligase to ligate the nucleic acid molecule hybridized with the first region and the nucleic acid molecule hybridized with the second region of the same bridging oligonucleotide I, to obtain a ligation product as a second nucleic acid molecule with a positioning tag; or,
the bridging oligonucleotide I comprises a first region, a second region and a third region located between them, and the ligation of the first nucleic acid molecule to the oligonucleotide probe comprises: using a nucleic acid polymerase to perform a polymerization reaction with the third region as a template, and using a nucleic acid ligase to ligate the nucleic acid molecule hybridized with the first region and the nucleic acid molecule hybridized with the third region and the second region of the same bridging oligonucleotide I, to obtain a ligation product as a second nucleic acid molecule with a positioning tag; preferably, the nucleic acid polymerase has no 5' to 3' exonucleolytic activity or strand displacement activity.

9. The method according to claim 7 or 8, which comprises step (1), step (2)(i) and step (3); wherein the ligation product obtained in step (3) is taken as the second nucleic acid molecule with a positioning tag.

10. The method according to claim 9, wherein in step (2)(i), the capture sequence A is a random oligonucleotide sequence;
preferably, in step (3), the ligation product derived from each copy of the oligonucleotide probe coupled to the same microdot has a different capture sequence A, and the capture sequence A serves as a unique molecular identifier (UMI) of the second nucleic acid molecule.

11. The method according to claim 9, wherein in step (2)(i), the capture sequence A is a poly(T) sequence or a specific sequence targeting a target nucleic acid; wherein the primer I-A further comprises a tag sequence A, such as a random oligonucleotide sequence;
preferably, in step (3), the ligation product derived from each copy of the oligonucleotide probe coupled to the same microdot has a different tag sequence A as a UMI.

12. The method according to claim 7 or 8, which comprises step (1), step (2)(ii) and step (3); wherein the ligation product obtained in step (3) is taken as the second nucleic acid molecule with a positioning tag.

13. The method according to claim 12, wherein in step (2)(ii)(a), the capture sequence A is a random oligonucleotide sequence;
preferably, in step (3), the ligation product derived from each copy of the oligonucleotide probe coupled to the same microdot has a different capture sequence A, and the capture sequence A serves as a unique molecular identifier (UMI) of the second nucleic acid molecule.

14. The method according to claim 12, wherein in step (2)(ii)(a), the capture sequence A is a poly(T) sequence or a specific sequence targeting a target nucleic acid; wherein the primer I-A further comprises a tag sequence A, such as a random oligonucleotide sequence;
preferably, in step (3), the ligation product derived from each copy of the oligonucleotide probe coupled to the same microdot has a different tag sequence A as a UMI.

15. The method according to any one of claims 9 to 14, wherein the primer I-A comprises a 5' phosphate at the 5' end.

16. The method according to claim 7 or 8, which comprises step (1), step (2)(iii) and step (3); wherein the ligation product obtained in step (3) is taken as the second nucleic acid molecule with a positioning tag.

17. The method according to claim 16, wherein in step (2)(iii)(c), the extension primer is the primer I-B or a primer B", and the primer B" is capable of annealing to a complementary sequence or partial sequence thereof of the consensus sequence B and initiating the extension reaction.

18. The method according to claim 16 or 17, wherein in step (2)(iii)(a), the capture sequence A of the primer I-A' is a random oligonucleotide sequence; wherein, in step (2)(iii)(b), the primer I-B comprises the consensus sequence B, a complementary sequence of the 3'-end overhang, and the tag sequence B.

19. The method according to claim 16 or 17, wherein in step (2)(iii)(a), the capture sequence A of the primer I-A' is a poly(T) sequence or a specific sequence targeting a target nucleic acid; wherein, the primer I-B comprises the consensus sequence B, a complementary sequence of the 3'-end overhang, and the tag sequence B;
preferably, in step (3), the ligation product derived from each copy of the oligonucleotide probe coupled to the same microdot has a different tag sequence B as a UMI.

20. The method according to any one of claims 16 to 19, wherein the extension primer comprises a 5' phosphate at the 5' end.

21. The method according to any one of claims 16 to 20, wherein, in step (2)(iii)(b), the cDNA strand anneals through its 3'-end overhang to the primer I-B, and, the cDNA strand is extended using the primer I-B as a template to generate the first extension product under the presence of a nucleic acid polymerase (e.g., a DNA polymerase or reverse transcriptase).

22. The method according to any one of claims 1 to 6, wherein in step (2), the pretreatment comprises:
(i) (a) using a primer II-A to reverse-transcribe the RNA (e.g., mRNA) of the one or more cells to generate a cDNA strand, in which the cDNA strand comprises a cDNA sequence that is formed by reverse transcription primed by the primer II-A and complementary to the RNA (e.g., mRNA), and a 3'-end overhang; wherein, the primer II-A comprises a capture sequence A, and the capture sequence A is capable of annealing to the RNA (e.g., mRNA) to be captured and initiating the extension reaction; and, (b) annealing a primer II-B to the cDNA strand generated in (a) and performing an extension reaction to generate a first extension product as a first nucleic acid molecule to be labeled, thereby generating the first nucleic acid molecule population; wherein, the primer II-B comprises a consensus sequence B, a complementary sequence of the 3'-end overhang, and optionally a tag sequence B; the complementary sequence of the 3'-end overhang is located at the 3' end of the primer II-B; the consensus sequence B is located upstream of the complementary sequence of the 3'-end overhang (e.g., located at the 5' end of the primer II-B); or,
(ii) (a) using a primer II-A' to reverse-transcribe the RNA (e.g., mRNA) of the one or more cells to generate a cDNA strand; in which the cDNA strand comprises a cDNA sequence that is formed by reverse transcription primed by the primer II-A' and complementary to the RNA (e.g., mRNA), and a 3'-end overhang; wherein, the primer II-A' comprises a consensus sequence A and a capture sequence A, and the capture sequence A is capable of annealing to the RNA (e.g., mRNA) to be captured and initiating an extension reaction; the consensus sequence A is located upstream of the capture sequence A (e.g., located at the 5' end of the primer II-A'); (b) annealing a primer II-B' to the cDNA strand generated in (a), and performing an extension reaction to generate a first extension product; wherein, the primer II-B' comprises a consensus sequence B and a complementary sequence of the 3'-end overhang, and optionally a tag sequence B; the complementary sequence of the 3'-end overhang is located at the 3' end of the primer II-B'; the consensus sequence B is located upstream of the complementary sequence of the 3'-end overhang (e.g., located at the 5' end of the primer II-B'); and, (c) providing an extension primer, using the first extension product as a template to perform an extension reaction to generate a second extension product as a first nucleic acid molecule to be labeled, thereby generating the first nucleic acid molecule population;
and, in step (3), generating a second nucleic acid molecule population labeled by the tag sequence Y by associating the first nucleic acid molecule population derived from each cell obtained in the previous step with the oligonucleotide probe coupled to the microdot occupied by the cell from which the first nucleic acid molecule population is derived, which comprises:
(i) annealing (e.g., in-situ annealing) the first nucleic acid molecule derived from each cell obtained in step (2) to the oligonucleotide probe coupled to the microdot occupied by the cell, by applying an annealing condition to the product of step (2), and performing an extension reaction to generate an extension product as a second nucleic acid molecule with a positioning tag, thereby generating the second nucleic acid molecule population; wherein, the consensus sequence X2 or partial sequence thereof of the oligonucleotide probe is (a) capable of annealing to a complementary sequence or partial sequence thereof of the consensus sequence B of the first extension product obtained in step (2)(i), or (b) capable of annealing to a complementary sequence or partial sequence thereof of the consensus sequence A of the second extension product obtained in step (2)(ii), or,
(ii) under a condition that allows annealing, contacting a bridging oligonucleotide pair with the first nucleic acid molecule derived from each cell obtained in step (2) and the oligonucleotide probe coupled to the microdot occupied by the cell, and annealing (e.g., in-situ annealing) the bridging oligonucleotide pair to the first nucleic acid molecule derived from each cell obtained in step (2) and the oligonucleotide probe coupled to the microdot occupied by the cell,
wherein, the bridging oligonucleotide pair is composed of a bridging oligonucleotide II-I and a bridging oligonucleotide II-II, the bridging oligonucleotide II-I and the bridging oligonucleotide II-II each independently comprise: a first region, a second region, and optionally a third region located between the first region and the second region, and the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is (a) capable of annealing to a complementary sequence or partial sequence thereof of the consensus sequence B of the first extension product obtained in step (2)(i), or, (b) capable of annealing to a complementary sequence or partial sequence thereof of the consensus sequence A of the second extension product obtained in step (2)(ii);
wherein, among the bridging oligonucleotide pair to be contacted with the first nucleic acid molecule and the oligonucleotide probe, the bridging oligonucleotide II-I and the bridging oligonucleotide II-II of the bridging oligonucleotide pair each exist in a single-strand form, or, the bridging oligonucleotide II-I and the bridging oligonucleotide II-II of the bridging oligonucleotide pair are annealed to each other and exist in a partially double-strand form;
performing a ligation reaction: ligating the nucleic acid molecule hybridized with the first region and the nucleic acid molecule hybridized with the second region of the same bridging oligonucleotide II-I, and/or, ligating the nucleic acid molecule hybridized with the first region and the nucleic acid molecule hybridized with the second region of the same bridging oligonucleotide II-II; and performing an extension reaction, to obtain a reaction product as a second nucleic acid molecule with a positioning tag, thereby generating a second nucleic acid molecule population; wherein the ligation reaction and the extension reaction are performed in any order.

23. The method according to claim 22, wherein in step (3)(ii):
(1) the first region and the second region of the bridging oligonucleotide II-I are directly adjacent, and the ligation of the nucleic acid molecule hybridized with the first region and the nucleic acid molecule hybridized with the second region of the same bridging oligonucleotide II-I comprises: using a nucleic acid ligase to ligate the nucleic acid molecule hybridized with the first region and the nucleic acid molecule hybridized with the second region of the same bridging oligonucleotide II-I; or,
the bridging oligonucleotide II-I comprises a first region, a second region and a third region between them, and the ligation of the nucleic acid molecule hybridized with the first region and the nucleic acid molecule hybridized with the second region of the same bridging oligonucleotide II-I comprises: using a nucleic acid polymerase (e.g., a nucleic acid polymerase with no 5' to 3' exonucleolytic activity or strand displacement activity) to perform a polymerization reaction with the third region as a template, and using a nucleic acid ligase to ligate the nucleic acid molecule hybridized with the first region and the nucleic acid molecule hybridized with the third region and the second region of the same bridging oligonucleotide II-I;
and/or
(2) the first region and the second region of the bridging oligonucleotide II-II are directly adjacent, and the ligation of the nucleic acid molecule hybridized with the first region and the nucleic acid molecule hybridized with the second region of the same bridging oligonucleotide II-II comprises: using a nucleic acid ligase to ligate the nucleic acid molecule hybridized with the first region and the nucleic acid molecule hybridized with the second region of the same bridging oligonucleotide II-II; or,
the bridging oligonucleotide II-II comprises a first region, a second region and a third region between them, and the ligation of the nucleic acid molecule hybridized with the first region and the nucleic acid molecule hybridized with the second region of the same bridging oligonucleotide II-II comprises: using a nucleic acid polymerase (e.g., a nucleic acid polymerase with no 5' to 3' exonucleolytic activity or strand displacement activity) to perform a polymerization reaction with the third region as a template, and using a nucleic acid ligase to ligate the nucleic acid molecule hybridized with the first region and the nucleic acid molecule hybridized with the third region and the second region of the same bridging oligonucleotide II-II.

24. The method according to claim 22 or 23, which comprises step (1), step (2)(i) and step (3); wherein, in step (2)(i)(b), the primer II-B comprises the consensus sequence B, a complementary sequence of the 3'-end overhang, and the tag sequence B;
preferably, in step (3), the second nucleic acid molecule derived from each copy of the oligonucleotide probe coupled to the same microdot has a different tag sequence B as a UMI.

25. The method according to claim 24, which comprises step (1), step (2)(i) and step (3)(i); wherein the consensus sequence X2 or partial sequence thereof is capable of annealing to a complementary sequence or partial sequence thereof of the consensus sequence B; the extension product obtained in step (3)(i) is taken as the labeled nucleic acid molecule, which comprises: a first strand comprising the sequence of the first nucleic acid molecule to be labeled, and/or, a second strand comprising the sequence of the oligonucleotide probe.

26. The method according to claim 24, comprising step (1), step (2)(i) and step (3)(ii); wherein the second region of the bridging oligonucleotide II-II is capable of annealing to a complementary sequence or partial sequence thereof of the consensus sequence B of the first extension product obtained in step (2)(i); and the reaction product obtained in step (3)(ii) is taken as the labeled nucleic acid molecule, which comprises: a first strand comprising the sequence of the first nucleic acid molecule to be labeled, and/or, a second strand comprising the sequence of the oligonucleotide probe.

27. The method according to any one of claims 24 to 26, wherein in step (2)(i)(a), the capture sequence A of the primer II-A is a random oligonucleotide sequence.

28. The method according to any one of claims 24 to 26, wherein in step (2)(i)(a), the capture sequence A of the primer II-A is a poly(T) sequence or a specific sequence targeting a target nucleic acid;
preferably, the primer II-A further comprises a consensus sequence A, and optionally a tag sequence A, such as a random oligonucleotide sequence.

29. The method according to claim 22 or 23, which comprises step (1), step (2)(ii) and step (3); wherein, in step (2)(ii)(b), the first extension product comprises from 5' to 3': the consensus sequence A, the cDNA sequence that is formed by reverse transcription primed by the primer II-A' and complementary to the RNA, the 3'-end overhang sequence, optionally a complementary sequence of the tag sequence B, and a complementary sequence of the consensus sequence B;
preferably, in step (2)(ii)(c), the extension primer is the primer II-B' or a primer B", wherein the primer B" is capable of annealing to a complementary sequence or partial sequence thereof of the consensus sequence B, and initiating an extension reaction.

30. The method according to claim 29, which comprises step (1), step (2)(ii) and step (3)(i); wherein the consensus sequence X2 or partial sequence thereof is capable of annealing to a complementary sequence or partial sequence thereof of the consensus sequence A; and the extension product obtained in step (3)(i) is taken as the labeled nucleic acid molecule, which comprises: a first strand comprising the sequence of the first nucleic acid molecule to be labeled, and/or, a second strand comprising the sequence of the oligonucleotide probe.

31. The method according to claim 29, comprising step (1), step (2)(ii) and step (3)(ii); wherein the second region of the bridging oligonucleotide II-II is capable of annealing to a complementary sequence or partial sequence thereof of the consensus sequence A of the second extension product obtained in step (2)(ii); and the reaction product obtained in step (3)(ii) is taken as the labeled nucleic acid molecule, which comprises: a first strand comprising the sequence of the first nucleic acid molecule to be labeled, and/or, a second strand comprising the sequence of the oligonucleotide probe.

32. The method according to any one of claims 29 to 31, wherein in step (2)(ii)(a), the capture sequence A of the primer II-A' is a random oligonucleotide sequence;
preferably, in step (3), the second nucleic acid molecule derived from each copy of the oligonucleotide probe coupled to the same microdot has a different capture sequence A as a UMI.

33. The method according to any one of claims 29 to 31, wherein in step (2)(ii)(a), the capture sequence A of the primer II-A' is a poly(T) sequence or a specific sequence targeting a target nucleic acid; wherein, the primer II-A' further comprises a tag sequence A, such as a random oligonucleotide sequence;
preferably, in step (3), the second nucleic acid molecule derived from each copy of the oligonucleotide probe coupled to the same microdot has a different tag sequence A as a UMI.

34. The method according to any one of claims 22 to 28, wherein, in step (2)(i)(b), the cDNA strand is annealed through its 3'-end overhang to the primer II-B, and, the cDNA strand is extended using the primer II-B as a template to generate the first extension product under the presence of a nucleic acid polymerase (e.g., a DNA polymerase or reverse transcriptase).

35. The method according to any one of claims 22 to 23 and 29 to 33, wherein in step (2)(ii)(b), the cDNA strand is annealed through its 3'-end overhang to the primer II-B', and the cDNA strand is extended using the primer II-B' as a template to generate the first extension product under the presence of a nucleic acid polymerase (e.g., a DNA polymerase or reverse transcriptase).

36. The method according to any one of claims 1 to 35, wherein in step (2), the pretreatment is performed intracellularly;
preferably, the RNA (e.g., mRNA) of the one or more cells is subjected to the pretreatment to generate the first nucleic acid molecule population before or after the one or more cells are contacted with the solid support of the nucleic acid array;
preferably, the cell is permeabilized before the pretreatment.

37. The method according to any one of claims 1 to 35, wherein in step (2), the pretreatment is performed extracellularly;
preferably, the RNA (e.g., mRNA) of the one or more cells is subjected to the pretreatment to generate the first nucleic acid molecule population after the one or more cells are contacted with the solid support of the nucleic acid array;
preferably, before the pretreatment is carried out, the method further comprises releasing intracellular RNA (e.g., mRNA); preferably, the intracellular RNA (e.g., mRNA) is released by cell permeabilization or cell lysis treatment.

38. The method according to any one of claims 1 to 37, wherein the reverse transcription as described in step (2) is performed by using a reverse transcriptase;
preferably, the reverse transcriptase has terminal deoxynucleotidyl transferase activity;
preferably, the reverse transcriptase is capable of synthesizing a cDNA strand using an RNA (e.g., mRNA) as a template, and adding an overhang at the 3' end of the cDNA strand.

39. The method according to any one of claims 1 to 38, wherein the method further comprises: (4) recovering and purifying the second nucleic acid molecule population.

40. The method according to any one of claims 1 to 39, wherein the obtained second nucleic acid molecule population and/or a complement thereof is used for constructing a transcriptome library or for transcriptome sequencing.

41. The method according to any one of claims 1 to 40, wherein the nucleic acid array of step (1) is provided by the following steps:
(1) providing multiple kinds of carrier sequences, in which each kind of carrier sequence comprises at least one copy of the carrier sequence, and the carrier sequence comprises in the direction from 5' to 3': a complementary sequence of the consensus sequence X2, a complementary sequence of the tag sequence Y, and an immobilization sequence; wherein, each kind of carrier sequence has a different complementary sequence of the tag sequence Y;
(2) attaching the multiple kinds of carrier sequences to the surface of a solid support (e.g., a chip);
(3) providing an immobilization primer, and using the carrier sequence as a template to perform a primer extension reaction to generate an extension product, so as to obtain the oligonucleotide probe; wherein the immobilization primer comprises the sequence of the consensus sequence X1, and is capable of annealing to the immobilization sequence of the carrier sequence and initiating the extension reaction; preferably, the extension product comprises or consists of in the direction from 5' to 3': the consensus sequence X1, the tag sequence Y and the consensus sequence X2;
(4) linking the immobilization primer to the surface of the solid support; wherein step (3) and step (4) are performed in any order;
(5) optionally, the immobilization sequence of the carrier sequence further comprises a cleavage site, and the cleavage can be selected from nicking enzyme digestion, USER enzyme digestion, light-responsive excision, chemical excision, or CRISPR-mediated excision; performing cleavage at the cleavage site contained in the immobilization sequence of the carrier sequence to digest the carrier sequence, so as to separate the extension product in step (3) from the template (i.e., the carrier sequence) from which the extension product is generated, thereby linking the oligonucleotide probe to the surface of the solid support (e.g., chip); preferably, the method further comprises separating the extension product in step (3) from the template (i.e., the carrier sequence) from which the extension product is generated through high-temperature denaturation;
preferably, each kind of carrier sequence is a DNB formed from a concatemer of multiple copies of the carrier sequence;
preferably, the multiple kinds of carrier sequences are provided in step (1) by the following steps:
(i) providing multiple kinds of carrier-template sequences, the carrier-template sequence comprises a complementary sequence of a carrier sequence;
(ii) using each kind of carrier-template sequence as a template to perform a nucleic acid amplification reaction to obtain an amplification product of each kind of carrier-template sequence, in which the amplification product comprises at least one copy of the carrier sequence; preferably, rolling circle replication is performed to obtain a DNB formed from a concatemer of the carrier sequence.

42. A method of constructing a library of nucleic acid molecules, comprising:
(a) generating a population of labeled nucleic acid molecules according to the method according to any one of claims 1 to 41;
(b) randomly fragmenting the nucleic acid molecules in the population of labeled nucleic acid molecules and linking an adapter thereto; and
(c) optionally, amplifying and/or enriching the product of step (b);
thereby obtaining a library of nucleic acid molecules;
preferably, the library of nucleic acid molecules comprises nucleic acid molecules from multiple single cells, and the nucleic acid molecules from different single cells have different tag sequences Y;
preferably, the library of nucleic acid molecules is used for sequencing, such as transcriptome sequencing, such as single-cell transcriptome sequencing (e.g., 5' or 3' transcriptome sequencing).

43. The method according to claim 42, wherein, before performing step (b), the method further comprises step (pre-b): amplifying and/or enriching the population of labeled nucleic acid molecules;
preferably, the amplification reaction is performed using at least a primer C and/or a primer D, wherein the primer C is capable of hybridizing with or annealing to a complementary sequence or partial sequence thereof of the consensus sequence X1, and initiating an extension reaction; the primer D is capable of hybridizing with or annealing to the nucleic acid molecule strand comprising the tag sequence Y in the population of labeled nucleic acid molecules, and initiating an extension reaction.

44. The method according to claim 43, wherein in step (b), the nucleic acid molecule obtained in the previous step is randomly fragmented and the resulting fragments are linked with an adapter at both ends respectively, by using a transposase;
preferably, in step (c), at least a primer C' and/or a primer D' is used to amplify the product of step (b),
wherein, the adapters at both ends of the fragment are a first adapter and a second adapter, respectively, the primer C' is capable of hybridizing with or annealing to the first adapter and initiating an extension reaction, and the primer D' is capable of hybridizing with or annealing to the second adapter and initiating an extension reaction.

45. A method for transcriptome sequencing of cells in a sample, which comprises:
(1) constructing a library of nucleic acid molecules according to the method according to any one of claims 42 to 44; and
(2) sequencing the library of nucleic acid molecules.

46. A method for performing single-cell transcriptome analysis, which comprises:
(1) performing transcriptome sequencing of single cells in a sample according to the method according to claim 45; and
(2) analyzing the sequencing data, which comprises performing match of the sequencing results of the sequencing library with the tag sequence Y of the oligonucleotide probe coupled to each microdot on the nucleic acid array or complementary sequence thereof, wherein the microdot is identified as a positive microdot if the match is successful, and, the sequencing data derived from the positive microdots with regional continuity in the nucleic acid array is identified as the transcription data of the same cell, thereby performing single-cell transcriptome analysis.

47. A kit, which comprises:
a nucleic acid array for labeling nucleic acids and optionally a first binding molecule, in which the nucleic acid array comprises a solid support, the solid support comprises (e.g., on its surface) a first label molecule, and the first binding molecule is capable of forming an interaction pair with the first label molecule;
the solid support further comprises a plurality of microdots, the size (e.g., equivalent diameter) of the microdots is less than 5 µm, and the center-to-center distance between adjacent microdots is less than 10 µm; each microdot is coupled with one kind of oligonucleotide probe; each kind of oligonucleotide probe comprises at least one copy; and, the oligonucleotide probe comprises or consists of in the direction from 5' to 3': a consensus sequence X1, a tag sequence Y, and a consensus sequence X2, wherein,
oligonucleotide probes coupled to different microdots have different tag sequences Y.

48. The kit according to claim 47, wherein the center-to-center distance between adjacent microdots is less than 10 µm, less than 5 µm, less than 1 µm, less than 0.5 µm, less than 0.1 µm, less than 0.05 µm, or less than 0.01 µm; and, the size (e.g., equivalent diameter) of the microdots is less than 5 µm, less than 1 µm, less than 0.3 µm, less than 0.5 µm, less than 0.1 µm, less than 0.05 µm, less than 0.01 µm, or less than 0.001 µm;
preferably, the center-to-center distance between adjacent microdots is 0.5 µm to 1 µm, such as 0.5 µm to 0.9 µm, 0.5 µm to 0.8 µm;
preferably, the size (e.g., equivalent diameter) of the microdots is 0.001 µm to 0.5 µm (e.g., 0.01 µm to 0.1 µm, 0.01 µm to 0.2 µm, 0.2 µm to 0.5 µm, 0.2 µm to 0.4 µm, 0.2 µm to 0.3 µm).

49. The kit according to claim 47 or 48, wherein the first binding molecule is capable of forming a specific interaction pair or a non-specific interaction pair with the first label molecule;
preferably, the interaction pair is selected from the group consisting of an interaction pair of positive charge and negative charge, affinity interaction pair (e.g., biotin/avidin, biotin/streptavidin, antigen/antibody, receptor/ligand, enzyme/cofactor), a pair of molecules capable of undergoing click chemical reaction (e.g., alkynyl-containing compound/azide compound), N-hydroxysulfosuccinate (NHS) ester/amino-containing compound, and any combination thereof;
for example, the first label molecule is polylysine, and the first binding molecule is a protein capable of binding to polylysine; the first label molecule is an antibody, and the first binding molecule is an antigen capable of binding to the antibody; the first label molecule is an amino-comprising compound, and the first binding molecule is N-hydroxysulfosuccinate (NHS) ester; or the first label molecule is biotin, and the first binding molecule is streptavidin.

50. The kit according to any one of claims 47 to 49, which further comprises:
(i) a primer I-A, or a primer set comprising a primer I-A' and a primer I-B, or a primer set comprising the primer I-A and the primer I-B, wherein:
the primer I-A comprises a consensus sequence A and a capture sequence A, and the capture sequence A is capable of annealing to an RNA (e.g., mRNA) to be captured and initiating an extension reaction; preferably, the consensus sequence A is located upstream of the capture sequence A (e.g., located at the 5' end of the primer I-A);
the primer I-A' comprises a capture sequence A, and the capture sequence A is capable of annealing to an RNA (e.g., mRNA) to be captured and initiating an extension reaction;
the primer I-B comprises a consensus sequence B, a complementary sequence of a 3'-end overhang, and optionally a tag sequence B; wherein the complementary sequence of a 3'-end overhang is located at the 3' end of the primer I-B, and the consensus sequence B is located upstream of the complementary sequence of a 3'-end overhang (e.g., located at the 5' end of the primer I-B); wherein the 3'-end overhang refers to one or more non-templated nucleotides contained in the 3' end of the cDNA strand generated by reverse transcription with the RNA captured by the capture sequence A of the primer I-A' as a template;
and,
(ii) a bridging oligonucleotide I, which comprises: a first region and a second region, and optionally a third region located between the first region and the second region, and the first region is located upstream of the second region (e.g., located 5' of the second region); wherein,
the first region is capable of (a) annealing to the whole or a part of the consensus sequence A of the primer I-A or (b) annealing to the whole or a part of the consensus sequence B of the primer I-B;
the second region is capable of annealing to the whole or a part of the consensus sequence X2.

51. The kit according to claim 50, which comprises: the primer I-A as described in (i), and the bridging oligonucleotide I as described in (ii); wherein, the first region of the bridging oligonucleotide I is capable of annealing to the whole or a part of the consensus sequence A of the primer I-A, and the second region of the bridging oligonucleotide I is capable of annealing to the whole or a part of the consensus sequence X2;
wherein, the capture sequence A of the primer I-A is a random oligonucleotide sequence; or, the capture sequence A of the primer I-A is a poly(T) sequence or a specific sequence targeting a target nucleic acid, and the primer I-A further comprises a tag sequence A, such as a random oligonucleotide sequence;
preferably, the primer I-A comprises a 5' phosphate at the 5' end.

52. The kit according to claim 50, which comprises: the primer set comprising the primer I-A' and the primer I-B as described in (i), and the bridging oligonucleotide I as described in (ii); wherein, the first region of the bridging oligonucleotide I is capable of annealing to the whole or a part of the consensus sequence B of the primer I-B, and the second region of the bridging oligonucleotide I is capable of annealing to the whole or a part of the consensus sequence X2;
wherein, the capture sequence A of the primer I-A' is a random oligonucleotide sequence; or, the capture sequence A of the primer I-A' is a poly(T) sequence or a specific sequence targeting a target nucleic acid, and the primer I-A' further comprises a tag sequence A, and a consensus sequence A;
wherein, the primer I-B comprises the consensus sequence B, a complementary sequence of the 3'-end overhang, and the tag sequence B;
preferably, the kit further comprises a primer B", and the primer B" is capable of annealing to a complementary sequence or partial sequence thereof of the consensus sequence B and initiating an extension reaction.
preferably, the primer I-B or primer B" comprises a 5' phosphate at the 5' end;
preferably, the primer I-B comprises a modified nucleotide (e.g., a locked nucleic acid); preferably, the primer I-B comprises one or more modified nucleotides (e.g., one or more locked nucleic acids) at the 3' end.

53. The kit according to claim 50, which comprises: the primer set comprising the primer I-A and the primer I-B as described in (i), and the bridging oligonucleotide I as described in (ii); wherein, the first region of the bridging oligonucleotide I is capable of annealing to the whole or a part of the consensus sequence A of the primer I-A, and the second region of the bridging oligonucleotide I is capable of annealing to the whole or a part of the consensus sequence X2;
wherein, the capture sequence A of the primer I-A is a random oligonucleotide sequence; or, the capture sequence A of the primer I-A is a poly(T) sequence or a specific sequence targeting a target nucleic acid, and the primer I-A further comprises a tag sequence A, such as a random oligonucleotide sequence;
preferably, the primer I-A comprises a 5' phosphate at the 5' end;
preferably, the primer I-B comprises a modified nucleotide (e.g., a locked nucleic acid); preferably, the primer I-B comprises one or more modified nucleotides (e.g., one or more locked nucleic acids) at the 3' end.

54. The kit according to any one of claims 47 to 49, which further comprises:
(i) a primer set comprising a primer II-A and a primer II-B, or a primer set comprising a primer II-A' and a primer II-B', wherein:
the primer II-A comprises a capture sequence A, and the capture sequence A is capable of annealing to an RNA (e.g., mRNA) to be captured and initiating an extension reaction;
the primer II-B comprises a consensus sequence B, a complementary sequence of a 3'-end overhang, and optionally a tag sequence B; wherein the complementary sequence of a 3'-end overhang is located at the 3' end of the primer II-B, the consensus sequence B is located upstream of the complementary sequence of a 3'-end overhang (e.g., located at the 5' end of the primer II-B); wherein the 3'-end overhang refers to one or more non-templated nucleotides contained in the 3' end of a cDNA strand generated by reverse transcription with the RNA captured by the capture sequence A of the primer II-A as a template;
the primer II-A' comprises a consensus sequence A and a capture sequence A; wherein the capture sequence A is located at the 3' end of the primer II-A', and the consensus sequence A is located upstream of the capture sequence A (e.g., located at the 5' end of the primer II-A');
the primer II-B' comprises a consensus sequence B, a complementary sequence of a 3'-end overhang, and optionally a tag sequence B; wherein the complementary sequence of a 3'-end overhang is located at the 3' end of the primer II-B', the consensus sequence B is located upstream of the complementary sequence of a 3'-end overhang (e.g., located at the 5' end of the primer II-B'); wherein the 3'-end overhang refers to one or more non-templated nucleotides contained in the 3' end of a cDNA strand generated by reverse transcription with the RNA captured by the capture sequence A of the primer II-A' as a template.

55. The kit according to claim 54, which comprises: the primer set comprising the primer II-A and the primer II-B as described in (i), and, (ii) a bridging oligonucleotide II-I and a bridging oligonucleotide II-II; wherein, the bridging oligonucleotide II-I and the bridging oligonucleotide II-II each independently comprise: a first region and a second region, and optionally a third region located between the first region area and the second region, the first region is located upstream of the second region (e.g., located 5' of the second region); wherein,
the first region of the bridging oligonucleotide II-I is capable of annealing to the first region of the bridging oligonucleotide II-II; and the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence B of the primer II-B;
wherein, the capture sequence A of the primer II-A is a random oligonucleotide sequence; or, the capture sequence A of the primer II-A is a poly(T) sequence or a specific sequence targeting a target nucleic acid, and the primer II-A preferably further comprises a consensus sequence A and optionally a tag sequence A, such as a random oligonucleotide sequence;
wherein, the primer II-B comprises the consensus sequence B, a complementary sequence of the 3'-end overhang, and the tag sequence B;
preferably, the primer II-B comprises a modified nucleotide (e.g., a locked nucleic acid); preferably, the primer II-B comprises one or more modified nucleotides (e.g., one or more locked nucleic acids) at the 3' end.

56. The kit according to claim 54, which comprises: the primer set comprising the primer II-A and primer II-B as described in (i);
wherein, the capture sequence A of the primer II-A is a random oligonucleotide sequence; or, the capture sequence A of the primer II-A is a poly(T) sequence or a specific sequence targeting a target nucleic acid, and the primer II-A preferably further comprises a consensus sequence A and optionally a tag sequence A, such as a random oligonucleotide sequence;
wherein, the primer II-B comprises the consensus sequence B, a complementary sequence of the 3'-end overhang, and the tag sequence B;
preferably, the primer II-B comprises a modified nucleotide (e.g., a locked nucleic acid); preferably, the primer II-B comprises one or more modified nucleotides (e.g., one or more locked nucleic acids) at the 3' end.

57. The kit according to claim 54, which comprises: the primer set comprising the primer II-A' and primer II-B' as described in (i), and, (ii) a bridging oligonucleotide II-I and a bridging oligonucleotide II-II; wherein, the bridging oligonucleotide II-I and the bridging oligonucleotide II-II each independently comprise: a first region and a second region, and optionally a third region located between the first region and the second region, the first region is located upstream of the second region (e.g., located 5' of the second region); wherein,
the first region of the bridging oligonucleotide II-I is capable of annealing to the first region of the bridging oligonucleotide II-II; the second region of the bridging oligonucleotide II-I is capable of annealing to the consensus sequence X2 or partial sequence thereof of the oligonucleotide probe;
the second region of the bridging oligonucleotide II-II is capable of annealing to a complementary sequence or partial sequence thereof of the consensus sequence A of the primer II-A';
wherein, the capture sequence A of the primer II-A' is a random oligonucleotide sequence; or, the capture sequence A of the primer II-A' is a poly(T) sequence or a specific sequence targeting a target nucleic acid, and the primer II-A' further comprises a tag sequence A, such as a random oligonucleotide sequence;
preferably, the primer II-B' comprises a modified nucleotide (e.g., a locked nucleic acid); preferably, the primer II-B' comprises one or more modified nucleotides (e.g., one or more locked nucleic acids) at the 3' end;
preferably, the kit further comprises a primer B", and the primer B" is capable of annealing to a complementary sequence or partial sequence thereof of the consensus sequence B, and initiating an extension reaction.

58. The kit according to claim 54, which comprises the primer set comprising the primer II-A' and primer II-B' as described in (i);
wherein, the capture sequence A of the primer II-A' is a random oligonucleotide sequence; or, the capture sequence A of the primer II-A' is a poly(T) sequence or a specific sequence targeting a target nucleic acid, and the primer II-A' further comprises a tag sequence A, such as a random oligonucleotide sequence;
wherein, the primer II-B' comprises the consensus sequence B, a complementary sequence of the 3'-end overhang, and the tag sequence B;
preferably, the primer II-B' comprises a modified nucleotide (e.g., a locked nucleic acid); preferably, the primer II-B' comprises one or more modified nucleotides (e.g., one or more locked nucleic acids) at the 3' end;
preferably, the kit further comprises a primer B", and the primer B" is capable of annealing to a complementary sequence or partial sequence thereof of the consensus sequence B, and initiating an extension reaction.

59. The kit according to any one of claims 47 to 58, which further comprises a reverse transcriptase, a nucleic acid ligase, a nucleic acid polymerase and/or a transposase;
preferably, the reverse transcriptase has terminal deoxynucleotidyl transferase activity; preferably, the reverse transcriptase is capable of synthesizing a cDNA strand using an RNA (e.g., mRNA) as a template, and adding a 3'-end overhang to the 3' end of the cDNA strand.

60. The kit according to any one of claims 47 to 59, which further comprises: a reagent for nucleic acid hybridization, a reagent for nucleic acid extension, a reagent for nucleic acid amplification, a reagent for recovering or purifying nucleic acid, a reagent for constructing transcriptome sequencing library, a reagent for sequencing (e.g., second- or third-generation sequencing), or any combination thereof.

61. Use of the method according to any one of claims 1 to 41 or the kit according to any one of claims 47 to 60 for constructing a library of nucleic acid molecules or for performing transcriptome sequencing;
preferably, the method or kit is used to construct a single-cell library of nucleic acid molecules or to perform single-cell transcriptome sequencing.
